(19) Europäisches Patentamt
European Patent Office
Office européen des brevets

(11) Publication number: 0 370 553 A2

(12) # EUROPEAN PATENT APPLICATION

(21) Application number: 89202845.7

(22) Date of filing: 08.11.89

(51) Int. Cl.5: B01J 29/06, //C07C5/25, C07C2/86,C07C37/16, C07C29/50

(30) Priority: 23.11.88 US 275169

(43) Date of publication of application: 30.05.90 Bulletin 90/22

(84) Designated Contracting States: BE CH DE ES FR GB GR IT LI NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Brownscombe, Thomas Fairchild
2737 Amherst
Houston Texas 77005(US)
Inventor: Slaugh, Lynn Henry
12638 Rifleman Trail
Cypress Texas 77429(US)

(74) Representative: Aalbers, Onno et al
P.O. Box 302
NL-2501 CH The Hague(NL)

(54) **Basic metal-zeolite compositions, their preparation and use in base catalyzed reactions.**

(57) Basic compositions comprising a zeolite and one or more metal compounds of Group IA, IIA, the Transition Metals or the Rare Earth Metals wherein the sum of the amount of the metal compound(s) in said compound(s) and any metal cation exchanged into the zeolite is in excess of that required to provide a fully metal cation-exchanged zeolite.

The basic compositions can be suitably applied in base catalyzed reactions such as the double bond isomerization of olefins, the alkylation of aromatic compounds and the oxidative production of primary alkanols and/or aldehydes from paraffins.

EP 0 370 553 A2

# BASIC METAL-ZEOLITE COMPOSITIONS, THEIR PREPARATION AND USE IN BASE CATALYZED REACTIONS

This invention relates to basic metal-containing zeolite compositions, their preparation and use in base catalyzed reactions.

Zeolites are crystalline aluminosilicate minerals of a cage-network structure with pores a few angstrom units in diameter. Some of the common materials, such as zeolite Y (faujasite) or zeolite A have a three dimensional structure with pore intersections ("supercages") somewhat larger than the pore size. Others such as zeolite L have channels with diffusional cross connections. Zeolites are useful as shape selective adsorbents for a variety of organic molecules and shape selective catalysts for a variety of chemical processes.

Levels of hydration of zeolites determine the number of Si-OH and Al-OH species present. The interaction of such hydroxyl groups with the aluminium oxide centres is generally thought to yield protonic (Bronsted) acid sites, while the aluminium oxide centres themselves are capable of serving as electron acceptor (Lewis acid) sites. Normal zeolitic materials have acid activity, and even if they are neutralized with base, such as with the conversion of H-zeolite to Na-zeolite, they retain some acid character. Some references consider zeolites exchanged with alkali metal cations to have some soft base sites, with an excess of acidic sites and some basic sites coexisting simultaneously. See, for example, Barthomeuf et.al., "Basicity and Basic Catalytic Properties of Zeolites", Materials Chemistry and Physics, 18(1988) 553-575 and U.S. pat. spec. 4,140,726, particularly at column 5, lines 39-44. However, neither of these references teach the use of alkali metal compounds in conjunction with a zeolite wherein the amount of alkali metal exceeds the exchange capacity of the zeolite. The acidic properties of zeolites allow them to be used to catalyze acid-catalyzed reactions such as cracking, rearrangements, alkylation of aromatic rings, etc. However, since all known zeolites, including ion exchanged materials, exhibit acid catalytic properties, it would thus be of considerable economic importance to produce materials having the narrow channels and potential for shape selective catalysis of zeolites wherein the basic properties of such materials predominated over the acidic properties, if any, present in the materials. Such materials would be useful for a variety of reactions such as side chain alkylation of aromatics, olefin oligomerization, selective oxidation, condensations and double bond isomerization without skeletal rearrangement which are difficult to achieve with normal acid zeolites.

The present invention thus relates to basic compositions comprising a zeolite and one or more metal compounds of Group IA, IIA, the Transition Metals or the Rare Earth Metals wherein the sum of the amount of the metal metal compounds in said compound and any metal cation exchanged into the zeolite is in excess of that required to provide a fully metal cation-exchanged zeolite. The present invention further relates to methods for preparing the instant compositions. In a preferred embodiment the instant compositions are prepared by impregnating a zeolite with a solution of one or more metal compounds of Group IA, Group IIA, the Transition Metals or the Rare Earth Metals wherein said metal(s) impregnated in the zeolite is (are) in excess of that required to exchange out the ion exchangable sites present in the zeolite; drying the resultant impregnated material and then calcining the resultant composition. Preferred metal compounds used in the impregnation are those which at least partially decompose in the presence of the zeolite to oxides or oxidic compounds upon heating to temperatures below 850 °C. Preferred calcining conditions generally range from 150 °C to 850 °C in neutral or oxidizing atmospheres.

It is a further object of this invention to provide metal-zeolite compositions which are basic in nature and which would be particularly suitable for the adsorption of acidic gases and for the catalysis of base-catalyzed reactions. The instant compositions are also useful as supports for other catalytic components.

Essentially any crystalline zeolitic alumino-silicate can be employed as starting material to prepare the compositions of the instant invention. The zeolites can include both synthetic and naturally occurring zeolites. Illustrative of the synthetic zeolites are Zeolite X (U.S. pat. spec. 2,882,244); Zeolite Y (U.S. pat. spec. 3,130,007); Zeolite A (U.S. pat. spec. 2,882,243; Zeolite L (Bel. pat. spec. 575,117); Zeolite D (Can. pat. spec. 611,981); Zeolite R (U.S. pat. spec. 3,030,181); Zeolite S (U.S. pat. spec. 3,054,657); Zeolite T (U.S. pat. spec. 2,950,952); Zeolite Z (Can. pat. spec. 614,995); Zeolite E (Can. pat. spec. 636,931); Zeolite F (U.S. pat. 2,995,358); Zeolite O (U.S. pat. spec. 3,140,252); Zeolite W (U.S. pat. spec. 3,008,803); Zeolite Q (U.S. pat. spec. 2,991,151); Zeolite M (U.S. pat. spec. 2,995,423); Zeolite H (U.S. pat. spec. 3,010,789); Zeolite J (U.S. pat. spec. 3,001,869); Zeolite W (U.S. pat. spec. 3,012,853); Zeolite KG (U.S. pat. spec. 3,056,654); Zeolite SL (Dutch pat. spec. 6,710,729); Zeolite Omega (Can. pat. spec. 817,915); Zeolite ZK-5 (U.S. pat. spec. 3,247,195); Zeolite Beta (U.S. pat. spec. 3.308,069); Zeolite ZK-4 (U.S. pat. spec. 3,314,752); Zeolite ZSM-5 (U.S. pat. spec. 3,702,886); synthetic mordenite; the so-called ultrastable zeolites described in U.S. pat. spec. 3,293,192 and 3,449,070; and the references cited therein, incorporated herein by reference. Other synthetic zeolites are described in the book "Zeolite Molecular Sieves-Structure, Chemistry and Use," by Donald W. Breck, 1974, John Wiley & Sons, incorporated by reference herein. Illustrative of the naturally occurring crystalline zeolites are analcime, bikitaite, edingtonite, epistilbite, levynite, dachiardite, erionite, faujasite, analcite, paulingite, noselite, ferrierite, heulandite, scolecite, stilbite, clinoptilolite, harmotone, phillipsite, brewsterite, flakite, datolite, chabazite, gmelinite, cancrinite, leucite, lazurite, scolecite, mesolite, ptilolite, mordenite, nepheline, natrolite, scapolite, thomsonite, gismondine, garronite, gonnardite, heulandite, laumontite, levynite, offretite, yugawaralite. Descriptions of certain naturally occurring zeolites are found in the aforementioned book by Breck and in the book "Molecular Sieves-Principles of Synthesis and Identification", R. Szostak, Van Nostrand Reinhold, New York, 1989, both incorporated by reference herein, and in other known references. These zeolites may be in the hydrogen form or may be partially or fully exchanged with ammonium or metal ions.

As used herein, the term "compound" as applied to Group IA, Group IIA, the Transition Metals or the Rare Earth metals refers to the combination of such metals with one or more elements by chemical and/or physical and/or surface bonding, such as ionic and/or covalent and/or coordinate and/or van der Waals bonding, but specifically excludes that bonding involved between such metal and a zeolite when such metal is located in a cation exchange site of the zeolite. The term "ionic" or "ion" refers to an electrically charged moiety; "cationic" or "cation" being positive and "anionic" or "anion" being negative. The term "oxyanionic" or "oxyanion" refers to a negatively charged moiety containing at least one oxygen atom in combination with another element. An oxyanion is thus an oxygen-containing anion. It is understood that ions do not exist in vacuo, but are found in combination with charge-balancing counter ions. The term "oxidic" refers to a charged or neutral species wherein an element such as a metal is bound to oxygen and possibly one or more different elements by surface and/or chemical bonding. Thus, an oxidic compound is an oxygen-containing compound, which also may be a mixed, double, or complex surface oxide. Illustrative oxidic compounds include, by way of non-limiting example, oxides (containing only oxygen as the second element), hydroxides, nitrates, sulphates, carboxylates, carbonates, bicarbonates, oxyhalides, etc, as well as surface species wherein the metal is bound directly or indirectly to an oxygen either in the substrate or the surface. "Surface" as applied to zeolites and the instant compositions refers to external surface as well as the internal pore surface, the internal surface being both the surface of the macro pores resulting from the agglomeration of individual particles or crystallites as well as the surface of the mesopores and micropores and supercages that result from the intrinsic zeolite crystal structure. The term "salt" as used in the instant specification and claims is meant to encompass a single salt as well as mixtures of two or more salts. The term "Group IA" is used herein as a descriptor of the "alkali metal" elements of the Periodic Table of the Elements (Li, Na, K, Rb, Cs, Fr). The term "Group IIA" is used herein as a description of the "alkaline earth metal" elements of the Periodic Table of the Elements (Be, Mg, Ca, Sr, Ba, Ra). The term "Transition Metals" as used herein denotes elements having atomic nos. 21-29, 39-47 and 72-79. The term "Rare Earth Metals" as used herein denotes elements having atomic nos. 57-71. The metals as described herein do not refer the elements in the metallic or zero valent state, but rather are a shorthand use for the elements in the positive valent state, that is, they will be understood to be combined as a salt, compound, complex, etc. The term "basic" refers to having the characteristic of a base; e.g., when placed in a solution, a basic material will have a pH consistent with a base rather than an acid and, if a catalyst, will catalyze chemical reactions that are catalyzed by bases.

The metal salts that are suitable for preparing the compositions of the instant invention are any salts that can be dissolved in a suitable impregnating solution or which can be melted to form their own impregnating solution or which can be sublimed and condensed on the zeolite. Illustrative but non-limiting examples of suitable salts are alkali metal bicarbonates, carbonates, chlorates, perchlorates, hypochlorites, cyanates, hydroxides, iodates, nitrates, nitrites, sulphates, hydrogen sulphates, sulphites, dithionates, thiosulphates, alkoxides, carboxylates, sulphonates, iodates, halides and the like. Of the alkali(ne earth) salts that can be utilized in the instant invention, the hydroxide salts, particularly of alkali metals, are less preferred since these strongly basic salts in high concentrations can contribute to a degradation of the crystallinity of the zeolite. Salts which can be solubilized in a suitable solution are preferred. Preferred salts are those which have an oxygen-containing anion or oxyanion. Useful salts are for instance alkali(ne earth) metal salts which decompose at least in part upon calcination in the presence of the zeolite to provide an alkali(ne earth) metal-oxygen-containing moiety (e.g. Na-O-, Ca-O-, etc.), that is, produce an oxidic compound. When the alkali(ne earth) metal salt is associated with an anion which does not contain oxygen it is necessary that the salt be precipitated in situ with a suitable oxyanion, or alternatively, after impregnation, the subsequent calcination carried out in an oxygen-containing atmosphere to cause the salt to react with the oxygen to provide the alkali(ne earth) metal-oxygen-containing moiety, that is, produce an alkali(ne earth) metal oxidic compound. Decomposition can be indicated by the evolution of gases such as carbon oxides, nitrogen oxides, sulphur oxides etc. Decomposition will also be indicated by disappearance at least in part of the particular anionic form associated with the alkali(ne earth) metal in the impregnation liquid. For example, when carboxylates and alkoxides are calcined the carboxylate and alkoxide moiety associated with the alkali(ne earth) metal will decompose giving off carbon oxides and/or water and/or hydrocarbons, thereby disappearing at least in part. Particularly preferred salts to be used in an impregnating solution are (alkali) nitrates bicarbonates, carbonates, acetates and oxalates, particularly of potassium. Mixtures of alkali(ne earth) metal salts, that is, two or more salts with differing anions, differing cations or differing anions and cations can be utilized to prepare the impregnated zeolite.

One method that can be used to prepare the compositions of the instant invention involves the use of molten alkali(ne earth) metal salts to impregnate the zeolite. In this method a suitable salt, that is, one melting below 850 °C, is melted and the zeolite is added to the molten salt(s) or the molten salt(s) is (are) added to the zeolite causing the molten salt(s) to impregnate the pores of the zeolite. A very suitable impregnation technique is to utilize that amount of molten salt that is equal to or less than that amount of molten salt that will just fill the pores of the zeolite. Alternatively, zeolite particles can be immersed in a molten salt bath to cause impregnation of the molten salt(s) into the zeolite followed by separation of the excess molten salt(s) from the zeolite, say by filtration, centrifugation or washing. Alternatively, zeolite particles can be coated with finely divided particles of suitable alkali(ne earth) metal salt(s) and heated to above the melting point of the salt(s), causing the molten salt(s) to impregnate the pores of the zeolite. Many other methods, such as fluid bed impregnation or spraying molten salt(s) or solid salt(s) onto the zeolite in a rotating kiln will be obvious to one skilled in the art. After impregnation, the impregnated zeolite is calcined to produce the composition and/or catalyst of the instant invention. The calcining temperature may be the same or lower than the impregnating temperature but frequently it is higher. Drying is not required when the molten salt technique is utilized, but may be utilized to remove residual water remaining in the zeolite. The impregnation and calcination can be carried out in one continuous step or sequence. The alkali(ne earth) metal nitrates and carboxylates are particularly suitable for use in the molten impregnation method.

Another method is to use a sublimable alkali(ne earth) metal salt. In this method a suitable salt is sublimed at above its sublimation temperature to produce a vaporous salt and the resulting vapour is contacted with the zeolite maintained at a temperature below the sublimation temperature of the salt thereby causing the vapour to condense upon and within the pores of the zeolite thereby impregnating it. Calcination follows to prepare the compositions of the instant invention. Drying before calcination is not required in this case, but may be utilized to remove residual water in the zeolite. The impregnation and calcination can be carried out in one continuous step or sequence.

When the catalyst comprises a zeolite-alkaline earth metal compound, a preferred variation on the impregnation technique comprises impregnating the zeolite with a soluble salt of an alkaline earth metal salt, followed by contact or reimpregnation with a precipitating agent, such as a suitable solubilized anion, that will form a precipitate in situ with alkaline earth metal ion. For example, a zeolite is first impregnated with an aqueous solution of barium or calcium nitrate or chloride. Then the impregnated zeolite, without intermediate drying and/or calcining is contacted with an aqueous solution of ammonium sulphate or hydroxide, causing barium or calcium sulphate or hydroxide to precipitate within the zeolite. This resultant material is then dried as necessary and optionally calcined. Gaseous precipitating agents may also be utilized. For example, after a zeolite is first impregnated with an aqueous solution of alkaline earth metal nitrate, it is then contacted with or without intermediate drying with gaseous ammonia or dimethylamine, resulting in the precipitation of or conversion to the alkaline earth metal hydroxide. Preferred precipitating agents are those which produce an oxidic compound or a compound which is converted to an oxidic compound upon calcination.

Most conveniently and preferably, solutions of alkali(ne earth) metal salts are used to impregnate the zeolites. The solvents utilized to dissolve the salts may be organic or inorganic. The only requirement is that the desired salt(s) be soluble in the particular solvent. Hydroxylic solvents are preferred. Water is a particularly preferred solvent. The lower alkanols are also particularly suitable for use with salts having strong basicity in water in order to minimize base-zeolite structure interactions during the impregnation process. Organic solvents are particularly useful as solvents for alkali(ne earth) metal salts which have organic ionic components such as carboxylate, sulphonate, alkoxide, etc. Organic solvents are also useful for inorganic alkali(ne earth) metal salts. Alkali(ne earth) metal salts having a low solubility in an organic solvent can be used with that solvent to provide small, but well controlled amounts of alkali(ne earth) metal to the zeolite while minimizing solvent-base-zeolite structure interactions. Illustrative, but non-limiting examples of organic solvents include alcohols, including polyhydric alcohols, ethers, esters, ketones, amides, sulphoxides and chloro/fluorohydrocarbons such as the various freons. Specific illustrative examples include methanol, ethanol, glycol, dimethyl ether, methyl acetate, methylethyl ketone, dimethyl formamide ("DMF"), dimethyl sulphoxide ("DMSO"), N-methyl pyrrolidone ("NMP"), hexamethylphosphoramide ("HMPA"), dichlorodifluoromethane, methyl chloride, ethylene dichloride, ethylene carbonate, etc. Illustrative, but not-limiting examples of inorganic solvents include water, liquid ammonia, liquid carbon dioxide, liquid sulphur dioxide, carbon disulphide, carbon tetrachloride, etc. Mixtures of solvents which are mutually miscible may be utilized.

Single or multiple impregnations may be used. When multiple impregnations are used intermediate drying steps, optionally followed by calcination may be utilized. Generally any amount of impregnating liquid can be used in the impregnation process. For example, the zeolite can be dipped into a large excess (compared to the pore volume of the zeolite), removed and shaken of excess liquid. Alternatively, an amount of impregnating liquid considerably less than the pore volume can be sprayed onto an agitated bed of zeolite. For purposes of economy, control and other reasons, the volume of impregnating liquid will preferably range from about the pore volume to about four or five times, preferably about twice the pore volume of the zeolite to be impregnated. Alternatively a "dry" impregnation technique is utilized wherein just that amount of impregnating solution is used which will just fill the pores of the zeolite. In another embodiment, baskets of zeolite material are dipped into a vat of impregnating solution, removed, dried and optionally calcined. Alternatively, the zeolite may be tumbled with the impregnating solution.

The concentration of the metal salts in the impregnating solution is not critical and is selected, inter alia, on the basis of the zeolite used, the amount of ion exchange capacity present in the zeolite, the degree of basicity of the final product desired, the impregnation solvent used and the type of impregnation utilized, that is, multiple of single. Concentrations of metal salt(s) in the impregnating solution will typically range from 0.01 moles per litre to the solubility limit of the salt(s). A suitable range is from 0.01 to 20 moles per litre, more preferably from 0.1 to 10 moles per litre.

The amount of metal which is impregnated into the zeolite must be in excess of that which would provide a fully cation-ion exchanged zeolite. For example, if the starting zeolite were completely in the hydrogen form and had an ion exchange capacity of 12% (basis $Na_2O$), then the equivalent amount of, e.g. alkali(ne earth) metal impregnated (basis $Na_2O$) must exceed the 12%. If the starting zeolite were one which had already been 80% exchanged with a metal cation, the amount of metal to be added by impregnation would be in excess of that amount required to exchange the remaining 20%. If the starting zeolite were fully metal cation exchanged, then any amount of metal in the impregnating solution would suffice. It is to be understood that impregnation of a partially or fully cation-exchanged zeolite will most likely result in some counter ion exchange between the impregnating metal cation(s) and cations already present in the zeolite, but the resulting composition will still be within the scope of the instant invention in having an excess of metal present over the amount exchanged into the fully exchanged zeolite. When the amount of impregnating solution that is utilized is such that after impregnation no excess solution is removed, then the amount of metal salt(s) in the impregnating solution will be the same as the amount impregnated into the zeolite. When an amount of impregnating solution is used that requires that an excess amount of solution must be removed, for example, by filtration or centrifugation, from the impregnated zeolite after impregnation, then the amount of metal(s) in the impregnating solution will exceed the amount of metal(s) impregnated into the zeolite. In this latter case, the amount of metal(s) impregnated into the zeolite can be determined by a knowledge of concentration of metal(s) in the impregnating solution before the impregnation, the concentration of metal(s) in the excess solution removed from the impregnated zeolite and the amount of solution remaining after impregnation (the excess). Alternatively, the impregnated zeolite can be analyzed for metal content.

The compositions and catalysts of the instant invention which comprise a zeolite and metal compound as defined hereinbefore can be divided into four somewhat arbitrary classes, depending on the amount of metal compound(s) that is (are) present in the composition. In order of increasing basicity, there are low base, low-intermediate base, high-intermediate base and high base compositions. The higher the basicity of the instant compositions, the higher will be the suppression of any acid function of the zeolite. The calculations of the ranges and limits for these various classes are to be made considering the metal as defined hereinbefore as the metal (ion) and any metal(s) exchanged into the zeolite as the (ionic) equivalent of a metal.

In general it is preferred to have a slight excess of an appropriate metal present. When considering as a basis for calculation the zeolite having no cations exchanged therein, the preferred compositions will have the sum of the metal(s) in the metal compound(s) and any metal cation exchanged into the zeolite being greater than 1, preferably greater than about 1.05, more preferably greater than about 1.1, even more preferably greater than about 1.15, even more preferably greater than about 1.2, even more preferably greater than about 1.5 and even more preferably greater than about 2 times the amount required to provide a fully cation-exchanged zeolite (or times the exchange capacity). When considering the fully cation-exchanged zeolite as a basis for calculation, the amount of metal(s) in the metal compound(s) is greater than zero, preferably greater than about 0.05, more preferably greater than about 0.1, even more preferably greater than about 0.15, even more preferably greater than about 0.2, even more preferably greater than about 0.5 and even more preferably greater than about 1 times the amount of metal(s) that would be required to provide a fully metal cation-exchanged zeolite (or times the exchange capacity).

When considering as a basis for calculation the zeolite having no cations exchanged therein, the low base compositions will have the sum of the metal(s) in the metal compound(s) and any metal cation exchanged into the zeolite ranging from greater than to about 1.2 times the amount required to provide a fully cation-exchanged zeolite (or times the exchange capacity). When considering the fully cation-exchanged zeolite as a basis for calculation, the amount of metal(s) in the metal compound(s) is greater than to about 0.2 times the amount of metal(s) that would be required to provide a fully metal cation-exchanged zeolite (or times the exchange capacity).

When considering as a basis for calculation the zeolite having no cations exchanged therein, the low-intermediate base compositions will have the sum of the metal(s) in the metal(s) compound(s) and any metal cation exchanged into the zeolite ranging from about 1.2 to about 1.5 times the amount required to provide a fully cation-exchanged zeolite (or times the exchange capacity). When considering the fully cation-exchanged zeolite as a basis for calculation, the amount of metal(s) in the metal compound(s) ranges from about 0.2 to about 0.5 times the amount of metal(s) that would be required to provide a fully metal cation-exchanged zeolite (or times the exchange capacity).

When considering as a basis for calculation the zeolite having no cations exchanged therein, the high-intermediate base compositions will have the sum of the metal(s) in the metal compound(s) and any metal cation exchanged into the zeolite ranging from about 1.5 to about 2 times the amount required to provide a fully cation-exchanged zeolite (or times the exchange capacity). When considering the fully cation-exchanged zeolite as a basis for calculation, the amount of metal(s) in the metal compound(s) ranges from 0.5 to about 1 times the amount of metal(s) that would be required to provide a fully metal cation-exchanged zeolite (or times the exchange capacity).

When considering as a basis for calculation the zeolite having no cations exchanged therein, the high base compositions will have the sum of the metal(s) in the metal compound(s) and any metal cation exchanged into the zeolite ranging from about 2 times the amount required to provide a fully cation-exchanged zeolite (or times the exchange capacity) to the limit of metal compound(s) that can be physically impregnated into the zeolite, which in a preferred embodiment is about 3.5 times the amount required to provide a fully cation-exchanged zeolite (or times the exchange capacity). When considering the fully cation-exchanged zeolite as a basis for calculation, the amount of metal(s) in the metal compound(s) ranges from about 1 times the amount of metal(s) that would be required to provide a fully metal cation-exchanged zeolite (or times the exchange capacity) to the limit of metal compound(s) that can be physically impregnated into the zeolite, which in a preferred embodiment is about 2.5 times the amount required to provide a fully cation-exchanged zeolite (or times the exchange capacity).

When considered in terms of those cases where increasing basicity is desired, and when considered in terms of the non-cation exchanged zeolite as a basis for calculation, the sum of metal(s) in the metal compound(s) and any metal cation exchanged into the zeolite is greater than 1, preferably greater than about 1.2, more preferably greater than about 1.5 and even more preferably greater than about 2 times the amount required to provide a fully cation-exchanged zeolite (or times the exchange capacity). When considering the fully cation-exchanged zeolite, the amount of metal(s) in the metal compound(s) is greater than zero, preferably greater than about 0.2, more preferably greater than about 0.5, and even more preferably greater than 1 times the amount of metal(s) that would be required to provide a fully metal cation-exchanged zeolite.

After impregnation utilizing an impregnating solution or a subsequent precipitating solution, the impregnated zeolite is dried to remove the solvent of the impregnating and/or precipitating solution. The drying conditions are not critical to the instant invention. Drying may be carried out at atmospheric pressure, superatmospheric pressure or under vacuum. It also may be carried out by passing a dry (with regard to the impregnating solvent) gas over a bed of the zeolite. Drying temperatures will depend upon the solvent used. For those solvents that are liquid at low temperatures, such as liquid carbon dioxide or liquid sulphur dioxide, the drying temperature can be relatively low, that is, below room temperature. For the more conventional solvents which are liquid at or above room temperature, higher temperatures will be used. For these solvents temperatures will typically range from room temperature to 200 °C. In most cases drying temperatures will be less than 200 °C, preferably less than 150 °C. Drying times are dependent upon the drying temperature and pressure, typically from one minute to twenty hours, although longer or shorter times can be utilized. Drying atmospheres and pressures are normally not critical. The drying atmosphere may be neutral, oxidizing, reducing or a vacuum.

After drying to remove an impregnating solvent or after impregnation by means of a molten or vaporous salt, the impregnated zeolite is optionally calcined at elevated temperatures. Calcination conditions will range from 150 °C to 850 °C, preferably from 200 °C to 750 °C, and more preferably from 200 °C to 600 °C. Calcining times are dependent on the calcining conditions selected and typically range from one minute to twenty hours, although longer or shorter times can be utilized. Calcining conditions and times are also adjusted according to the thermal stability. Calcination conditions should not be so extreme as to cause substantial loss of zeolite crystallinity. Calcining atmospheres may be neutral, oxidizing or reducing. When the impregnating salt has an anionic component which does not contain oxygen, an oxygen-containing calcining atmosphere is preferably utilized. When the calcined composition is a catalyst or a catalyst support, particular calcining atmospheres may be called for. For example, when certain catalytic metals need to be present in the reduced state, a reducing atmosphere is advantageously utilized. Barring special circumstances, neutral atmospheres such as provided by nitrogen and oxidizing atmospheres such as provided by air are preferred.

In a preferred embodiment when using an impregnation or an impregnation/precipitating solution, the drying and calcining steps are combined into one integrated process step. In this combined step the impregnated zeolite is heated through the lower temperatures at a rate slow enough that physical disruption of the zeolite does not occur due to rapid volatilization of the solvent from the impregnation. After the solvent has been removed, the zeolite is then heated to the desired calcining temperature, maintained for the desired calcining time and then cooled to room temperature. Calcining (and drying) can be carried out in situ during the operation of a catalytic process in a reactor.

The exact form of the metal(s) after calcination in the instant compositions is not known. Without intending to limit the scope of the instant invention, it is believed that the metal(s), such as the alkali(ne earth) metals is (are) present as one or more metal oxidic compounds. It is speculated that the alkali(ne earth) metal compound(s) are probably in the form of a surface oxide or multiple surface oxides with the zeolite, in particular with the aluminium and/or silicon and/or oxygen of the zeolite lattice, possible in combination with species contained in or formed from the impregnation solution or during the calcination process.

The calcination contributes to the production of a composition and/or a catalyst which is basic and this basic nature is thought to derive from the particular nature of the metal compound(s) present after calcination. However, those catalysts produced by precipitation with a basic precipitating agent are within the scope of the instant invention, even without calcination taking place. The basic nature of those materials can be seen from the fact that instant compositions or catalysts when placed in a solvent produce effects that are basic rather than acidic in nature. This can been seen by the use of suitable chemical or electrochemical indicators. The basic nature of the instant compositions or catalysts can also be seen from the fact that they will catalyze or carry out chemical reactions that are catalyzed or carried out by bases. For example, the dehydrochlorination of chlorohydrocarbons such as 1-chlorooctane, are well known to be carried out by bases and the instant compositions also carry out this reaction.

The basicity of the instant compositions can be determined in various ways. For example, it can be determined by measuring the extent to which various base-catalyzed reactions are carried out in the presence of the instant compositions or catalysts. Another method is to place the instant composition or catalyst in a solvent and measure the resulting pH by use of chemical or electrochemical indicators. A specific example would involve placing 20 mg of composition or catalyst in 2 g of water and using a pH meter or pH paper to measure the resulting pH. Another method is to use various indicators in non-aqueous solutions and compare the indicator response caused by the instant compositions or catalysts with the indicator response caused by selected reference samples. Two such suitable indicators are 4-nitroaniline or 4-chloroaniline dissolved in dimethyl sulphoxide ("DMSO" or benzene (0.1 g/cc)). These indicators, however, may have too large a molecular cross-section to be used with very small pore zeolites, and other indicators or methods of determining basicity would have to be used. Examples of indicator responses with various reference samples are shown in Table 1 below.

Table 1

| Reference | 4-nitroaniline/DMSO | 4-chloroaniline/benzene |
|---|---|---|
| $NaNH_2$ | very dark blue | purplish brown |
| KOH | dark blue | cream |
| NaY-Zeolite | yellow | cream |
| amorphorous $SiO_2$ | faint yellow | cream |

In general terms the compositions and/or catalysts of the instant invention comprise a basic, structured, that is a zeolitically structured, metal(s)-containing aluminosilicate containing in compound form an excess of metal(s) over that necessary to provide a fully metal cation-exchanged aluminosilicate. More specifically, the instant compositions comprise a zeolite and an alkali(ne earth) metal compound, particularly an oxidic compound, wherein the sum of the amount of the alkali(ne earth) metal in the compound plus any metal cation exchanged into the zeolite is in excess of that required to provide a fully metal cation-exchanged zeolite. The instant compositions and/or catalysts will contain at least a portion of their pore volume in micropores in the range of from about four to about twelve angstroms. The instant compositions react as bases when placed in solvents and catalyze base-catalyzed reactions. In a preferred embodiment for shape selective catalysis, the alkali(ne earth) metal compound(s) is (are) substantially located on the internal pore surfaces of the zeolite rather than the external surfaces.

The instant compositions and/or catalysts retain at least a portion of a crystalline zeolite structure. The term "crystalline" is employed herein to designate an ordered structure capable of being detected by electrooptical or diffraction techniques, normally by X-ray diffraction, giving a consistent crystallographic pattern. Such an ordered structure can persist even after some of the structural silica or alumina is removed from the crystal lattice, as by leaching with acids, or with bases such as might occur during the impregnation process, or by other physical or chemical methods. Sometimes the ordered structure may become so attenuated by these or other means as to fail to diffract X-rays, but in such cases other electrooptical methods, such as electron beam diffraction may be utilized. In other cases the crystallite size may become so small that diffraction effects may become so diffuse that the amount of crystalline structure may be difficult to detect or determine. In this latter instance, however, the retention of a large surface area after chemical and/or physical processing will indicate the retention of a certain amount crystalline zeolite structure. Thus these latter materials are still structured aluminosilicates as opposed to amorphous aluminosilicates and are within the scope of the instant invention.

The compositions of the instant invention find many uses. They are useful as adsorbents. They can be used, for example, to adsorb acidic gases or vapours from a vapour phase, or to adsorb acidic species from a liquid phase.

The instant compositions can also serve as catalysts or catalyst supports for other catalytic components. Catalytic components may be incorporated into the instant compositions at any stage of their preparation, that is, prior to, during and after the conversion of the starting zeolite to final basic composition. Large numbers of materials can be added to the compositions of the instant invention in order to make catalysts. Illustrative but non-limiting examples include Group IIB metals (atomic nos. 30, 48, 80); Group IIIA metals (atomic nos. 5, 13, 31, 49, 81); Group IVA metals (atomic nos. 14, 32, 50, 82); Group VA metals (atomic nos. 15, 33, 51, 83); Group VIA metals (atomic nos. 34, 52); as well as their oxides, sulphides, halides, salts, complexes, compounds and the like.

Illustrative but non-limiting examples of organic chemical reactions for which the instant compositions will find use as catalysts include double bond isomerization, dehydrogenation, cyclization of polyenes, H-D exchange, condensation, side chain alkylation of alkyl benzenes, olefin dimerization, olefin oligomerization, dehydrohalogenation, cleavage coupled with oxidation, oxidation, reduction/elimination, nucleophilic addition, etc. Good results have been obtained using alkali(ne earth) metal containing zeolites according to the present invention in the double bond isomerization of olefins. The isomerization can be suitably applied using $C_4$-$C_{18}$ olefins, in particular olefins in the additive range ($C_4$ to $C_8$) an in the detergent range ($C_{10}$-$C_{18}$). The isomerization is suitably applied at a temperature between 0° and 500 °C, in particular between 100 °C and 500 °C.

The catalysts according to the present invention can be used advantageously in alkylating aromatic compounds. Lower alkanols, in particular methanol can be used as alkylating agent. Suitable aromatic compounds comprise phenol and toluene.

The catalysts according to the present invention can also be applied advantageously in preparing alkanols and/or aldehydes by reacting one or more paraffins with an oxygen-containing gas provided a metal compound having oxidative properties, scuh as a Mn, Ce or Sm compound, is also present. Air can be suitably applied as oxygen-containing gas.

Illustrative but non-limiting examples of organic chemical reactions for which the instant compositions when combined with other catalytic components will find use are illustrated in Table 2 below.

Table 2

| Catalytic Component | Reaction |
| --- | --- |
| Sc | C-H activation |
| V | oxidation |
| Cr | oxidation, coupling, cyclization, reductive cleavage |
| Mn | oxidation |
| Fe | oxidation, olefin isomerization, cyclization, reduction, halogenation |
| Co | oxidation, hydroformylation, hydrogenation, olefin isomerization, dimerization |
| Ni | hydrogenation, oligomerization, isomerization, carbonylation, dimerization, coupling, cyclization |
| Cu | oxidation, hydrogenation, coupling, cyclization, decarboxylation, cyanoethylation, halogenation |
| Y | oxidation, C-H activation |
| Nb | oxidation, olefin isomerization |
| Mo | oxidation, olefin isomerization |
| Ru,Rh,Pd | CO activation, oxidation, amination, hydrogenation, olefin isomerization, isomerization, cyclization |
| Ag | oxidation, hydrogenation, rearrangement, hydroxylation |
| La-Lu | oxidation, condensation, amination, dehydrogenation, oligomerization |
| Re | oxidation, hydration |
| Os | oxidation, epoxidation, hydrogenation |
| Pt | C-H activation, hydrogenation, CO activation, isomerization |
| Bi | oxidation, suppression of dehydrogenation |
| Th,U | CO activation, oxidation |

Table 2 (cont'd)

| Catalytic Component | Reaction |
|---|---|
| W | oxidation, activation |
| Sb | combustion suppression |
| Sn | weak acid binding site, reduction |
| Ir | hydrogenation, olefin isomerization |
| Hg | hydrogenation, oxidation, hydration, halogenation |
| Pb | oxidation, coupling, cyclization, decarboxylation, cleavage |
| Ti | oxidation, dimerization, rearrangements, isomerization |
| Tl | oxidation |
| Zn | ether cleavage |
| Zr | alkylation |

When catalytic components are added to the instant compositions numerous factors will be considered by one skilled in the art when preparing the combination of catalytic components and instant compositions. Non-limiting examples of these factors include pore structure; ease of activation; number of base sites; location and type of catalytic components; stability of the catalyst(temporal, thermal, hydrolytic, etc.); polarity (ionization ability) of zeolitic pore/cage; binding affinity (lipophilicity or hydrophilicity) of zeolitic pore/cage; use of promoters or activators to modify catalytic effects; shape selectivity; presence or absence of dual channel network; etc.

The compositions of the instant invention, alone or in combination with other catalytic components, may be distributed throughout an inert inorganic diluent which also may serve as a binder. Non-limiting examples of such diluents include aluminas, silicas, silica-aluminas, charcoal, pumice, magnesia, zirconia, kieselguhr, fullers' earth, silicon carbide, clays and other ceramics. In a preferred use of binders the instant zeolitic compositions are intimately mixed a finely divided, hydrous, refractory oxide of a difficulty reducible metal. The term "hydrous" is used to designate oxides having structural surface hydroxyl groups detectable by infrared analysis. The preferred oxides are alumina, silica, magnesia, beryllia, zirconia, titania, thoria, chromia, and combinations thereof such silica-alumina, silica-magnesia, and the like. Naturally occurring clays comprising silica and alumina may also be utilized, preferably after acid treatment. The metal oxide can be combined with the instant compositions as a hydrous sol or gel, an an anhydrous activated gel, a spray dried powder or a calcined powder. In one modification a sol or solution of the metal oxide precursor such as an alkali metal silicate or aluminate can be precipitated to form a gel in the presence of the compositions of the instant invention. When less hydrous form of the metal oxide are combined with the instant compositions, essentially any method of effecting intimate admixture of the components may by utilized. One such method is mechanical admixture, e.g., mulling, which involves admixing the instant compositions in the form of a powder with the slightly hydrous, finely divided form of the metal oxide. The diluent or binder may be added to the instant compositions at any point in their preparation, that is, before, during or after impregnation, drying and/or calcination. It may be advantageous to incorporate a binder, in particular alumina into the pores and/or the supercages of the zeolite prior to treatment with one or more metal compounds of Group IA, IIA, the Transition Metals or the Rare Earth Metals.

The ranges and limitations provided in the instant specification and claims are those which are believed to particularly point out and distinctly claim the instant invention. It is, however, understood that other ranges and limitations that perform substantially the same function in substantially the same manner to obtain the same or substantially the same result are intended to be within the scope of the instant invention as defined by the instant specification and claims.

The following illustrative embodiments are provided for illustration and are not to be construed as limiting the invention.

The following illustrative embodiments, which are also summarized in Table 3, are grouped according to typical Example types, described below. Table 3 also provides selected reference materials and comparative examples along with certain physical properties and basicity test results. Table 3 also provides data relative to compositions prepared similar to examples A through M and AA through QQ along with the variations used in starting zeolites, impregnating solutions, impregnating salts, impregnating conditions, resulting selected properties and basicity test results. It is apparent that many variations and extensions of these examples may be performed by one skilled in the art to achieve results within the scope of the instant invention. These examples are provided only to illustrate the invention, not to limit it.

Example A: Low Level Impregnation of Salt into Zeolite

50g Baylith CP-190 NaY zeolite was impregnated with 2.12g KOH (Aldrich) dissolved in 20cc water. The zeolite was mixed in a dish during impregnation, then dried 1 hour at 100 °C in a vacuum oven. This material is B21 in Table 3.

36g of the KOH/NaY (B21) was placed in a vycor tube in an upright furnace. With a slow nitrogen flow through the tube, the furnace was heated to 550 °C over a period of 30 minutes. After 1 hour, the furnace was turned off and allowed to cool, while still under flowing nitrogen. The catalyst was removed and labelled B22 in Table 3.

Example B: Calcination of Previously Impregnated Zeolite

84.07g Baylith CP-190 NaY zeolite was dried for 2 hours in a 190 °C vacuum oven with a low nitrogen bleed. 50g of this dried NaY was impregnated with 1.9g potassium carbonate (MCB ACS reagent) dissolved in 25cc water. The zeolite was mixed in a dish during impregnation, then dried at 100 °C in a vacuum oven with a low nitrogen bleed. The dried impregnated zeolite (labelled A1 in Table 3) was then stored in a dry box.

Three months later, 8.59g of the masterbatch material A1 was placed in a fritted tube in the dry box. This tube was brought out and placed in an upright heating mantle. The nitrogen flow was set at 90cc/min and the mantle was heated to 450 °C over a period of 40 minutes. After 116 minutes, the mantle was turned off and allowed to cool while still under flowing nitrogen. The cooled tube was sent into the dry box, where the zeolite (material B3 in Table 3) was unloaded and stored.

Example C: Impregnation with a High Loading, with Solvent Greater in Amount than Salt in the Impregnating Solution

20.02g 4A zeolite was impregnated with 4.53g sodium carbonate dissolved in 10cc water. The zeolite was mixed in a dish during impregnation, then dried 16 hours at 70 °C in a vacuum oven. The resulting material is indicated as B6 in Table 3.

10g of material B6 was placed in a fritted tube in an upright heating mantle. The nitrogen flow was set at 90cc/min and the mantle was heated to 450 °C over a period of 50 minutes. After 115 minutes, the mantle was cooled while still under flowing nitrogen. The resulting material is indicated as B7 in Table 3.

Example D: Zeolite Slurried in Salt Solution and Dried with the Solution Left in It

100cc potassium acetate solution (29.74g Aldrich acetic acid, potassium salt dissolved in 300cc water) was added to an Erlenmeyer flask containing 10.01g Baylith CP-190 NaY zeolite. This mixture was stirred at room temperature. After 1 hour, the mixture was filtered in a fritted vacuum funnel. The resulting paste was returned to the Erlenmeyer flask with a fresh 100cc of the potassium acetate solution. The solution was allowed to rest over the weekend, before being filtered again. Once again the paste was returned to the Erlenmeyer flask with a fresh 100cc of the potassium acetate solution. After stirring for approximately 24 hours, it was filtered a final time. The paste was dried for 20 hours at 100 °C in a vacuum oven. The dried zeolite was pressed in bags in the isostatic press at 20,000 psi for 2 minutes. It was then ground and sieved to 16-30 mesh. The resulting material is indicated as C4 in Table 3.

The dried zeolite C4 was placed in a vycor tube in an upright furnace. The nitrogen flow was set at 20cc/min, then the furnace was heated to 200° C over a period of 15 minutes. After 35 minutes at 200° C, the furnace was heated to 550° C over a period of 15 minutes. After 40 minutes, the heater was turned off and allowed to cool while still under flowing nitrogen. The vycor tube was sent into the dry box and stored for about 16 hours, at which time the tube was returned to the upright furnace. The nitrogen flow was set at 30 litres/hour, then the furnace was heated to 200° C over a period of 20 minutes. After 25 minutes the temperature was raised to 550° C, this took a period of 11 minutes. After 2 hours, the furnace was turned off and allowed to cool while still under flowing nitrogen. The resulting material is indicated as C5 in Table 3.

Example E: Exchange of Zeolite Followed by Impregnation

100cc 1M cesium acetate solution (76.4g Aldrich cesium acetate dissolved in 400cc Barnstead deionized water) was added to a flask containing 20.02g LZY-82 pellets (Union Carbide). After stirring for 16 hours, the liquid was decanted off the pellets. A fresh 100 cc aliquot of the 1M cesium acetate solution was added to the flask. After 2 hours, the liquid was decanted off. The addition, stirring 2 hours and decanting of liquid were repeated two more times. After the final decanting of the liquid, the pellets were vacuum filtered, then dried 85 minutes at 100° C in a vacuum oven. This is indicated as material C20 in Table 3.

20g of this dried material was impregnated with 1.82g cesium acetate (Aldrich) dissolved in 1.8cc water (Barnstead deionized). The zeolite was mixed in a dish during impregnation and allowed to rest for 25 minutes, then dried 16 hours at 100° C in a vacuum oven, the resulting material is indicated C21 in Table 3.

The dried zeolite C21 was placed in a vycor tube in an upright furnace. The nitrogen flow was set at 15 litres/hour and the furnace heated to 200° C. After holding at 200° C for 25 minutes, the temperature was increased to 550° C. After 2 hours, the furnace was turned off and allowed to cool while still under flowing nitrogen. The resulting material is indicated as C22 in Table 3.

Example F: Impregnation with Amount of Salt Greater than Amount of Solvent in Solution

35.0g LZY-52 pellets were dried for 2 hours at 160° C in a vacuum oven. 2.08g of this dried LZY-52 was impregnated with 1.04g of potassium acetate solution (20.38g Aldrich potassium acetate dissolved in 10cc Barnstead deionized water). The LZY-52 was mixed in a vial during impregnation, then dried 16 hours at 160° C in a vacuum oven. The dried material was placed in a vycor tube in an upright furnace. The nitrogen flow was set at 400cc/min and the furnace was heated to 200° C over a period of 20 minutes. After 25 minutes at 200° C, the temperature was raised (over a period of 12 minutes) to 550° C. After 2 hours, the furnace was turned off and allowed to cool while still under flowing nitrogen. 16 hours later, the nitrogen was turned off and an air (dried over Drierite) flow was stated. The air flow was set at 450cc/min and the furnace was heated to 200° C. After 21 minutes at 200° C, the temperature was raised to 550° C. After 2 hours, the temperature was 600° C. After 50 more minutes, the flow rate of air was reduced to 200cc/min. After an additional 1.5 hours at 600° C, the air flow was blocked , nitrogen flow of 100cc/min was begun, and the heater turned off and allowed to cool. The resulting material is indicated as C24 in Table 3.

Example G: Impregnation with Pure Water on Zeolite Previously Impregnated with Salt to Distribute the Salt

Natural erionite was dried 1 hour at 150° C in a vacuum oven. 1.05g of this dried erionite was impregnated with 2.02g potassium oxalate solution (16.12g potassium oxalate dissolved in 50cc Barnstead deionized water). The erionite was mixed in a vial during impregnation, then dried 105 minutes at 150° C in a vacuum oven. Upon removing from the vacuum oven, .60cc Barnstead water (.5cc water/ g of catalyst) was added. The mixture was allowed to rest for 20 minutes, then dried 1 hour at 150° C in a vacuum oven. 0.6cc Barnstead water was added to the material which then left for 20 minutes, and was dried 1 hour at 150° C in a vacuum oven.

The dried material was placed in a vycor tube in an upright furnace. The nitrogen flow was set at 350cc/min, and the furnace was heated to 200° C. After 25 minutes, the furnace temperature was raised to 550° C. After 2 hours at 500° C, the furnace was turned off and allowed to cool, while still under flowing nitrogen. This material is listed under D7 in Table 3.

Example H: Slurrying the Zeolite Followed by Washing

Method of preparation of the materials referred to under E9 in Table 3:

40cc 2M potassium carbonate solution (138.22g MCB potassium carbonate dissolved in 500cc Barnstead deionized water) was added to a flask containing 4.01g ammonium mordenite (dried 2 hours at 150 °C in a vacuum oven). After stirring 1 hour, this suspension was filtered and the solid washed with two 50cc aliquots of Barnstead deionized water. The paste was dried 1 hour at 150 °C in a vacuum oven. The dried zeolite was returned to the flask with 40cc 2M potassium carbonate solution. After stirring for 1 hour, it was filtered and then washed with two 50cc aliquots of Barnstead deionized water. The paste was dried 1 hour at 150 °C in a vacuum oven.

Example I: Molten Salt Impregnation

209g Baylith CP-190 NaY (out of the can) was stirred with 2 litres of Barnstead deionized water. After 2 hours, it was filtered. An additional 400cc Barnstead water was poured through the funnel. The zeolite was then dried 16 hours at 150 °C in a vacuum oven.

0.87g cesium acetate was heated to 200 °C in a 250 ml 3-neck round bottom flask equipped with a condenser, a thermometer, a stirring bar and a heating mantle. At 200 °C all the cesium acetate appeared to be a fluid. At this point, 8.63g of the washed NaY zeolite was added to the flask. The solid was stirred in the flask, and the temperature raised to 220 °C. After 70 minutes, the heat was turned off and the flask allowed to cool. The product is indicated as F1 in Table 3. After calcination in flowing nitrogen at 550 °C the material referred to as F2 in Table 3 was obtained.

Example J: Impregnation with Solvent to Redistribute Salt on Previously Calcined Material

30.01g LZY-82 (Lot # 9661796179 Union Carbide powder) zeolite was impregnated with 25.09g lithium acetate solution (20.75g lithium acetate dihydrate dissolved in 22cc Barnstead deionized water). The zeolite was mixed in a dish during impregnation, allowed to rest for 10 minutes, then dried 30 minutes at 150 °C in a vacuum oven. The zeolite was then impregnated with an additional 13.39g lithium acetate solution. The impregnation was done in a dish, then the zeolite dried 33 minutes at 150 °C in a vacuum oven. This was material referred to as F11 in Table 3. 10cc Barnstead deionized water was added to the dried zeolite. After 15 minutes, the zeolite was dried 30 minutes at 150 °C in a vacuum oven. 35.85g of the impregnated zeolite was recovered, material referred to as F12 in Table 3.

17.93g of F12 was placed in a vycor tube in an upright furnace. Nitrogen flow was set at 250cc/min, then the furnace heated to 200 °C. After 25 minutes, the temperature was raised to 550 °C. After 2 hours at 550 °C, the furnace was turned off and allowed to cool while still under flowing nitrogen. The product material is referred to as F13 in Table 3.

5cc Barnstead water was added to 17.92g of F12 in a dish. After 25 minutes, it was dried 65 minutes at 150 °C in a vacuum oven. An additional 5cc water was added to the zeolite. After 25 minutes, it was dried 1 hour at 150 °C in a vacuum oven. An additional 5cc water was added to the zeolite. After 1 hour, it was dried 66 hours at 150 °C in a vacuum oven. The resulting was material is indicated in Table 3 as F14.

5cc Barnstead deionized water was added to 14g of F13. This was dried 1 hour at 150 °C in a vacuum oven, yielding a material referred to as F15 in Table 3. The dried material was placed in a vycor tube in an upright furnace. The nitrogen flow rate was set at 200cc/min and the furnace was heated to 200 °C. After 23 minutes, the furnace temperature was increased to 550 °C. After 2 hours at 550 °C, the furnace was turned off and allowed to cool, while still under flowing nitrogen. The product is referred to as F16 in Table 3.

Example K: Impregnation with a Catalytic Metal Salt Followed by Impregnation with Base Progenitor

31.15g Baylith CP-190 NaY zeolite was impregnated with 5g ruthenium nitrosyl nitrate (Alfa) dissolved in 18cc water. The zeolite was mixed in a dish during impregnation, and allowed to rest for 29 minutes, then dried 61 minutes at 100 °C in a vacuum oven. The product is referred to as G1 in Table 3.

33.52g of G1 was placed in a vycor tube in an upright furnace. Over a period of 23 minutes, the furnace was heated to 550° C under flowing nitrogen. After 1 hour, the furnace was cooled to 500° C, the nitrogen flow was stopped and an air flow was begun. After 30 minutes, the furnace was turned off, the air was stopped , nitrogen flow was restarted, and the tube was allowed to cool. The product is referred to as G2 in Table 3.

12.01g of G2 was impregnated with 5.50g potassium carbonate dissolved in 6cc water. The zeolite was mixed in a dish during impregnation, allowed to rest for 49 minutes, then dried 64 minutes at 100° C in a vacuum oven. The resulting material is indicated in Table 3 as G3.

The dried material was placed in a vycor tube in an upright furnace. With nitrogen flowing, the furnace was heated to 550° C. After 1 hour, the furnace was turned off and the tube allowed to cool, while still under flowing nitrogen. The product material is referred to as G4 in Table 3.

Example L: Impregnation with Alcoholic Solution

30.73g K-L zeolite was stirred with 300cc Barnstead deionized water. After 1 hour of stirring, the mixture was filtered. an additional 200cc water was poured through the funnel. The paste was dried 1 hour at 150° C in a vacuum oven.

12.61g washed K-L was impregnated with 3.13g potassium methoxide dissolved in 8cc methanol. The zeolite was mixed in a dish during impregnation, allowed to rest for 15 minutes, then dried 16 hours at 150° C in a vacuum oven. The resulting material is referred to as F33 in Table 3.

The material F33 was placed in a vycor tube in an upright furnace. The nitrogen flow rate was set at 250cc/min and the furnace was heated to 200° C over a period of 6 minutes. After 25 minutes, the furnace temperature was increased to 550° C. After 2 hours, the furnace was turned off and allowed to cool, while still under flowing nitrogen. The product material is referred to as F34 in Table 3.

Example M: Impregnation with Non Hydroxylic Solution

5.04g washed NaY zeolite (washed as in Example I) was impregnated with 1.04g lithium acetate dissolved in 3cc dimethylsulphoxide. The zeolite was mixed in a dish during impregnation, allowed to rest for 10 minutes, then dried 18 hours and 19 minutes in a 200° C vacuum oven. The resulting material is indicated in Table 3 as F37.

The dried material F37 was placed in a vycor tube in an upright furnace. The nitrogen flow rate was set at 225cc/min and the furnace was heated to 200° C over a period of 10 minutes. After 40 minutes, the furnace temperature was raised to 550° C. After 2 hours at 550° C, the furnace was turned off and the tube allowed to cool while still under flowing nitrogen. The resulting product material is referred to as F38 of Table 3.

<u>Table 3</u>

Illustrative Embodiments, part A

| --Description--- | ----Materials used----- | | | | -----Conditions----- | | | -Structure- | |
|---|---|---|---|---|---|---|---|---|---|
| CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| REFERENCE MATERIALS: | | | | | | | | | |
| KOH pellets | -- | -- | --- | --- | --- | - | - | --- | --- |
| Shell Silica Spheres | -- | -- | --- | --- | --- | - | - | --- | --- |
| Sodium Amide | -- | -- | --- | --- | --- | - | - | --- | --- |
| NaY, washed | -- | -- | --- | --- | 150 | - | - | 100 | 714 |
| $NH_4$+ Mordenite | -- | -- | --- | --- | --- | - | - | 100 | 362 |
| Na Mordenite, washed | -- | -- | --- | --- | --- | - | - | 97.2 | 358 |
| Water (Barnstead 20 Mohm) | | -- | --- | --- | --- | - | - | --- | --- |
| MgO | -- | -- | --- | --- | --- | - | - | --- | --- |
| LiOH | -- | -- | --- | --- | --- | - | - | --- | --- |
| CaO (quicklime) | -- | -- | --- | --- | --- | - | - | --- | --- |
| K/L zeolite | -- | -- | --- | --- | --- | - | - | --- | --- |
| LZY82 pellets | -- | -- | --- | --- | --- | - | - | 60.8 | 556 |
| LZY62 pellets | -- | -- | --- | --- | --- | - | - | 73.5 | 624 |
| LZY52 pellets | -- | -- | --- | --- | --- | - | - | 73.7 | 603 |
| LZY82 powder | -- | -- | --- | --- | --- | - | - | 109.8 | 550 |

Table 3 (cont'd)

Illustrative Embodiments, part A

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| REFERENCE MATERIALS: | | | | | | | | | |
| SDUSY | -- | -- | --- | --- | --- | - | - | 85.6 | 595 |
| $NH_4^+$ Ferrierite | -- | -- | --- | --- | --- | - | - | 107.1 | nd |
| Natural Erionite | -- | -- | --- | --- | 150 | - | - | 103.3 | nd |
| Natural Mordenite | -- | -- | --- | --- | 150 | - | - | 85.1 | 264 |
| Natural Chabazite | -- | -- | --- | --- | 150 | - | - | 39.8 | 356 |
| NaX | -- | -- | --- | --- | --- | - | - | nd | nd |
| 4A (Na/A zeol.) | -- | -- | --- | --- | --- | - | - | nd | 15 |
| 5A (Ca/A zeol.) | -- | -- | --- | --- | --- | - | - | 1.3.1 | 456 |
| K methoxide | -- | -- | --- | --- | --- | - | - | --- | --- |
| $K_2CO_3$ | -- | -- | --- | --- | --- | - | - | --- | --- |

Table 3 (cont'd)

Illustrative Embodiments, part A

| --Description--- | ----Materials used----- | | | | -----Conditions----- | | | -Structure- | |
|---|---|---|---|---|---|---|---|---|---|
| CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| REFERENCE MATERIALS: | | | | | | | | | |
| K oxalate | -- | -- | --- | --- | --- | - | - | --- | --- |
| $KNO_3$ | -- | -- | --- | --- | --- | - | - | --- | --- |
| K acetate | -- | -- | --- | --- | --- | - | - | --- | --- |
| Cs acetate | -- | -- | --- | --- | --- | - | - | --- | --- |
| Li acetate | -- | -- | --- | --- | --- | - | - | --- | --- |
| $CaCO_3$ | -- | -- | --- | --- | --- | - | - | --- | --- |
| $Ca(OH)_2$ (lime) | -- | -- | --- | --- | --- | - | - | --- | --- |
| $Ca(NO_3)_2$ | -- | -- | --- | --- | --- | - | - | --- | --- |
| Ca acetate | -- | -- | --- | --- | --- | - | - | --- | --- |
| Mg acetate | -- | -- | --- | --- | --- | - | - | --- | --- |
| Ba acetate | -- | -- | --- | --- | --- | - | - | --- | --- |
| Sr acetate | -- | -- | --- | --- | --- | - | - | --- | --- |
| Ba hydroxide | -- | -- | --- | --- | --- | - | - | --- | --- |
| Sr hydroxide | -- | -- | --- | --- | --- | - | - | --- | --- |
| Mg hydroxide | -- | -- | --- | --- | --- | - | - | --- | --- |

Table 3 (cont'd)

Illustrative Embodiments, part A

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| REFERENCE MATERIALS: | | | | | | | | | | |
| Illustrative Embodiments of Basic Zeolites: | | | | | | | | | | |
| A1 | $K_2CO_3$/NaY | $H_2O$ | 25 | 1.9 | 50.01 | 100 | I | A | 105 | 1.5 |
| A2 | $K_2CO_3$/NaY | Same as A1, | | calcined | | 460 | I | B | 110 | 1.5 |
| A3 | $K_2CO_3$/NaY | Same as A1, | | calcined | | 250 | I | B | 107 | 1.47 |
| A4 | $K_2CO_3$/NaY | Same as A1, | | calcined | | 350 | I | B | 108 | 1.51 |

Table 3

Illustrative Embodiments, Part A (continued)

| --Description--- | --Structure- | | ------------Basicity Resulting------------ | | | | |
|---|---|---|---|---|---|---|---|
| CATALYST System Designation (Salt/Zeolite) | n-C8 wet-ness cc/g f) j) | Pore vol. cc/g g) | pH 1% aq. h) | --------- INDICATOR COLORS --(i)------ 4-nitro-aniline /DMSO | 4-chloro aniline /DMSO | 4-nitro-aniline /benzene | 4-chloro-aniline /benzene |
| REFERENCE MATERIALS: | | | | | | | |
| KOH pellets | ---- | ---- | 14 | blue | beige | pinkish/ lavender | clear |
| Shell Silica Spheres | ---- | ---- | 6 | yellow | beige | yellow | clear |
| Sodium Amide | ---- | ---- | 14 | dk blue | olive drab | dk blue | black |
| NaY, washed | ---- | 0.37 | 6 | yellow | nd | nd | cream |
| $NH_4$+ Mordenite | ---- | 0.22 | 6 | yellow | nd | nd | cream |
| Na Mordenite, washed | ---- | 0.19 | 7 | yellow | nd | nd | cream |
| Water (Barnstead 20Mohm) | - | ---- | 6 | ---- | ---- | ---- | ----- |
| MgO | ---- | ---- | 10 | yellow | nd | nd | cream |
| LiOH | ---- | ---- | 13 | green | nd | nd | cream |
| CaO (quicklime) | ---- | ---- | 13 | lt green | white | milky-yellow | cream |
| K/L zeolite | ---- | ---- | 7 | lt lime grn | cream | yellow | white |
| LZY82 pellets[p] | ---- | 0.396 | 6 | yellow | nd | nd | cream |
| LZY62 pellets | ---- | 0.395 | 5 | yellow | nd | nd | cream |
| LZY52 pellets | ---- | 0.411 | 6 | yellow/grn | nd | nd | cream |

Table 3

Illustrative Embodiments, Part A (continued)

| --Description--- | --Structure- | | ------------Basicity Resulting------------ | | | | |
|---|---|---|---|---|---|---|---|
| CATALYST | n-C8 | Pore | pH | --------- INDICATOR COLORS --(i)------ | | | |
| System | wet- | vol. | 1% | 4-nitro- | 4-chloro | 4-nitro- | 4-chloro- |
| Designation | ness | cc/g | aq. | aniline | aniline | aniline | aniline |
| (Salt/Zeolite) | cc/g f) j) | g) | h) | /DMSO | /DMSO | /benzene | /benzene |
| REFERENCE MATERIALS: | | | | | | | |
| LZY82 powder | 0.524 | 0.398 | 6 | yellow | nd | nd | cream |
| SDUSY[q] | 0.590 | 0.54 | 6 | yellow | nd | nd | cream |
| $NH_4$+ Ferrierite | 0.696 | ---- | 5 | yellow | nd | nd | white |
| Natural Erionite | nd | ---- | 6 | golden | nd | nd | sl orng |
| Natural Mordenite | 1.03 | 0.409 | 6 | golden | nd | nd | cream |
| Natural Chabazite | nd | 0.164 | 7 | yellow | nd | nd | cream |
| NaX | 0.423 | nd | 7 | yellow | nd | nd | cream |
| A (Na/A zeol.) | 0.171 | 0.065 | 7 | yellow | nd | nd | cream |
| 5A (Ca/A zeol.) | 0.263 | 0.321 | 7 | yellow | nd | nd | cream |
| K methoxide | ---- | ---- | 14 | dk brown | nd | nd | off-white |
| $K_2CO_3$ | | -- | 11 | dk green | | | cream |
| K oxalate | | -- | 7 | yellow | nd | nd | cream |

Table 3

Illustrative Embodiments, Part A (continued)

| --Description--- CATALYST System Designation (Salt/Zeolite) | --Structure-- n-C8 wetness cc/g f) j) | --Structure-- Pore vol. cc/g g) | Basicity Resulting pH 1% aq. h) | INDICATOR COLORS (i) 4-nitro-aniline /DMSO | INDICATOR COLORS (i) 4-chloro aniline /DMSO | INDICATOR COLORS (i) 4-nitro-aniline /benzene | INDICATOR COLORS (i) 4-chloro-aniline /benzene |
|---|---|---|---|---|---|---|---|
| REFERENCE MATERIALS: | | | | | | | |
| $KNO_3$ | | -- | 6 | yellow | nd | nd | cream |
| K acetate | | -- | 7 | yellow | nd | nd | cream |
| Cs acetate | | -- | 7 | yellow/grn | nd | nd | cream |
| Li acetate | | -- | 8 | green | nd | nd | cream |

Table 3

Illustrative Embodiments, Part A (continued)

| --Description--- | --Structure- | | ------------Basicity Resulting------------ | | | | |
|---|---|---|---|---|---|---|---|
| | | | | --------- INDICATOR COLORS --(i)------ | | | |
| CATALYST System Designation (Salt/Zeolite) | n-C8 wet-ness cc/g f) | Pore vol. cc/g g) | pH 1% aq. h) | 4-nitro-aniline /DMSO | 4-chloro aniline /DMSO | 4-nitro-aniline /benzene | 4-chloro-aniline /benzene |
| REFERENCE MATERIALS: j) | | | | | | | |
| $CaCO_3$ | ---- | ---- | 7 | yellow | cream | pale-yellow | cream |
| $Ca(OH)_2$ (lime) | ---- | ---- | 14 | dk green | white | dk gray | cream |
| $Ca(NO_3)_2$ | ---- | ---- | 7 | yellow | nd | nd | lt bluish |
| Ca acetate | ---- | ---- | 7 | yellow | nd | nd | white |
| Mg acetate | ---- | ---- | 7 | yellow | nd | nd | white |
| Ba acetate | ---- | ---- | 7 | yellow | cream | yellow | white |
| Sr acetate | ---- | ---- | 7 | yellow | white | pale-yellow | white |
| Ba hydroxide | ---- | ---- | 13 | dk green | nd | nd | cream |
| Sr hydroxide | ---- | ---- | | | | | |
| Mg hydroxide | ---- | ---- | 8 | lime green | nd | nd | cream |

Table 3

Illustrative Embodiments, Part A (continued)

| --Description--- | --Structure- | | ------------Basicity Resulting------------ | | | | |
|---|---|---|---|---|---|---|---|
| CATALYST System Designation (Salt/Zeolite) | n-C8 wetness cc/g f) | Pore vol. cc/g g) | pH 1% aq. h) | --------- INDICATOR COLORS --(i)------ 4-nitro-aniline /DMSO | 4-chloro aniline /DMSO | 4-nitro-aniline /benzene | 4-chloro-aniline /benzene |
| Illustrative Embodiments of Basic Zeolites: | | | | | | | |
| A1 $K_2CO_3$/NaY | | -- | -- | dk green | cream | sl orng yell | cream |
| A2 $K_2CO_3$/NaY | | -- | -- | dk blue | lt beige | muddy grn/orng | lt pink |
| A3 $K_2CO_3$/NaY | | -- | -- | dk blue | lt beige | orng/yell | cream |
| A4 $K_2CO_3$/NaY | | -- | -- | dk green | lt beige | sl bwn in orng/yell | cream |

Table 3 Illustrative Embodiments, Part A (Continued)

Footnotes:

a) Maximum temperature used in drying (calcining) the particular material, in degrees Centigrade.

b) General method of preparation. I = Impregnation S = Slurry (Exchange in a wash of salt solution) M = impregnation with molten salt.

c) Closest example described in text above, by code letter.

d) Per cent crystallinity by X-ray diffraction comparison to best available standard sample material by ASTM D3906-80 for faujasites, and a modification of this method for the other zeolites.

e) Surface area in square meters/gram by nitrogen absorption, via the two point surface area method on a Digisorb 2500 from Micromeritics using the Micromeritics program table for this machine.

f) Amount of n-octane, cc/g, necessary to make the catalyst powder appear moist.

g) Pore volume in cubic centimeters per gram, determined by nitrogen absorption via the equipment described in e).

h) pH of a one percent suspension of the catalyst in deionized (Barnstead, 20 meg ohm) water, read with pH paper after 20 minutes.

i) Colour of a suspension of about 60mg of catalyst in about .1 cc of a 10% weight solution of the indicator solution listed, by a modification of the method of Tanabe (in Solid Acids and Bases, Academic Press, 1970). The leftmost system probably indicates pH of about 16 or more, while the rightmost system should indicate pH of 27 or more, according to Tanabe. No entry means no colour change relative to the indicator solution itself. (orng = orange, orangish; yell = yellow; grn = green, greenish; dk = dark; bwn = brown, brownish; lt = light; sl = slight; ex = extremely; dp = deep; w/ = with; nd = not determined; liq = liquid purpe purple, lvnder-lavender).

j) KOAc = potassium acetate; CsOAc = cesium acetate; LZY--plt = Linde zeolite Y type -- pellets from Union Carbide Corp.; 4A = sodium zeolite A type -- from Aldrich; 5A = calcium zeolite A type - from Davison; VUSY = very ultra stable zeolite Y; USY = ultra stable zeolite Y.

k) "I" = impregnation with pure water followed by drying to redistribute the loading of salt previously impregnated, as in method G.

l) WshNaMord = washed Na mordenite, thoroughly washed with purified Barnstead water until pH of solid is constant at 6.

m) LZY82pwdr = hydrostatically pressed (20,000 to 30,000 psi) and sieved 16-30 mesh powder.

n) The molten salt was impregnated into the washed NaY zeolite at 200 degrees Centigrade to 220 degrees Centigrade.

o) Black due to the colour of ruthenium. Note colour changes with other metals and the ability of the indicator to shade and bury the metal species' colour.

p) Pellets contain approximately 20% $Al_2O_3$.

q) Super Dealuminated USY; $SiO_2/Al_2O_3$ ~25.

Table 3

Illustrative Embodiments, Part B.

(Footnotes are those of Part A)

| --Description-- | | -----Materials used----- | | | | -----Conditions----- | | | ---Structure--- | | |
|---|---|---|---|---|---|---|---|---|---|---|---|
| #: & & & | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) | $n-C_8$ wet-ness cc/g f) |
| B1 | $K_2CO_3$/NaY | $H_2O$ | 25 | 15 | 25.39 | 100 | I | C | 33 | -- | ---- |
| B2 | $K_2CO_3$/NaY | ------(same as A1)----- | | | | 250 | I | B | 75 | -- | ---- |
| B3 | $K_2CO_3$/NaY | ------(same as A1)----- | | | | 450 | I | B | 78 | -- | ---- |
| B4 | $K_2CO_3$/NaY | ------(same as B1)----- | | | | 250 | I | C | 19 | -- | ---- |
| B5 | $K_2CO_33$/NaY | ------(same as B1)----- | | | | 450 | I | C | 11 | -- | ---- |
| B6 | $Na_2CO_3$/4A j) | $H_2O$ | 10 | 4.53 | 20.02 | 70 | I | C | -- | -- | ---- |
| B7 | $Na_2CO_3$/4A | ------(same as B6)----- | | | | 450 | I | C | -- | -- | ---- |
| B8 | $K_2CO_3$/NaY | $H_2O$ | 50 | 3.84 | 100.5 | 100 | I | A | 84 | -- | ---- |
| B9 | $K_2CO_3$/NaY | $H_2O$ | 75 | 5.76 | 150 | 100 | I | A | 88.4 | -- | ---- |
| B10 | $K_2CO_3$/NaY | $H_2O$ | 61 | 36.53 | 61.7 | 100 | I | C | 40 | -- | ---- |
| B11 | $K_2CO_3$/5A j) | $H_2O$ | 60 | 45.53 | 100 | 500 | I | C | -- | -- | ---- |
| B12 | KOH/NaY | $H_2O$ | 6000 | 168 | 50.6 | 100 | S | D | -- | -- | ---- |
| B13 | KOH/NaY | $H_2$ | 3000 | 168 | 50.4 | 100 | S | D | -- | -- | ---- |

Table 3 (cont'd)

Illustrative Embodiments, Part B.
(Footnotes are those of Part A)

| #: | CATALYST System Designation (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
|---|---|---|---|---|---|---|---|---|---|---|---|
| --Description-- | | -----Materials used----- | | | | -----Conditions----- | | | ---Structure--- | | |
| B14 | $K_2CO_3$/NaY | $H_2O$ | 50 | 45.6 | 100 | 100 | I | C | 89.5 | -- | ---- |
| B15 | $K_2CO_3$/NaY | $H_2O$ | --(same as B14)---- | | | 550 | I | C | 89.3 | -- | ---- |
| B16 | $NaNO_3/NH_4$-USY | $H_2O$ | 1000 | 171 | 101 | - | S | E | | | |
| | | $H_2O$ | 1000 | 0 | 101 | - | wash | | | | |
| | | $H_2O$ | 1000 | 171 | 101 | - | S | | | | |
| | | $H_2O$ | 1000 | 0 | 101 | 100 | wash | | -- | 757 | ---- |
| B17 | $KNO_3$/NaY | $H_2O$ | 20 | 16.64 | 50.8 | 100 | I | C | 67.1 | -- | ---- |
| B18 | $KNO_3$/NaY | $H_2O$ | --(same as B17)-- | | | 550 | I | C | 56.2 | -- | ---- |
| B19 | KOH/NaY | $H_2O$ | 20 | 20.1 | 50 | 100 | I | C | 11.4 | -- | ---- |
| B20 | KOH/NaY | $H_2O$ | --(same as B19)-- | | | 550 | I | C | 0 | -- | ---- |
| B21 | KOH/NaY | $H_2O$ | 20 | 2.12 | 50 | 100 | I | A | 84.3 | -- | ---- |
| B22 | KOH/NaY | $H_2O$ | --(same as B21)-- | | | 550 | I | A | 87.9 | -- | ---- |
| B23 | K oxalate/NaY | $H_2O$ | 70 | 23.4 | 50 | 100 | I | C | 37.3 | -- | ---- |
| B24 | K oxalate/NaY | $H_2O$ | --(same as B23)-- | | | 550 | I | C | 9 | -- | ---- |

Table 3

Illustrative Embodiments, Part B. (Continued)

(Footnotes are those of Part A)

| --Description--- #: CATALYST & System & Designation & (Salt/Zeolite) | | Conditions Temp. a) | Structure Pore vol. cc/g g) | pH 1% aq. h) | Basicity Resulting INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|---|
| B1 | $K_2CO_3$/NaY | 100 | -- | 11 | Kelly grn | cream | dirty yell | cream |
| B2 | $K_2CO_3$/NaY | 250 | -- | 9 | Blue grn | lt pink | orange | cream |
| B3 | $K_2CO_3$/NaY | 450 | -- | 8 | dp blue grn | amber | yell bwn | cream |
| B4 | $K_2CO_3$/NaY | 250 | -- | -- | ex dp blue green | amber | olive drab | cream |
| B5 | $K_2CO_3$/NaY | 450 | -- | 9 | dp blue/dp green | lt pink | opaque blackish grn | cream |
| B6 | $Na_2CO_3$/4A | 70 | -- | -- | | | | |
| B7 | $Na_2CO_3$/4A | 450 | -- | -- | | | | |

Table 3

Illustrative Embodiments, Part B. (Continued)

(Footnotes are those of Part A)

| --Description--- | Conditions | Structure | ---Basicity Resulting--- | | | | |
|---|---|---|---|---|---|---|---|
| #: CATALYST & System & Designation & (Salt/Zeolite) | Temp. a) | Pore vol. cc/g g) | pH 1% aq. h) | ---INDICATOR COLORS---(i)--- 4-nitroaniline /DMSO | 4-chloro aniline /DMSO | 4-nitroaniline /benzene | 4-chloroaniline /benzene |
| B8 $K_2CO_3$/NaY | 100 | -- | 10 | green | nd | nd | white |
| B9 $K_2CO_3$/NaY | 100 | -- | 10 | dk lime grn | nd | nd | white |
| B10 $K_2CO_3$/NaY | 100 | -- | 11 | dk grn blue | nd | nd | white |
| B11 $K_2CO_3$/5A | 500 | -- | -- | | | | |

Table 3 (cont'd)

Illustrative Embodiments, Part B. (Continued)

(Footnotes are those of Part A)

| --Description--- #: CATALYST & System & Designation & (Salt/Zeolite) | | Conditions Temp. a) | Structure Pore vol. cc/g) | pH 1% aq. h) | Basicity Resulting INDICATOR COLORS (i) 4-nitroaniline /DMSO | 4-chloroaniline /DMSO | 4-nitroaniline /benzene | 4-chloroaniline /benzene |
|---|---|---|---|---|---|---|---|---|
| B12 | KOH/NaY | 100 | -- | 9.5 | green | nd | nd | white |
| B13 | KOH/NaY | 100 | -- | 8 | dk green | nd | nd | white |
| B14 | $K_2CO_3$/NaY | 100 | -- | 10 | dk green | nd | nd | white |
| B15 | $K_2CO_3$/NaY | 550 | -- | 10 | nd | nd | nd | nd |
| B16 | $NaNO_3/NH_4$-USY | - - - 100 | -- | 7 | yellow | nd | nd | white |
| B17 | $KNO_3$/NaY | 100 | -- | 7 | yellow | nd | nd | cream |
| B18 | $KNO_3$/NaY | 550 | -- | 7 | lt yell grn | nd | nd | lt pink |
| B19 | KOH/NaY | 100 | -- | 12 | | | | |
| B20 | KOH/NaY | 550 | -- | 11 | dk blue grn | nd | nd | dk cream |

Table 3 (cont'd)

Illustrative Embodiments, Part B. (Continued)

(Footnotes are those of Part A)

| --Description--- #: CATALYST & System & Designation & (Salt/Zeolite) | Conditions Temp. a) | Structure Pore vol. cc/g g) | pH 1% aq. h) | Basicity Resulting: INDICATOR COLORS (i) 4-nitroaniline /DMSO | 4-chloro aniline /DMSO | 4-nitroaniline /benzene | 4-chloroaniline /benzene |
|---|---|---|---|---|---|---|---|
| B21 KOH/NaY | 100 | -- | 9 | | | | |
| B22 KOH/NaY | 550 | -- | 10 | very dk grn; blue green | nd | nd | lt amber w/ sl lavender |
| B23 K oxalate/NaY | 100 | -- | 8 | | | | |
| B24 K oxalate/NaY | 550 | -- | 11 | very dk grn solid; blue liq | nd | nd | purple & gray |

Table 3

Illustrative Embodiments, Part C.

(Footnotes are those of Part A)

| --Description--- #: CATALYST & System & Designation & (Salt/Zeolite) | | ----Materials used----- Solvent | Solv. Wt. | Salt Wt. | Zeol. Wt. | -----Conditions--- Temp. a) | Method b) | Example c) | -----Structure-- XRD % xtal d) | S.A. sq.m. /g e) | $n-C_8$ wetness cc/g f) |
|---|---|---|---|---|---|---|---|---|---|---|---|
| C1 | $K_2CO_3$/NaY | $H_2O$ | 10 | 11.6 | 24.9 | 100 | I | C | 37.4 | 129 | ---- |
| C2 | $K_2CO_3$/NaY | $H_2O$ | -(same as C1)-- | | | 550 | I | C | 12 | 74 | ---- |
| C3 | $K_2CO_3$/NaY | $H_2O$ | 14 | 4.02 | 25 | 580 | I | C | 1 | 38 | ---- |
| C4 | K acetate/NaY | $H_2O$ | 300 | 29.8 | 10 | 100 | S | D | -- | -- | ---- |
| C5 | K acetate/NaY | $H_2O$ | --(same as C4)-- | | | 550 | S | D | 16 | -- | ---- |
| C6 | K acetate/NaY | $H_2O$ | 4 | 0.12 | 10.1 | 100 | I | A | | | |
| C7 | K acetate/NaY | $H_2O$ | --(same as C6)-- | | | 550 | I | A | 96 | -- | ---- |
| C8 | K acetate/NaY | $H_2O$ | 4 | 0.22 | 10.1 | 100 | I | A | | | |
| C9 | K acetate/NaY | $H_2O$ | --(same as C8)-- | | | 550 | I | A | 97 | -- | ---- |

Table 3

Illustrative Embodiments, Part C.

(Footnotes are those of Part A)

| --Description--- | | ----Materials used----- | | | -----Conditions--- | | | -----Structure-- | | |
|---|---|---|---|---|---|---|---|---|---|---|
| #: CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| C10 K acetate/NaY | $H_2O$ | 4 | 1.2 | 10 | 100 | I | C | | | |
| C11 K acetate/NaY | $H_2O$ | --(same as C10)- | | | 550 | I | C | 37 | -- | ---- |
| C12 K acetate/NaY | $H_2O$ | 4 | 2.02 | 10 | 100 | I | C | | | |
| C13 K acetate/NaY | $H_2O$ | --(same as C12)- | | | 550 | I | C | 15 | -- | ---- |
| C14 K acetate/KL | $H_2O$ | 10 | 22.1 | 20 | 100 | I | C | | | |

Table 3 (cont'd)

Illustrative Embodiments, Part C.

(Footnotes are those of Part A)

| --Description--- | ----Materials used----- | | | | -----Conditions--- | | | -----Structure-- | | |
|---|---|---|---|---|---|---|---|---|---|---|
| #: CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| C15 K acetate/KL | | ---(as above)--- | | | 550 | I | C | 29 | 74 | ---- |
| C16 K acetate/KL | $H_2O$ | 10 | 2.24 | 20.1 | 100 | I | C | | | |
| C17 K acetate/KL | $H_2O$ | ---(as C16------ | | | 550 | I | C | 81 | --- | ---- |
| C18 KOAc/LZY82plt(j) | $H_2O$ | 3.4 | 8.6 | 21.2 | 100 | I | C | 31 | 145 | ---- |
| C19 KOAc/LZY82plt | $H_2O$ | ---(as C18)----- | | | 550 | I | C | 14 | 135 | ---- |
| C20 CsOAc/LZY82plt | $H_2O$ | 400 | 76.4 | 20.2 | 100 | S | D | 24 | 371 | ---- |
| C21 CsOAc/LZY82plt | $H_2O$ | 1.8 | 1.82 | 20 | 100 | I/S | E | 32 | 347 | ---- |
| C22 CsOAc/LZY82plt | $H_2O$ | -(sample above)- | | | 550 | I/S | E | 24 | 370 | ---- |
| C23 CsOAc/LZY52plt | $H_2O$ | 0.74 | 1.48 | 2.04 | 550 | I | F | 21 | --- | ---- |
| C24 KOAc/LZY52plt | $H_2O$ | 0.34 | 0.70 | 2.08 | 550 | I | F | 42 | --- | ---- |
| C25 KOAc/LZY82plt | $H_2O$ | 0.67 | 1.37 | 2.02 | 550 | I | F | 3 | --- | ---- |
| C26 CsOAc/LZY62plt | $H_2O$ | 0.72 | 1.43 | 2.01 | 550 | I | F | 11.2 | --- | ---- |
| C27 CsOAc/LZY62plt | $H_2O$ | 1.02 | 2.04 | 2.00 | 550 | I | F | 7.1 | --- | ---- |
| C28 KOAc/LZY62plt | $H_2O$ | 0.33 | 0.68 | 2.07 | 550 | I | F | 27.4 | --- | ---- |

Table 3

Illustrative Embodiments, Part C. (Continued)

(Footnotes are those of Part A)

| --Description--- #: CATALYST & System & Designation & (Salt/Zeolite) | | Conditions Temp. a) | Structure Pore vol. cc/g g) | Basicity Resulting pH 1% aq. h) | INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|---|
| C1 | $K_2CO_3$/NaY | 100 | 0.1 | 11 | | | | |
| C2 | $K_2CO_3$/NaY | 550 | 0.06 | 11 | very dk grn | nd | nd | amber |
| C3 | $K_2CO_3$/NaY | 580 | 0.05 | 10 | dk green | nd | nd | purple solid amber liq. |
| C4 | K acetate/NaY | 100 | -- | 7 | | | | |
| C5 | K acetate/NaY | 550 | -- | 8 | dk kelly grn | nd | nd | dark |
| C6 | K acetate/NaY | 100 | | 9 | | | | |
| C7 | K acetate/NaY | 550 | -- | 9 | dk kelly grn | nd | nd | dk purple bwn |
| C8 | K acetate/NaY | 100 | | 9 | | | | |

Table 3

Illustrative Embodiments, Part C. (Continued)

(Footnotes are those of Part A)

| --Description--- #: CATALYST & System & Designation & (Salt/Zeolite) | Cond-itions Temp. a) | Struc-ture Pore vol. cc/g g) | pH 1% aq. h) | Basicity Resulting INDICATOR COLORS---(i) 4-nitro-aniline /DMSO | 4-chloro aniline /DMSO | 4-nitro-aniline /benzene | 4-chloro-aniline /benzene |
|---|---|---|---|---|---|---|---|
| C13 K acetate/NaY | 550 | -- | 9 | dk kelly grn | nd | nd | black |
| C9 K acetate/NaY | 550 | -- | 9 | dk kelly grn | nd | nd | dk purple bwn |
| C10 K acetate/NaY | 100 | | 10 | | | | |
| C11 K acetate/NaY | 550 | -- | 9 | dk kelly grn | nd | nd | very dark |
| C12 K acetate/NaY | 100 | | 10 | | | | |

Table 3 (cont'd)

Illustrative Embodiments, Part C. (Continued)

(Footnotes are those of Part A)

| --Description--- | Cond-itions | Struc-ture | --------------Basicity Resulting--------------- | | | | |
|---|---|---|---|---|---|---|---|
| #: CATALYST & System & Designation & (Salt/Zeolite) | Temp. a) | Pore vol. cc/g g) | pH 1% aq. h) | ---------- INDICATOR COLORS---(i)------- 4-nitro-aniline /DMSO | 4-chloro aniline /DMSO | 4-nitro-aniline /benzene | 4-chloro-aniline /benzene |
| C14 K acetate/KL | 100 | | | | | | |
| C15 K acetate/KL | 550 | 0.04 | 12 | deep green | nd | nd | black |
| C16 K acetate/KL | 100 | | | | | | |
| C17 K acetate/KL | 550 | 0.06 | 11 | deep green | nd | nd | black |
| C18 KOAc/LZY82plt(j) | 100 | 0.17 | | | | | |
| C19 KOAc/LZY82plt | 550 | 0.17 | 10 | dk green | nd | nd | black |
| C20 CsOAc/LZY82plt | 100 | 0.3 | | | | | |
| C21 CsOAc/LZY82plt | 100 | 0.28 | | | | | |
| C22 CsOAc/LZY82plt | 550 | 0.3 | 7 | dk grn/yell liq | nd | nd | beige/bwn |
| C23 CsOAc/LZY52plt | 550 | --- | 10 | dk green | nd | nd | beige |
| C24 KOAc/LZY52plt | 550 | --- | 9 | dk kelly green liquid | nd | nd | dark;clear liquid |

Table 3 (cont'd)

Illustrative Embodiments, Part C. (Continued)

(Footnotes are those of Part A)

| --Description--- #: CATALYST & System & Designation & (Salt/Zeolite) | Cond-itions Temp. a) | Struc-ture Pore vol. cc/g g) | pH 1% aq. h) | Basicity Resulting INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|
| C25 KOAc/LZY82plt | 550 | --- | 9 | black; yell grn liquid | nd | nd | dk grn & white |
| C26 CsOAc/LZY62plt | 550 | --- | 9 | dk plts;kelly green liq | nd | nd | lt gray plt amber liq |
| C27 CsOAc/LZY62plt | 550 | --- | 9 | ditto | nd | nd | ditto |
| C28 KOAc/LZY62plt | 550 | --- | 9 | black/blue grn liq | nd | nd | dark/amber liq |

Table 3

Illustrative Embodiments, Part D.

(Footnotes are those of Part A)

| ---Description--- | | ----Materials used----- | | | | -----Conditions--- | | | ---Structure-- | | |
|---|---|---|---|---|---|---|---|---|---|---|---|
| #: & & & | CATALYST System Designation (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| D1 | KOAc/LZY62plts | $H_2O$ | 0.52 | 1.06 | 2.07 | 550 | I | F | 16.2 | -- | ---- |
| D2 | KOAc/LZY62plts | $H_2O$ | 0.68 | 1.39 | 2.03 | 550 | I | F | 8 | -- | ---- |
| D3 | CsOAc/LZY82plts | $H_2O$ | 0.67 | 1.33 | 2.03 | 550 | I | F | 15 | -- | ---- |
| D4 | KOAc/LZY82plts | $H_2O$ | 0.50 | 1.03 | 2.04 | 550 | I | F | 7 | -- | ---- |
| D5 | KOAc/LZY82plts | $H_2O$ | 0.33 | 0.67 | 2.01 | 550 | I | F | 28.4 | -- | ---- |
| D6 | $K_2CO_3$/Erionite | $H_2O$ | 0.5 | 0.51 | 1.04 | 150 | I | F | | | |
| | | $H_2O$ | 2x0.65 | 0 | above | 150 | "I"(k) | G | | | |
| | | | ----- | above | ----- | 550 | | G | 20 | -- | ---- |
| D7 | K oxalate/Erion. | $H_2O$ | 1.53 | 0.49 | 1.05 | 150 | I | C | | | |
| | | $H_2O$ | 2x0.6 | 0 | above | 150 | "I" | G | | | |
| | | | ------ | above | ------ | 550 | | G | 43 | -- | ---- |
| D8 | $K_2CO_3$/Mordenite | $H_2O$ | 1.02 | 1.02 | 1.99 | 150 | I | C | | | |
| | | $H_2O$ | 2x1.4 | 0 | above | 150 | "I" | G | | | |
| | | | ------ | above | ------ | 550 | | G | 33 | -- | ---- |

Table 3 (cont'd)

Illustrative Embodiments, Part D.

(Footnotes are those of Part A)

| ---Description--- #: CATALYST & System & Designation & (Salt/Zeolite) | ----Materials used----- Solvent | Solv. Wt. | Salt Wt. | Zeol. Wt. | -----Conditions--- Temp. a) | Method b) | Example c) | ---Structure-- XRD % xtal d) | S.A. sq.m./g e) | $n-C_8$ wetness cc/g f) |
|---|---|---|---|---|---|---|---|---|---|---|
| D9 K oxalate/Mord. | $H_2O$ | 3.05 | 0.98 | 2.05 | 150 | I | C | | | |
| | $H_2O$ | 2x1.5 | 0 | above | 150 | "I" | G | | | |
| | | ------above------ | | | 550 | | G | 46.4 | -- | ---- |
| D10 $K_2CO_3$/Chabazite | $H_2O$ | 0.05 | 0.06 | 0.10 | 150 | I | C | | | |
| | $H_2O$ | 2x0.05 | 0 | above | 150 | "I" | G | | | |
| | | ------above------ | | | 550 | | G | 14.4 | -- | ---- |
| D11 K oxalate/Chab. | $H_2O$ | 0.15 | 0.05 | 0.10 | 150 | I | C | | | |
| | $H_2O$ | 2x0.05 | 0 | above | 150 | "I" | G | | | |
| | | ------above------ | | | 550 | | G | 18 | -- | ---- |
| D12 CsOAc/Mordenite | $H_2O$ | 0.33 | 0.67 | 2.03 | 150 | I | C | | | |
| | $H_2O$ | 1.00 | 0 | above | 150 | "I" | G | | | |

Table 3 (cont'd)

Illustrative Embodiments, Part D.

(Footnotes are those of Part A)

| ---Description--- | | ----Materials used----- | | | | -----Conditions--- | | | ---Structure-- | | |
|---|---|---|---|---|---|---|---|---|---|---|---|
| #: & System & Designation & (Salt/Zeolite) | CATALYST | Solvent | Solv. Wt. | Salt Wt. | Zeol. Wt. | Temp. a) | Method b) | Example c) | XRD % xtal d) | S.A. sq.m./g e) | n-$C_8$ wetness cc/g f) |
| D13 | CsOAc/Mordenite | | ------above------ | | | 550 | | G | 45 | -- | ---- |
| D14 | CsOAc/Erionite | $H_2O$ | 0.06 | 0.13 | 0.33 | 150 | I | C | | | |
| | | $H_2O$ | 0.2 | 0 | above | 150 | "I" | G | | | |
| | | | ------above------ | | | 550 | | G | 38 | -- | ---- |

Table 3

Illustrative Embodiments, Part D. (Continued)

(Footnotes are those of Part A)

| --Description--- #: CATALYST & System & Designation & (Salt/Zeolite) | | Conditions Temp. a) | Structure Pore vol. cc/g g) | pH 1% aq. h) | ------------Basicity Resulting---------------- ----------INDICATOR COLORS---(i)--------- 4-nitro-aniline /DMSO | 4-chloro aniline /DMSO | 4-nitro-aniline /benzene | 4-chloro-aniline /benzene |
|---|---|---|---|---|---|---|---|---|
| D1 | KOAc/LZY62plts | 550 | -- | 9 | dp blue grn | nd | nd | dk/amber liq |
| D2 | KOAc/LZY62plts | 550 | -- | 9 | dk/dp blue grn | nd | nd | dk/amber liq |
| D3 | CsOAc/LZY82plts | 550 | -- | 9 | dk/kelly grn | nd | nd | dk grn/amberliq |
| D4 | KOAc/LZY82plts | 550 | -- | 9 | dk grn/lime grn liq | nd | nd | dk/amber liq |
| D5 | KOAc/LZY82plts | 550 | -- | 8 | dk/lime grn liq | nd | nd | dk bwn/clear |
| D6 | $K_2CO_3$/Erionite | 150 | | | | | | |
| | | 150 | | | | | | |
| | | 550 | -- | 12 | dk green | nd | nd | lt bwn/amber |

Table 3

Illustrative Embodiments, Part D. (Continued)

(Footnotes are those of Part A)

| --Description--- #: CATALYST & System & Designation & (Salt/Zeolite) | | Cond-ditions Temp. a) | Struc-ture Pore vol. cc/g g) | pH 1% aq. h) | Basicity Resulting INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|---|
| D7 | K oxalate/Erion. | 150 | | | | | | |
| | | 150 | | | | | | |
| | | 550 | -- | 11 | dk green | nd | nd | dk bwn/amber |
| D8 | $K_2CO_3$/Mordenite | 150 | | | | | | |
| | | 150 | | | | | | |
| | | 550 | -- | 12 | dk green | nd | nd | bwn/clear liq |

Table 3 (cont'd)

Illustrative Embodiments, Part D. (Continued)

(Footnotes are those of Part A)

| --Description--- #: CATALYST & System & Designation & (Salt/Zeolite) | Conditions Temp. a) | Structure Pore vol. cc/g g) | pH 1% aq. h) | Basicity Resulting INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|
| D9 K oxalate/Mord. | 150 | | | | | | |
| | 150 | | | | | | |
| | 550 | -- | 12 | dk green | nd | nd | bwn/lavender |
| D10 $K_2CO_3$/Chabazite | 150 | | | | | | |
| | 150 | | | | | | |
| | 550 | -- | 11 | green | nd | nd | white/clear |
| D11 K oxalate/Chab. | 150 | | | | | | |
| | 150 | | | | | | |
| | 550 | -- | 11 | green/yell | nd | nd | dk/clear |
| D12 CsOAc/Mordenite | 150 | | | | | | |
| | 150 | | | | | | |

Table 3 (cont'd)

Illustrative Embodiments, Part D. (Continued)

(Footnotes are those of Part A)

| --Description--- #: CATALYST & System & Designation & (Salt/Zeolite) | Cond-ditions Temp. a) | Structure Pore vol. cc/g g) | pH 1% aq. h) | Basicity Resulting INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|
| D13 CsOAc/Mordenite | 550 | -- | 9 | blue green | nd | nd | purple/amber |
| D14 CsOAc/Erionite | 150 | | | | | | |
| | 150 | | | | | | |
| | 550 | -- | 7 | blue green lavender liq | nd | nd | dp purple/ lavender liq |

Table 3

Illustrative Embodiments, Part E.

(Footnotes are those of Part A)

| --Description--- | | ----Materials used----- | | | | -----Conditions--- | | | ---Structure--- | | |
|---|---|---|---|---|---|---|---|---|---|---|---|
| #: | CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m. /g e) | $n$-$C_8$ wetness cc/g f) |
| E1 | $K_2CO_3$/NaMord. | $H_2O$ | 0.5 | 0.5 | 4.08 | 150 | I | C | | | |
| | | $H_2O$ | 1.0 | 0 | above | 150 | "I" | G | | | |
| | | | -----above----- | | | 550 | | G | 42 | -- | ----- |
| E2 | K oxalate/NaMord | $H_2O$ | 0.75 | 0.24 | 4.06 | 150 | I | C | | | |
| | | $H_2O$ | 1.0 | 0 | above | 150 | "I" | G | | | |
| | | | -----above----- | | | 550 | | G | 75 | -- | ----- |
| E3 | LiOAc/NaMord. | $H_2O$ | 0.51 | 0.49 | 4.06 | 150 | I | C | | | |
| | | $H_2O$ | 1.0 | 0 | above | 150 | "I" | G | | | |
| | | | -----above----- | | | 550 | | G | 46 | -- | ----- |

Table 3

Illustrative Embodiments, Part E.

(Footnotes are those of Part A)

| --Description--- | | ----Materials used----- | | | | -----Conditions--- | | | ---Structure--- | | |
|---|---|---|---|---|---|---|---|---|---|---|---|
| #: & & & | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) | $n-C_8$ wetness cc/g f) |
| E4 | CsOAc/NaMord. | $H_2O$ | 0.33 | 0.67 | 4.06 | 150 | I | C | | | |
| | | $H_2O$ | 1.0 | 0 | above | 150 | "I" | G | | | |
| | | | -----above----- | | | 550 | | G | 50 | -- | ----- |
| E5 | $K_2CO_3$/wshNaMord (1) | $H_2O$ | 0.5 | 0.5 | 4.01 | 150 | I | C | | | |
| | | $H_2O$ | 1.0 | 0 | above | 150 | "I" | G | | | |
| | | | -----above----- | | | 550 | | G | 64 | -- | ----- |

Table 3 (cont'd)

Illustrative Embodiments, Part E.

(Footnotes are those of Part A)

| --Description--- | | ----Materials used----- | | | | -----Conditions--- | | | ---Structure--- | | |
|---|---|---|---|---|---|---|---|---|---|---|---|
| #: | CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| E6 | Koxalate/wshNaM. | $H_2O$ | 0.75 | 0.24 | 4.06 | 150 | I | C | | | |
| | | $H_2O$ | 1.0 | 0 | above | 150 | "I" | G | | | |
| | | | -----above----- | | | 550 | | G | 83 | -- | ----- |
| E7 | KOAc/wshNaMord. | $H_2O$ | 0.33 | 0.67 | 4.04 | 150 | I | C | | | |
| | | $H_2O$ | 1.0 | 0 | above | 150 | "I" | G | | | |
| | | | -----above----- | | | 550 | | G | 31 | -- | ----- |
| E8 | LiOAc/wshNaMord | $H_2O$ | 0.51 | 0.49 | 4.03 | 150 | I | C | | | |
| | | $H_2O$ | 1.0 | 0 | above | 150 | "I" | G | | | |
| | | | -----above----- | | | 550 | | G | 50.4 | -- | ----- |
| E9 | $K_2CO_3/NH_4$Mcrden. | $H_2O$ | 40/2Molar | | 4.01 | 23 | S | H | | | |
| | | $H_2O$ | 2x50 | 0 | above | 150 | Wash | H | -- | -- | ----- |
| | | $H_2O$ | 4 0/2Molar | | above | 23 | S | H | | | |
| | | $H_2O$ | 2x50 | 0 | above | 23 | Wash | H | -- | -- | ----- |
| | | | -----above----- | | | 150 | | H | -- | -- | ----- |

Table 3 (cont'd)

Illustrative Embodiments, Part E.

(Footnotes are those of Part A)

| --Description--- | ----Materials used----- | | | | -----Conditions--- | | | ---Structure--- | | |
|---|---|---|---|---|---|---|---|---|---|---|
| #: CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| E10 $K_2CO_3$/LZY82pwdr (m) | $H_2O$ | 40/2Molar | | 4.03 | 23 | S | H | | | |
| | $H_2O$ | 2x50 | 0 | above | 150 | Wash | H | -- | -- | ----- |
| | $H_2O$ | 40/2Molar | | above | 23 | S | H | -- | -- | ----- |
| | $H_2O$ | 2x50 | 0 | above | 23 | Wash | H | -- | -- | ----- |
| | | -----above----- | | | 150 | | H | -- | -- | ----- |

Table 3

Illustrative Embodiments, Part E. (Continued)
(Footnotes are those of Part A)

| --Description--- #: CATALYST & System & Designation & (Salt/Zeolite) | Conditions Temp. a) | Structure Pore vol. cc/g g) | pH 1% aq. h) | Basicity Resulting INDICATOR COLORS (i) 4-nitroaniline /DMSO | 4-chloroaniline /DMSO | 4-nitroaniline /benzene | 4-chloroaniline /benzene |
|---|---|---|---|---|---|---|---|
| E1 $K_2CO_3$/NaMord. | 150 | | | | | | |
| | 150 | | | | | | |
| | 550 | -- | 9 | lt grn/yell | nd | nd | offwhite |
| E2 K oxalate/NaMord | 150 | | | | | | |
| | 150 | | | | | | |
| | 550 | -- | 9 | lt grn/blue grn | nd | nd | offwhite |
| E3 LiOAc/NaMord. | 150 | | | | | | |
| | 150 | | | | | | |
| | 550 | -- | 9 | green/golden | nd | nd | dk purple |
| E4 CsOAc/NaMord. | 150 | | | | | | |
| | 150 | | | | | | |
| | 550 | -- | 8 | dark green | nd | nd | dk brown |

<u>Table 3</u>

Illustrative Embodiments, Part E. (Continued)
(Footnotes are those of Part A)

| --Description--- #: CATALYST & System & Designation & (Salt/Zeolite) | | Conditions Temp. a) | Structure Pore vol. cc/g g) | pH 1% aq. h) | Basicity Resulting: INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|---|
| E5 | $K_2CO_3$/wshNaMord (1) | 150 | | | | | | |
| | | 150 | | | | | | |
| | | 550 | -- | 10 | lt green/yell | nd | nd | cream |

Table 3 (cont'd)

Illustrative Embodiments, Part E. (Continued)

(Footnotes are those of Part A)

| --Description--- #: CATALYST & System & Designation & (Salt/Zeolite) | | Conditions Temp. a) | Structure Pore vol. cc/g g) | pH 1% aq. h) | Basicity Resulting INDICATOR COLORS (i) 4-nitroaniline /DMSO | 4-chloroaniline /DMSO | 4-nitroaniline /benzene | 4-chloro-aniline /benzene |
|---|---|---|---|---|---|---|---|---|
| E6 | Koxalate/wshNaM. | 150 | | | | | | |
| | | 150 | | | | | | |
| | | 550 | -- | 7 | lime green | nd | nd | white |
| E7 | KOAc/wshNaMord. | 150 | | | | | | |
| | | 150 | | | | | | |
| | | 550 | -- | 8 | blue green | nd | nd | dk purple |
| E8 | LiOAc/wshNaMord | 150 | | | | | | |
| | | 150 | | | | | | |
| | | 550 | -- | 7 | dk grn/yell | nd | nd | dk purple w/lavender |

Table 3 (cont'd)

Illustrative Embodiments, Part E. (Continued)

(Footnotes are those of Part A)

| --Description--- #: CATALYST & System & Designation & (Salt/Zeolite) | Conditions Temp. a) | Structure Pore vol. cc/g g) | pH 1% aq. h) | Basicity Resulting: INDICATOR COLORS (i): 4-nitroaniline /DMSO | 4-chloroaniline /DMSO | 4-nitroaniline /benzene | 4-chloroaniline /benzene |
|---|---|---|---|---|---|---|---|
| E9 $K_2CO_3/NH_4$Morden. | 23 | | | | | | |
| | 150 | -- | 8 | | | | |
| | 23 | | | | | | |
| | 23 | -- | 9 | | | | |
| | 150 | -- | 9 | dark green | nd | nd | pink |
| E10 $K_2CO_3$/LZY82pwdr (m) | 23 | | | | | | |
| | 150 | -- | 8 | | | | |
| | 23 | -- | - | | | | |
| | 23 | -- | 9 | | | | |
| | 150 | -- | 9 | green | nd | nd | cream |

Table 3

Illustrative Embodiments, Part F.

(Footnotes are those of Part A)

| #: | CATALYST System Designation (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
|---|---|---|---|---|---|---|---|---|---|---|---|
| | --Description--- | ----Materials used----- | | | | -----Conditions--- | | | ----Structure--- | | |
| F1 | CsOAc/wshNaY | None | 0 | 0.87 | 8.63 | 200(n) | M | I | -- | -- | ---- |
| F2 | | ----------above------- | | | | 550 | | I | 84 | -- | ---- |
| F3 | $K_2CO_3$/wshNaY | $H_3O$ | 5.5 | 7.07 | 15.03 | 150 | I | C | | | |
| | | ----------above------- | | | | 550 | | C | 12 | -- | ---- |
| F4 | $K_2CO_3$/LZY82pwdr | $H_2O$ | 12.64 | 12.64 | 30.0 | 150 | I | C | | | |
| F5 | | $H_2O$ | 10 | 0 | above | 150 | "I" | G | | | |
| F6 | | ------1/2 above mat'l-- | | | | 550 | | G | -- | -- | ---- |
| F7 | -other 1/2 of F5 | $H_2O$ | 8 | 0 | 1/2 of F5 | 150 | "I" | J | | | |
| F8 | | $H_2O$ | 2x5.0 | 0 | above | 150 | "I" | J | -- | -- | ---- |
| F9 | | $H_2O$ | 5 | 0 | all F6 | 150 | "I" | J | | | |
| F10 | | --------above mat'l---- | | | | 550 | | J | -- | -- | ---- |
| F11 | LiOAc/LZY82pwdr | $H_2O$ | 22 | 20.75 | 30.0 | 150 | I | C | | | |
| F12 | | $H_2O$ | 10 | 0 | above | 150 | "I" | G | | | |
| F13 | | --1/2 mat'l above------ | | | | 550 | | G | -- | -- | ---- |

Table 3 (cont'd)

Illustrative Embodiments, Part F.

(Footnotes are those of Part A)

| --Description--- | | ----Materials used----- | | | | -----Conditions--- | | ----Structure--- | | | |
|---|---|---|---|---|---|---|---|---|---|---|---|
| #: & | CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| F14 | | $H_2O$ | 3x5.0 | 0 | 1/2 F12 | 150 | "I" | J | -- | -- | ---- |
| F15 | | $H_2O$ | 5.0 | 0 | all F13 | 150 | "I" | J | | | |
| F16 | | ------------above------ | | | | 550 | | J | -- | -- | ---- |
| F17 | CsOAc/LZY82pwdr | $H_2O$ | 10.0 | 35 | 30.0 | 150 | I | F | | | |
| F18 | | $H_2O$ | 10 | 0 | above | 150 | "I" | G | | | |
| F19 | | -----1/2 of F18 used--- | | | | 550 | | G | -- | -- | ---- |
| F20 | | $H_2O$ | 3x5.0 | 0 | 1/2 F18 | 150 | "I" | J | 13 | -- | ---- |
| F21 | | $H_2O$ | 10 | 0 | all F19 | 150 | "I" | J | | | |
| F22 | | ----above material------ | | | | 550 | | J | -- | -- | ---- |

Table 3 (cont'd)

Illustrative Embodiments, Part F.

(Footnotes are those of Part A)

| --Description--- #: CATALYST & System & Designation & (Salt/Zeolite) | | ----Materials used----- Solvent | Solv. Wt. | Salt Wt. | Zeol. Wt. | -----Conditions--- Temp. a) | Method b) | Example c) | ----Structure--- XRD % xtal d) | S.A. sq.m. /g e) | n-$C_8$ wetness cc/g f) |
|---|---|---|---|---|---|---|---|---|---|---|---|
| F23 | NaOAc/LZY82pwdr | $H_2O$ | 31.8 | 15.03 | 30.0 | 150 | I | C | | | |
| F24 | | $H_2O$ | 10 | 0 | above | 150 | "I" | G | | | |
| F25 | | $H_2O$ | 3x5.0 | 0 | 1/2 ofF24 | 150 | "I" | J | 115 | -- | ---- |
| F26 | | ---Other 1/2 of F24----- | | | | 550 | | J | 42 | -- | ---- |
| F27 | | $H_2O$ | 5 | 0 | all above | 150 | "I" | J | | | |
| F28 | | ----above material------ | | | | 550 | | J | 29 | -- | ---- |

Table 3 (cont'd)

Illustrative Embodiments, Part F.

(Footnotes are those of Part A)

| --Description--- | | ----Materials used----- | | | | -----Conditions--- | | | ----Structure--- | | |
|---|---|---|---|---|---|---|---|---|---|---|---|
| #: | CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | $n-C_8$ wetness cc/g f) |
| F29 | LiOAc/wshNaY | $CH_3OH$ | 6cc | 2.06 | 10.05 | 150 | I | L | 121 | -- | ---- |
| F30 | | -----above material------- | | | | 550 | | L | 120 | -- | ---- |
| F31 | CsOAc/wshNaY | MeOH | 8cc | 2.03 | 10.08 | 150 | I | L | 51 | -- | ---- |
| F32 | | -----above material------- | | | | 550 | | L | 57 | -- | ---- |
| F33 | MeOK/wshKL | MeOH | 8cc | 3.13 | 12.61 | 150 | I | L | 46 | -- | ---- |
| F34 | | -----above material------- | | | | 550 | | L | 68 | -- | ---- |
| F35 | CsOAc/wshNaY | DMSO | 5.5cc | 1.12 | 5.11 | 200 | I | M | 50 | -- | ---- |
| F36 | | -----above material------- | | | | 550 | | M | 48 | -- | ---- |
| F37 | LiOAc/wshNaY | DMSO | 4cc | 1.04 | 5.04 | 200 | I | M | 110 | -- | ---- |
| F38 | | -----above material------- | | | | 550 | | M | 98 | -- | ---- |

Table 3

Illustrative Embodiments, Part F. (Continued)

(Footnotes are those of Part A)

| --Description--- #: CATALYST & System & Designation & (Salt/Zeolite) | Conditions Temp. a) | Structure Pore vol. cc/g g) | pH 1% aq. h) | 4-nitro-aniline /DMSO | 4-chloro aniline /DMSO | 4-nitro-aniline /benzene | 4-chloro-aniline /benzene |
|---|---|---|---|---|---|---|---|
| | | | Basicity Resulting INDICATOR COLORS---(i) | | | | |
| F1 CsOAc/wshNaY | 200(n) | -- | 6 | yellow | nd | nd | white |
| F2 | 550 | -- | 10 | Dark Green | nd | nd | pink/amber |
| F3 $K_2CO_3$/wshNaY | 150 | | | | | | |
| | 550 | -- | 11 | Dark Green | nd | nd | dk purple bwn |
| F4 $K_2CO_3$LZY82pwdr | 150 | | | | | | |
| F5 | 150 | | | | | | |
| F6 | 550 | -- | 10 | Dark Green | nd | nd | dark lavender |
| F7-other 1/2 of F5-- | 150 | | | | | | |
| F8 | 150 | -- | 11 | light green | nd | nd | white |
| F9 | 150 | | | | | | |
| F10 | 550 | -- | 7 | Dark Green | nd | nd | lavender |
| F11 LiOAc/LZY82pwdr | 150 | | | | | | |
| F12 | 150 | | | | | | |
| F13 | 550 | -- | 10 | Dark Green | nd | nd | dark brown |

Table 3 (cont'd)

Illustrative Embodiments, Part F. (Continued)

(Footnotes are those of Part A)

| --Description--- #: CATALYST & System & Designation & (Salt/Zeolite) | Conditions Temp. a) | Structure Pore vol. cc/g g) | Basicity Resulting: pH 1% aq. h) | INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|
| F14 | 150 | -- | 8 | yellow | nd | nd | white |
| F15 | 150 | | | | | | |
| F16 | 550 | -- | 9 | Dark Green | nd | nd | dark lavender |
| F17 CsOAc/LZY82pwdr | 150 | | | | | | |
| F18 | 150 | | | | | | |
| F19 | 550 | -- | 12 | Dark Green | nd | nd | dark lavender |
| F20 | 150 | -- | 8 | light green | nd | nd | white |
| F21 | 150 | | | | | | |
| F22 | 550 | -- | 11 | Dark Green | nd | nd | black |

Table 3 (cont'd)

Illustrative Embodiments, Part F. (Continued)

(Footnotes are those of Part A)

| --Description--- #: CATALYST & System & Designation & (Salt/Zeolite) | Conditions Temp. a) | Structure Pore vol. cc/g g) | pH 1% aq. h) | Basicity Resulting INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|
| F23 NaOAc/LZY82pwdr | 150 | | | | | | |
| F24 | 150 | | | | | | |
| F25 | 150 | -- | 8 | yellow | nd | nd | cream |
| F26 | 550 | -- | 11 | Dark Green | nd | nd | black |
| F27 | 150 | | | | | | |
| F28 | 550 | -- | 8 | Dark Green | nd | nd | deep purple |

Table 3 (cont'd)

Illustrative Embodiments, Part F. (Continued)

(Footnotes are those of Part A)

| --Description--- #: CATALYST & System & Designation & (Salt/Zeolite) | Conditions Temp. a) | Structure Pore vol. cc/g) | pH 1% aq. h) | Basicity Resulting INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|
| F29 LiOAc/wshNaY | 150 | -- | 8 | lime green | nd | nd | cream |
| F30 | 550 | -- | 10 | lime green | nd | nd | lt lavender/ specks dk purp |
| F31 CsOAc/wshNaY | 150 | -- | 7 | yellow | nd | nd | cream |
| F32 | 550 | -- | 11 | medium grn | nd | nd | lavnder/dk pur |
| F33 MeOK/wshKL | 150 | -- | 11 | medium grn | nd | nd | cream |
| F34 | 550 | -- | 12 | dark green | nd | nd | lavender |
| F35 CsOAc/wshNaY | 200 | -- | 6 | golden bwn | nd | nd | lt brown |
| F36 | 550 | -- | 9 | dark green | nd | nd | lavender&pink |
| F37 LiOAc/wshNaY | 200 | -- | 6 | golden | nd | nd | lt pink-bwn |
| F38 | 550 | -- | 10 | dark olive | nd | nd | dark purple |

Table 3

Illustrative Embodiments, Part G.

(Footnotes are those of Part A)

Alkali metal basified zeolites containing catalytic metal centers:

| ---Description--- | | ----Materials used----- | | | | -----Conditions--- | | | ---Structure--- | | |
|---|---|---|---|---|---|---|---|---|---|---|---|
| #: | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) | $n-C_8$ wetness cc/g f) |
| G1 | $Ru(NO)(NO_3)_3$/NaY | $H_2O$ | 18 | 5 | 31.15 | 100 | I | K | | | |
| G2 | $Ru(NO)(NO_3)_3$/NaY | ---material above--- | | | | 550 | | K | 74 | 574 | ---- |
| G3 | $K_2CO_3$/G2 (Ru/NaY) | $H_2O$ | 6 | 5.5 | 12 | 100 | I | K | | | |
| G4 | | ---material above--- | | | | 550 | | K | 23 | 190 | ---- |
| G5 | $Cu(NO_3)_2$/NaY | $H_2O$ | 40 | 7.7 | 50 | 100 | I | K | 41.1 | -- | ---- |
| G6 | | ---material above--- | | | | 550 | | K | 5 | -- | ---- |
| G7 | $K_2CO_3$/G5 (CuNaY) | $H_2O$ | 14 | 12.74 | 28 | 100 | I | K | 45.1 | -- | ---- |
| G8 | | ---material above--- | | | | 550 | | K | 28 | -- | ---- |

Table 3

Illustrative Embodiments, Part G.

(Footnotes are those of Part A)

Alkali metal basified zeolites containing catalytic metal centers:

| ---Description--- | ----Materials used----- | | | | -----Conditions--- | | | ---Structure--- | | |
|---|---|---|---|---|---|---|---|---|---|---|
| #: CATALYST & System & Designation & (Salt/Zeolite) | Solvent | Solv. Wt. | Salt Wt. | Zeol. Wt. | Temp. a) | Method b) | Example c) | XRD % xtal d) | S.A. sq.m./g e) | n-$C_8$ wetness cc/g f) |
| G9 $Fe(NO_3)_3$/NaY | $H_2O$ | 40 | 5.82 | 50 | 100 | I | K | -- | -- | ---- |
| G10 Fe/NaY | ---material above--- | | | | 550 | I | K | 81 | -- | ---- |
| G11 $K_2CO_3$/G10(FeNaY) | $H_2O$ | 6 | 5.7 | 12.5 | 100 | I | K | 49 | -- | ---- |
| G12 | ---material above--- | | | | 550 | | K | 29 | -- | ---- |

Table 3

Illustrative Embodiments, Part G. (Continued)
(Footnotes are those of Part A)

| ---Description--- | Conditions | Structure | ------------Basicity Resulting------------ | | | | |
|---|---|---|---|---|---|---|---|
| #: CATALYST & System & Designation & (Salt/Zeolite) | Temp. a) | Pore vol. cc/g g) | pH 1% aq. h) | ---------- INDICATOR COLORS---(i)------- 4-nitro-aniline /DMSO | 4-chloro aniline /DMSO | 4-nitro-aniline /benzene | 4-chloro-aniline /benzene |
| G1 Ru(NO)$(NO_3)_3$/NaY | 100 | | | | | | |
| G2 Ru(NO)$(NO_3)_3$/NaY | 550 | 0.31 | 7 | black(o) | nd | nd | black |
| G3 $K_2CO_3$/G2 (Ru/NaY) | 100 | | | | | | |
| G4 --matl above-- | 550 | 0.12 | 10 | blackish grn | nd | nd | dp purple |
| G5 $Cu(NO_3)_2$/NaY | 100 | -- | 7 | yellow | nd | nd | brown |
| G6 --matl above-- | 550 | -- | 7 | brownish | nd | nd | black |
| G7 $K_2CO_3$/G5 CuNaY | 100 | -- | 10 | dk green | nd | nd | deep purple |
| G8 --matl above-- | 550 | -- | 11 | dark green | nd | nd | deep purple |
| G9 $Fe(NO_3)_3$/NaY | 100 | -- | 7 | orng-yellow | nd | nd | amber |
| G10matl above Fe/NaY | 550 | -- | 8 | Orng-yellow | nd | nd | rose |
| G11 $K_2CO_3$/G10(FeNaY) | 100 | -- | 10 | green | nd | nd | rose |
| G12 matl above-- | 550 | -- | 11 | blue green | nd | nd | rose |

Further experiments were carried out using alkaline earth metal compounds; the details are given in the Examples AA-RR and in Table 4. The footnotes are as given in Table 3 (for footnote b: G = gas; P = precipitation; I = impregnation with solvent alone to redistribute material; C = calcination).

Example AA:

10.17g Davison 5A zeolite (8-12 mesh beads) was impregnated with 4.57g barium acetate (Alfa) dissolved in 6.94cc of Barnstead deionized water. Four impregnations were necessary. After each impregnation the zeolite was rotated in a round bottom flask on a rotary evaporator (rotation only). After the second rotation, the zeolite was dried in a 100 °C vacuum oven. The zeolite was dried in a 60 °C vacuum oven after the third rotation. After the fourth impregnation, 16.33g of the zeolite was placed in fritted glassware and 19.4g of anhydrous ammonia was bubbled up through the beads. These zeolite beads were transferred to an Erlenmeyer flask and washed with two 20cc portions of lime water, vacuum filtering after each wash. Then the zeolite was dried for 2.5 days in a 100 °C vacuum oven. The zeolite beads were placed in a vycor tube in an upright furnace. With nitrogen flowing over the beads, the temperature was raised to 450 °C and held for 2 hours. After cooling, the tube was sent into the dry box for bottling and storage.

Example BB:

In a dish, 50.02g Baylith CP-190 NaY zeolite was impregnated with 23.65g magnesium nitrate hexahydrate (Aldrich) dissolved in 55cc water. The impregnation was done in two steps, with drying for 1hr in a 100 °C vacuum oven between steps, followed by a final drying at 100 °C for about 16 hrs. The dried material was placed in a vycor tube in an upright furnace and heated to 550 °C for 1hr under flowing nitrogen. The heater was turned off and allowed to cool with nitrogen still flowing. A cold trap and an ascarite trap were placed in the exiting nitrogen line during the procedure and 6.65g water and .13g "$CO_2$" were trapped.

Example CC1:

30.73g K-L zeolite was washed with 300cc Barnstead deionized water, then filtered. 200cc more water was poured through the funnel. The material was then dried for 1hr in a 150 °C vacuum oven.

2.00g of the washed and dried K-L was impregnated with .93g magnesium acetate (MCB reagent) dissolved in 1.51g Barnstead water. Two impregnations were necessary. After the first, the material was allowed to rest for 38 minutes, and then dried 17.5hrs in a 150 °C vacuum oven. After the second, the material was allowed to rest about 30 minutes, then dried for 63 minutes in a 150 °C vacuum oven. The dried material was then placed in a vycor tube in an upright furnace and heated to 200 °C with nitrogen flowing over the material. After 25 minutes, the temperature was raised to 550 °C, where it was held for 2hrs. After cooling the tube was sent into the dry box for bottling and storage.

Example CC2:

1.36g $NH_4$+ Ferrierite (Engelhard Lot # 15264-18) was impregnated with .61g magnesium acetate tetrahydrate (MCB reagent) dissolved in .99cc water (Barnstead deionized). After 10 minutes, the zeolite was dried for about 17.5 hours in a 150 °C vacuum oven. The dried zeolite was placed in a vycor tube in an upright furnace and was heated to 200 °C under flowing nitrogen. After 25 minutes, the temperature was raised to 550 °C and held for 2hrs. The cooled tube was sent into the dry box for bottling and storage.

Example D:

4.00g 5A zeolite (Davison 8-12 mesh) was impregnated with 1.47g strontium acetate (ICN) dissolved in 4cc Barnstead water. After 1hr, the zeolite was dried for 2.5hrs in a 150 °C vacuum oven. The dried material was impregnated with 1.6cc 10N $NH_4OH$, then washed with a mixture of .5cc 10N $NH_4OH$ in 5cc water, and filtered. The filtered zeolite was dried for 1hr 25 minutes in a 150 °C vacuum oven, then placed in a vycor tube in an upright furnace, where it was heated to 400 °C under flowing nitrogen. After 2hrs, the heater was turned off and the tube allowed to cool. The cooled tube was sent into the dry box for bottling and storage.

Example EE:

LZY 82 powder (Union Carbide) was pressed for 2 minutes at 20000psi in an isostatic press, then ground and sieved to give a 16-30 mesh powder. This sieved LZY 82 powder was placed in a vycor tube in an upright furnace and heated to 550° C under flowing nitrogen. After 2.3hrs, the heater was turned off and the tube allowed to cool, still under flowing nitrogen. The material was stored in the dry box. 4.03g of dried LZY 82 was impregnated with 2.40g magnesium acetate tetrahydrate dissolved in 2.23cc water. After drying for 1hr in a 150° C vacuum oven, 1.5cc water (Barnstead) was added before the zeolite was returned to the 150° C vacuum oven for another 2.25hrs. The water addition was repeated two more times, with the final drying being 17.5hrs in a 150° C vacuum oven. The zeolite was then impregnated with 1.6cc 10N $NH_4OH$, followed by a wash with .5cc $NH_4OH$ in 5cc water, followed by filtering. The filtered material was dried for 3 hrs in a 150° C vacuum oven, then placed in a vycor tube in an upright furnace. The furnace was heated to 200° C under flowing nitrogen. After 25 minutes, the temperature was raised to 550° C. After 2hrs, the furnace was turned off and the tube allowed to cool under flowing nitrogen. The material was bottled and stored in the dry box.

Example FF:

4.00g Na mordenite was impregnated with .67g magnesium acetate tetrahydrate dissolved in .33cc water and allowed to rest 30 minutes before being dried 55 minutes in a 150° C vacuum oven. 1cc water was added to the dried zeolite. After mixing, the zeolite was allowed to rest 1/2 hr before being dried 1hr in a 150° C in a vacuum oven. The dried material was placed in a vycor tube in an upright furnace. The furnace was heated to 200° C under flowing nitrogen. After 15 minutes the temperature was raised to 550° C and held for 2hrs. Upon cooling, the tube was sent into the dry box for bottling and storage.

Example GG:

In a vial .11g chabazite (dried 1hr in a 150° C vacuum oven) was impregnated with .01g calcium acetate dissolved in .03cc water. The chabazite was allowed to rest 13 minutes before being dried 1.2hrs in a 150° C vacuum oven. The dried material was placed in a vycor tube in an upright furnace and was heated to 200° C under flowing nitrogen. After 20 minutes, the temperature was raised to 550° C. After 2hrs, the furnace was turned off and allowed to cool. The tube was sent into the drybox for bottling and storage.

Example HH:

48.23g 16-30 mesh Na mordenite was placed into a fritted funnel and washed with 150cc Barnstead deionized water and then vacuum filtered. This washing was repeated three more times. The washed material was placed in a dish and dried 35 minutes in a 150° C vacuum oven.

In a vial, 4.00g washed Na mordenite was impregnated with .26g calcium acetate dissolved in .74cc Barnstead water. After 30 minutes, the zeolite was dried 1hr in a 150° C vacuum oven. 1cc water was added and the vial was laid on its side. After 30 minutes, it was dried 1hr in a 150° C vacuum oven. The dried material was placed in a vycor tube in an upright furnace and was heated to 200° C under flowing nitrogen. After 24 minutes, the temperature was raised to 550° C and held for 2 hrs. After cooling, the tube was sent into the drybox for bottling and storage.

Example II:

4.03g dried LZY 82 (2hrs in a 150° C vacuum oven) was stirred with 40cc 2M calcium acetate solution for 1hr after which time it was filtered, and then dried overnight in a 150° C vacuum oven. Then it was stirred with an additional 40cc 2M calcium acetate solution for 1hour, then filtered and washed with two 50cc portions of Barnstead water. A fresh portion of 2M calcium acetate was added, followed by stirring, filtering and 2 water washes. This material was dried overnight in a 150° C vacuum oven.

Example JJ:

209g NaY (Baylith CP-190) was stirred with 2 litres Barnstead water for 2hrs and then filtered. An additional 1.4 litres of water was poured through the filter funnel. The material in the filter was dried overnight in a 150 °C vacuum oven.

In a dish, 15.03g washed NaY was impregnated with 5.14g calcium acetate hydrate (Aldrich) dissolved in 15cc Barnstead water. After 27 minutes, the material was dried 1hr in a 150 °C vacuum oven. The dried material was impregnated with 6cc 10N $NH_4OH$ (Aldrich) and allowed to rest for 45 minutes before being washed with a solution of 2cc 10N $NH_4OH$ in 20cc Barnstead water. After filtering, the material was dried 1hr in a 150 °C vacuum oven. The dried material was placed in a vycor tube in an upright furnace and was heated to 200 °C under flowing nitrogen. After 27 minutes, the temperature was raised to 550 °C and held for 2hrs before the heaters were turned off. The cooled tube was sent into the drybox for bottling and storage.

Example KK:

50.04g NaY (Baylith CP-190) was impregnated with 20.5g calcium nitrate dissolved in 8.5cc water. The impregnation was done in a round bottom flask which was tumbled on a rotary evaporator. After about 1hr, a solution of 15.2g potassium carbonate dissolved in 10.8cc water was added to the round bottom flask. This was tumbled for 1hr. The water bath was then heated to 60 °C. After 1hr, the round bottom was removed from the heater and 100cc lime water was added and then the zeolite filtered and the liquid poured back through the funnel. This lime water wash was repeated 3 more times. Then the material was dried over the weekend in a 100 °C vacuum oven. Half of the dried material was placed in a metal tube in an upright furnace and heated to 550 °C under flowing nitrogen. After 97 minutes the furnace was cooled, the tube was removed and the material bottled. The other half of the dried material was placed in a quartz tube and placed in an upright furnace and heated to 700 °C under flowing nitrogen. After 4.5hrs, the furnace was cooled, the tube removed, and the material bottled.

Example LL:

In a vial, 3.32g dried LZY 82 pellets (2.5hrs in 160 °C vacuum oven) was impregnated with 3.10g magnesium acetate tetrahydrate dissolved in 2.5cc Barnstead water. The impregnation was done in three steps with heating for 30 minutes in a 150 °C vacuum oven between steps, and after the final step, 1cc water was added to the dried material. After 54 minutes, it was dried 1hr in a 150 °C vacuum oven. An additional 1cc water was added and after 37 minutes, the material was dried overnight in a 150 °C vacuum oven. The dried material was placed in a vycor tube in an upright furnace and heated to 200 °C under flowing nitrogen. After 25 minutes the temperature was raised to 550 °C. After 2hrs, the furnace was turned off and allowed to cool. The cooled tube was bottled and stored in the dry box.

Example MM:

In a vial, 2.60g $NH_4$ + USY (CB 86-133) was impregnated with 2.44g magnesium acetate tetrahydrate dissolved in 1.96cc Barnstead water. The impregnation was done in two steps, with drying for 30 minutes at 150 °C between steps and 1hr drying after the last step. The dried material was placed in a vycor tube in an upright furnace and heated to 200 °C under flowing nitrogen. After 23 minutes, the temperature was raised to 550 °C and held for 2hrs. The cooled tube was sent into the dry box for bottling and storage.

Example NN:

4.02g dried $NH_4$ + mordenite (2hrs in a 150 °C vacuum oven with a slow nitrogen bleed) was slurried with 40cc 2M magnesium acetate for 2hrs, and then filtered and washed with two 50cc portions Barnstead water. The filtered material was dried 1hr in a 150 °C vacuum oven. Then slurrying with 2M magnesium acetate and washing with water was repeated three more times. The material was then dried 1hr in a 150 °C vacuum oven.

In a vial 1.00g of the Mg mordenite was impregnated with .33cc of a solution of 1g magnesium acetate tetrahydrate in 1.4cc methanol. After 42 minutes, the material was dried overnight in a 150 °C vacuum oven. The dried material was placed in a vycor tube in an upright furnace and heated to 200 °C under flowing nitrogen. After 25 minutes, the temperature was raised to 550 °C and held for 2hrs. The cooled tube was sent into the drybox for bottling and storage.

Example OO:

In a vial 2.05g 5A zeolite was impregnated with .6cc of a solution of 1.02g magnesium acetate tetrahydrate dissolved in 1.8cc dimethylsulphoxide. The material was dried 1.25hrs in a 150 °C vacuum oven and then placed in a vycor tube in an upright furnace and heated to 200 °C under flowing nitrogen. After 29 minutes, the temperature was raised to 550 °C and held for 2hrs. Upon cooling,the tube was sent into the drybox for bottling and storage.

Example PP:

A mixture of 21.36g ground 5A zeolite and 2.24g calcium butyrate was added to a vycor tube in an upright furnace and slowly heated to 550 °C under flowing nitrogen. After 1hr at 550 °C, the furnace was cooled, and the cooled tube was sent into the dry box for bottling and storage.

Example QQ:

In a vial, 1.00g Ca-LZY82 (preparation described in Example 1) was impregnated with .47g magnesium acetate tetrahydrate dissolved in .75cc Barnstead water. The impregnation took place in two steps. After the first step, the material was left for 2.33hrs, and then was dried overnight in a 150 °C vacuum oven. After the second step, the material was left for 10 minutes before being dried 2.2hrs in a 150 °C vacuum oven with a slow nitrogen bleed. The dried material was placed in a vycor tube in an upright furnace and heated to 200 °C under flowing nitrogen. After 15 minutes, the temperature was raised to 550 °C and held 2hrs. The cooled material was bottled and stored in the dry box.

Example RR:

In a vial, 2.53g dried NH4+ mordenite (dried 2hrs in a 150 °C vacuum oven) was impregnated with 1.18g magnesium acetate tetrahydrate dissolved in 1.9cc water. After 22 minutes, the material was dried overnight in a 150 °C vacuum oven. The dried material was placed in a vycor tube in an upright furnace and heated to 200 °C under flowing nitrogen. After 25 minutes, the temperature was raised to 550 °C and held for 2hrs. The cooled tube was sent into the dry box for bottling and storage.

Table 4

Illustrative Embodiments

(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| B-1 | $Ca(NO_3)_2$/NaY | $H_2O$ | 9 | 20.52 | 50.31 | 23 | I | AA | | --- |
| B-2 | $NH_3$/Ca/NaY | 44g NH3 gas over 41.49g of above zeolite | | | | 23 | GI;P | AA | | --- |
| B-3 | Lime wash/Ca/NaY | $H_2O$ | 200 | 0.28 | above | 110 | Wash/dry | AA | 91 | --- |
| B-4 | Ca(OH)x/NaY | --sample above------- | | | | 450 | C | AA | 92 | --- |
| B-5 | $Ca(NO_3)_2$/NaY | $H_2O$ | 30 | 23.31 | 50.0 | 100 | I | BB | 53.7 | 7 |
| B-6 | | ---sample above------ | | | | 550 | C | BB | 59.1 | 305 |
| B-7 | $Mg(NO_3)_2$/NaY | $H_2O$ | 55 | 23.65 | 50.02 | 100 | I | BB | 89.2 | 97 |
| B-8 | | ---sample above------- | | | | 550 | C | BB | 93.4 | 496 |

Table 4

Illustrative Embodiments

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| B-9 $Mg(OAc)_2$/NaY | $H_2O$ | 38 | 23.48 | 50.49 | 100 | I | CC | 95.7 | 105 |
| B-10 | ---sample above------- | | | | 550 | C | CC | 71.6 | 525 |
| B-11 $Ca(OAc)_2$/LZY52plt | $H_2O$ | 1.66 | 0.59 | 2.05 | 550 | I | CC | 73 | 464 |
| B-12 $Ca(OAc)_2$/LZY82plt | $H_2O$ | 2.27 | 0.81 | 2.05 | 550 | I | CC | 74.4 | 447 |
| B-13 $Ca(OAc)_2$/LZY62plt | $H_2O$ | 1.56 | 0.56 | 2.07 | 550 | I | CC | 74.1 | 524 |
| B-14 $Ca(OAc)_2$/LZY62plt | $H_2O$ | 2.21 | 0.79 | 2.03 | 550 | I | CC | 71.5 | 474 |

Table 4 (cont'd)

Illustrative Embodiments

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| B-15$_1$ $Ca(OAc)_2$/LZY82plt | $H_2O$ | 1.56 | 0.55 | 2.00 | 550 | I | CC | 76.3 | 461 |
| B-15$_2$ $Ca(OAc)_2$/LZY82plt | $H_2O$ | 4.46 | 1.58 | 4.01 | 150 | I | CC | | |
| B-15$_3$ $NH_4OH$/Ca/LZY82plt | -1.6cc 10N $NH_4OH$ on above- | | | | | I/P | DD | | |
| B-16 | $H_2O$,5cc+0.5cc 10N $NH_4OH$ | | | | 150 | I/W | DD | | |
| B-17 | -----sample above------ | | | | 550 | C | DD | 67.4 | 426 |
| B-18 $Mg(OAc)_2$/LZY82plt | $H_2O$ | 1.23 | 2.47 | 4.09 | 150 | I | CC | | |
| $NH_4OH$ on above | 1.6cc of 10N $NH_4OH$ on B-18 | | | | | I/P | DD | | |
| B-19 $NH_4OH$ on above | $H_2O$,5cc+.5cc 10N $NH_4OH$ | | | | 150 | I/W | DD | | |
| B-20 | -----sample above------ | | | | 550 | C | DD | 89.1 | 438 |
| B-21 $Ca(OAc)_2$/LZY82pwd | $H_2O$ | 4.45 | 1.58 | 4.00 | 150 | I | CC | | |
| $NH_4OH$ on above | 1.6cc of 10N $NH_4OH$ on B-21 | | | | | I/P | DD | | |
| B-22 $NH_4OH$ on above | 5cc$H_2O$+.5cc 10N $NH_4OH$ | | | | 150 | I/W | DD | | |
| B-23 | ------sample above----- | | | | 550 | C | DD | 73.9 | 481 |

Table 4 (cont'd)

Illustrative Embodiments

(Footnotes are those of Table 3)

| --Description--- | ----Materials used----- | | | | -----Conditions----- | | | -Structure- | |
|---|---|---|---|---|---|---|---|---|---|
| CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| B-24 $Mg(OAc)_2$/LZY82pwd | $H_2O$ | 2.23 | 2.4 | 4.03 | 150 | I | CC | | |
| redistribute salt | $H_2O$ | 3x1.5cc | 0 | above | 150 | "I" | EE | | |
| $NH_4OH$ on above | 1.6cc of 10N $NH_4OH$ on B-24 | | | | | I/P | EE | | |
| $NH_4OH$ on above | 5cc $H_2O$+.5cc 10N $NH_4OH$ | | | | 150 | I/W | EE | | |
| | ------sample above----- | | | | 550 | C | EE | 82.5 | 479 |
| B-25 $Ca(OAc)_2$/Erionite | $H_2O$ | 2.22 | 0.79 | 1.01 | 150 | I | CC | | |
| | $H_2O$ | 2x1.5 | 0 | above | 150 | "I" | FF | | |
| | ---sample above-------- | | | | 550 | C | FF | 54.5 | 68 |
| B-26 $Ca(OAc)_2$/Mordent. | $H_2O$ | 2.98 | 1.06 | 2.03 | 150 | I | CC | | |
| | $H_2O$ | 2x1.9 | 0 | above | 150 | "I" | FF | | |
| | ----sample above------- | | | | 550 | C | FF | 26 | 103 |

Table 4

Illustrative Embodiments, (Continued)

(Footnotes are those of Table 3)

| --Description--- | | --Structure-- | | ------------Basicity Resulting------------- | | | | |
|---|---|---|---|---|---|---|---|---|
| | CATALYST System Designation (Salt/Zeolite) | n-C wet- ness cc/g f) | Pore vol. cc/g g) | pH 1% aq. h) | INDICATOR COLORS (i): 4-nitro-aniline /DMSO | 4-chloro aniline /DMSO | 4-nitro-aniline /benzene | 4-chloro-aniline /benzene |
| B-1 | $Ca(NO_3)_2$/NaY | ---- | | -- | | | | |
| B-2 | $NH_3$/Ca/NaY | ---- | | 7 | yellow | nd | nd | white |
| B-3 | Lime wash/Ca/NaY | ---- | | 7 | yellow | nd | nd | white |
| B-4 | Ca(OH)x/NaY | ---- | | 8 | yellow | nd | nd | cream |
| B-5 | $Ca(NO_3)_2$/NaY | ---- | .02 | 7 | yellow | nd | nd | cream |
| B-6 | | | .181 | 9 | lt yell/grn | nd | nd | cream |
| B-7 | $Mg(NO_3)_2$/NaY | ---- | .072 | 8 | yellow | nd | nd | cream |
| B-8 | | ---- | .281 | 9 | yellow/grn | nd | nd | cream |
| B-9 | $Mg(OAc)_2$/NaY | ---- | .09 | 8 | yellow | nd | nd | cream |
| B-10 | | ---- | .287 | 10 | dk blue/grn | nd | nd | bwn/amber |
| B-11 | $Ca(OAc)_2$/LZY52plt | --- | 0.347 | 11 | kelly green | nd | nd | dk brown |
| B-12 | $Ca(OAc)_2$/LZY82plt | --- | 0.34 | 10 | yellow | nd | nd | dk brown |
| B-13 | $Ca(OAc)_2$/LZY62plt | --- | 0.34 | 8 | blk/yell liq | nd | nd | dk/amber liq |

Table 4 (cont'd)

Illustrative Embodiments, (Continued)

(Footnotes are those of Table 3)

| --Description--- | --Structure-- | | ------------Basicity Resulting------------ | | | | |
|---|---|---|---|---|---|---|---|
| CATALYST System Designation (Salt/Zeolite) | n-C wet- ness cc/g f) | Pore vol. cc/g g) | pH 1% aq. h) | ---------- INDICATOR COLORS -----(i)----- 4-nitro-aniline /DMSO | 4-chloro aniline /DMSO | 4-nitro-aniline /benzene | 4-chloro-aniline /benzene |
| B-14 $Ca(OAc)_2$/LZY62plt | --- | 0.33 | 6 | dk lime grn | nd | nd | brown |
| $B\text{-}15_1$ $Ca(OAc)_2$/LZY82plt | -- | 0.34 | 9 | lime green | nd | nd | cream |
| $B\text{-}15_2$ $NH_4OH$/Ca/LZY82plt | | | | | | | |
| $B\text{-}15_3$ $Ca(OAc)_2$/LZY82plt | | | | | | | |
| B-16 | | | | | | | |
| B-17 | ---- | 0.33 | 9 | gray/green | nd | nd | gray/bwn |
| B-18 $Mg(OAc)_2$/LZY82plt | | | | | | | |
| $NH_4OH$ on above | | | | | | | |
| B-19 $NH_4H$ on above | | | | | | | |
| B-20 | ---- | 0.349 | 9 | gray/grn liq | nd | nd | gray&bwn |
| B-21 $Ca(OAc)_2$/LZY82pwd | | | | | | | |
| $NH_4OH$ on above | | | | | | | |
| B-22 $NH_4OH$ on above | | | | | | | |
| B-23 | ---- | 0.296 | 8 | dk/goldenliq | nd | nd | dk bwn |

Table 4 (cont'd)

Illustrative Embodiments, (Continued)

(Footnotes are those of Table 3)

| --Description--- CATALYST System Designation (Salt/Zeolite) | --Structure-- n-C wet- ness cc/g f) | Pore vol. cc/g g) | pH 1% aq. h) | Basicity Resulting / INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|
| B-24 $Mg(OAc)_2$/LZY82pwd redistribute salt $NH_4OH$ on above $NH_4OH$ on above | ---- | 0.294 | 9 | dark/dk golden liq | dk-purple | black | dark |
| B-25 $Ca(OAc)_2$/Erionit | ---- ---- | 0.06 | 9 | dark green | nd | nd | dark |
| B-26 $Ca(OAc)_2$/Mordent | ---- ---- | 0.29 | 9 | dark green | nd | nd | lt bwn |

Table 4

Illustrative Embodiments,
(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. sq.m./g e) |
| C-1 | $Ca(OAc)_2$/Chabaz. | $H_2O$ | .03 | .01 | .11 | 150 | I | GG | | |
| C-2 | | -----sample above----- | | | | 550 | C | GG | 21 | -- |
| C-3 | $Mg(OAc)_2$/NaMord. | $H_2O$ | .33 | .67 | 4.01 | 150 | I | CC | | |
| | | $H_2O$ | 1.0 | 0 | above | 150 | "I" | FF | | |
| | | -----sample above----- | | | | 550 | C | FF | 93.2 | 21 |
| C-4 | $Ca(OAc)_2$/NaMord. | $H_2O$ | .74 | .26 | 4.0 | 150 | I | GG | | |
| | | $H_2O$ | 1.0 | 0 | above | 150 | "I" | HH | | |
| | | -----sample above---- | | | | 550 | C | HH | 89.6 | 19 |
| C-5 | $Mg(OAc)_2$/WshNaMord | $H_2O$ | .33 | .67 | 4.00 | 150 | I | GG | | |
| | | $H_2O$ | 1.00 | 0 | above | 150 | "I" | HH | | |
| | | -----sample above---- | | | | 550 | C | HH | 96.4 | 122 |
| C-6 | $Ca(OAc)_2$/WshNaMord | $H_2O$ | .74 | .26 | 4.06 | 150 | I | GG | | |
| | | $H_2O$ | 1.00 | 0 | above | 150 | "I" | HH | | |
| | | -----sample above---- | | | | 550 | C | HH | 81.7 | 171 |

Table 4 (cont'd)

Illustrative Embodiments,
(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| C-7 $Mg(OAc)_2/NH_4$Morden. | $H_2O$ | 40cc | 2Molar | 4.02 | 23 | S | II | | |
| | $H_2O$ | 2x50 | 0 | -above | 150 | W | II | ---- | ---- |
| | $H_2O$ | 40cc | 2Molar | -above | 23 | S | II | | |
| | $H_2O$ | 2x50 | 0 | -above | 150 | W | II | ---- | ---- |
| | $H_2O$ | 40cc | 2Molar | -above | 23 | S | II | | |
| C-8 | $H_2O$ | 2x50 | 0 | -above | 150 | W | II | 108 | 326 |
| C-9 $Ca(OAc)_2/NH_4$Morden. | $H_2O$ | 40cc | 2Molar | 4.01 | 23 | S | II | | |
| | $H_2O$ | 2x50 | 0 | -above | 150 | W | II | ---- | ---- |
| | $H_2O$ | 40cc | 2Molar | -above | 23 | S | II | | |
| | $H_2O$ | 2x50 | 0 | -above | 150 | W | II | ---- | ---- |
| | $H_2O$ | 40cc | 2Molar | -above | 23 | S | II | | |
| C-10 | $H_2O$ | 2x50 | 0 | -above | 150 | W | II | 94 | 340 |

Table 4 (cont'd)

Illustrative Embodiments,

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| C-11 $Mg(OAc)_2$/LZY82 | $H_2O$ | 40cc | 2Molar | 4.03 | 23 | S | II | | |
| | $H_2O$ | 2x50 | 0 | -above | 150 | W | II | ---- | ---- |
| | $H_2O$ | 40cc | 2Molar | -above | 23 | S | II | | |
| | $H_2O$ | 2x50 | 0 | -above | 150 | W | II | ---- | ---- |
| | $H_2O$ | 40cc | 2Molar | -above | 23 | S | II | | |
| C-12 | $H_2O$ | 2x50 | 0 | -above | 150 | W | II | 130 | 552 |
| C-13 $Ca(OAc)_2$/LZY82 | $H_2O$ | 40cc | 2Molar | 4.03 | 23 | S | II | | |
| | $H_2O$ | 2x50 | 0 | -above | 150 | W | II | ---- | ---- |
| | $H_2O$ | 40cc | 2Molar | -above | 23 | S | II | | |
| | $H_2O$ | 2x50 | 0 | -above | 150 | W | II | ---- | ---- |
| | $H_2O$ | 40cc | 2Molar | -above | 23 | S | II | | |
| C-14 | $H_2O$ | 2x50 | 0 | -above | 150 | W | II | 119 | 549 |

Table 4

Illustrative Embodiments, (Continued)

(Footnotes are those of part A.)

| --Description--- | | --Structure-- | | ------------Basicity Resulting------------ | | | | |
|---|---|---|---|---|---|---|---|---|
| | CATALYST System Designation (Salt/Zeolite) | n-C8 wet-ness cc/g f) | Pore vol. cc/g g) | pH 1% aq. h) | 4-nitro-aniline /DMSO | 4-chloro aniline /DMSO | 4-nitro-aniline /benzene | 4-chloro-aniline /benzene |
| | | | | | --------- INDICATOR COLORS --(i) | | | ---- |
| C-1 | $Ca(OAc)_2$/Chabaz. | | | | | | | |
| C-2 | | ---- | --- | 7 | yellow | nd | nd | white |
| C-3 | $Mg(OAc)_2$/NaMord. | ---- | | | | | | |
| | | ---- | | | | | | |
| | | ---- | 0.03 | 8 | lt green | nd | nd | pale lav. |
| C- 4 | $Ca(OAc)_2$/NaMord. | ---- | | | | | | |
| | | ---- | | | | | | |
| | | ---- | 0.03 | 7 | lime green | nd | nd | cream |
| C-5 | $Mg(OAc)_2$/WshNaMord | -- | | | | | | |
| | | ---- | | | | | | |
| | | ---- | 0.08 | 9 | lime&dk green | nd | nd | lt lav. &bwn |
| C-6 | $Ca(OAc)_2$/WshNaMord | -- | | | | | | |
| | | ---- | | | | | | |
| | | ---- | 0.1 | 9 | lime green | nd | nd | lt lav. &bwn |

Table 4 (cont'd)

Illustrative Embodiments, (Continued)

(Footnotes are those of part A.)

| --Description--- | --Structure-- | | ------------Basicity Resulting------------- | | | | |
|---|---|---|---|---|---|---|---|
| CATALYST System Designation (Salt/Zeolite) | n-C8 wet-ness cc/g f) | Pore vol. cc/g g) | pH 1% aq. h) | INDICATOR COLORS --(i) 4-nitro-aniline /DMSO | 4-chloro aniline /DMSO | 4-nitro-aniline /benzene | 4-chloro-aniline /benzene |
| C-7 $Mg(OAc)_2/NH_4$Morden | | | | | | | |
| | ---- | --- | 6 | nd | nd | nd | nd |
| | ---- | | | | | | |
| | ---- | --- | 6 | nd | nd | nd | nd |
| | ---- | | | | | | |
| C-8 | ---- | 0.18 | 6 | yellow | nd | nd | cream |
| C-9 $Ca(OAc)_2/NH_4$Morden. | | | | | | | |
| | ---- | --- | 6 | nd | nd | nd | nd |
| | ---- | | | | | | |
| | ---- | --- | 6 | nd | nd | nd | nd |
| | ---- | | | | | | |
| C-10 | ---- | 0.19 | 6 | yellow | nd | nd | cream |

Table 4 (cont'd)

Illustrative Embodiments, (Continued)

(Footnotes are those of part A.)

| --Description--- | --Structure-- | | ------------Basicity Resulting------------- | | | | |
|---|---|---|---|---|---|---|---|
| CATALYST System Designation (Salt/Zeolite) | n-C8 wet-ness cc/g f) | Pore vol. cc/g g) | pH 1% aq. h) | ---------- INDICATOR COLORS --(i) ---- 4-nitro-aniline /DMSO | 4-chloro aniline /DMSO | 4-nitro-aniline /benzene | 4-chloro-aniline /benzene |
| C11 $Mg(OAc)_2$/LZY82 | | | | | | | |
| | ---- ---- | --- | 6 | nd | nd | nd | nd |
| | ---- ---- | --- | 6 | nd | nd | nd | nd |
| C-12 | 0.557 | 0.314 | 6 | yellow | nd | nd | cream |
| C-13 $Ca(OAc)_2$/LZY82 | | | | | | | |
| | ---- ---- | --- | 6 | nd | nd | nd | nd |
| | ---- ---- | --- | 6 | nd | nd | nd | nd |
| C-14 | 0.748 | 0.317 | 6 | yellow | nd | nd | cream |

## Table 4

Illustrative Embodiments,

(Footnotes are those of Table 3)

| | --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. q.m. /g e) |
|---|---|---|---|---|---|---|---|---|---|---|
| D-1 | $Mg(OAc)_2$/WshNaY | $H_2O$ | 8.5 | 7.0 | 15.02 | 550 | I | CC | 97.3 | 573 |
| D-2 | $Ca(OAc)_2$/WshNaY | $H_2O$ | 15 | 5.14 | 15.03 | 150 | I | JJ | | |
| | $NH_4OH$ on above.. | 6cc of 10N | | | | | I/P | JJ | | |
| | | 22cc of 1N | | | | | W | JJ | | |
| | | -----above sample----- | | | | 550 | C | JJ | 88.4 | 619 |
| D-3 | $Ca(NO_3)_2$/NaY | $H_2O$ | 8.5 | 20.5 | 50.04 | 23 | K | | | |
| | $K_2CO_3$ on above | $H_2O$ | 10.8 | 15.2 | above | 60 | I/P | KK | | |
| D-4 | Lime wash of above | $H_2O$ | 400 | 0.56 | above | 100 | W | KK | 63 | --- |
| D-5 | | -----above sample------ | | | | 550 | C | KK | 50 | --- |
| D-6 | | -----above sample------ | | | | 700 | C/C | KK | low | |

Table 4

Illustrative Embodiments,

(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. q.m. /g e) |
| D-7 | $Mg(OAc)_2$/WshKL | $H_2O$ | 1.51 | 0.93 | 2.00 | 150 | I | CC | 44.1 | 18 |
| D-8 | | -----above sample------ | | | | 550 | C | CC | 94.2 | 34 |
| D-9 | $Mg(OAc)_2$/Mordenit | $H_2O$ | 0.99 | 0.62 | 1.32 | 150 | I | CC | 96.7 | 135 |
| D-10 | | -----above sample------ | | | | 550 | C | CC | 88.7 | 163 |
| D-11 | $Mg(OAc)_2$/WshNaMord | $H_2O$ | 2.32 | 2.44 | 3.08 | 150 | I | CC | | |
| D-12 | | $H_2O$ | 1 | 0 | above | 150 | "I" | FF | 94.0 | 32 |
| D-13 | | -----above sample------ | | | | 550 | C | FF | 85.1 | 18 |

Table 4 (cont'd)

Illustrative Embodiments,

(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. q.m. /g e) |
| D-14 | $Mg(OAc)_2/NH_4$Morden | $H_2O$ | 1.90 | 1.18 | 2.53 | 150 | I | CC | 79.6 | 88 |
| D-15 | | -----above sample------ | | | | 550 | C | CC | 43.4 | 197 |
| D-16 | Mg(OAc)/LZY82pwdr | $H_2O$ | 1.98 | 1.23 | 2.62 | 150 | I | CC | 83.8 | 351 |
| D-17 | | -----above sample----- | | | | 550 | C | CC | 76.0 | 455 |
| D-18 | $Mg(OAc)_2/NH_4$Ferri. | $H_2O$ | 0.99 | 0.61 | 1.36 | 150 | I | CC | 77.6 | 179 |
| D-19 | | -----above sample------ | | | | 550 | C | CC | 109.9 | 257 |
| D-20 | $Mg(OAc)_2$/KL | $H_2O$ | 1.61 | 0.99 | 2.20 | 150 | I | CC | 74.8 | 21 |
| D-21 | | -----above sample------ | | | | 550 | C | CC | 89.4 | 29 |
| D-22 | $Mg(OAc)_2$/NH VUSY | $H_2O$ | 1.65 | 1.01 | 2.14 | 150 | I | CC | 83.1 | 399 |
| D-23 | | -----sample above------ | | | | 550 | C | CC | 75.7 | 485 |

Table 4 (cont'd)

Illustrative Embodiments,

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. q.m. /g e) |
|---|---|---|---|---|---|---|---|---|---|
| D-24 $Mg(OAc)_2$/Chabazite | $H_2O$ | 0.86 | 0.53 | 1.13 | 150 | I | CC | 32.9 | 268 |
| D-25 | -----sample above------ | | | | 550 | C | CC | 83.6 | 347 |
| D-26 $Mg(OAc)_2$/LZY82plts | $H_2O$ | 2.50 | 3.10 | 3.52 | 150 | I | CC | | |
| | $H_2O$ | 1 | 0 | -above- | 150 | "I" | LL | | |
| D-27 | $H_2O$ | 1 | 0 | -above- | 150 | "I" | LL | 37 | 278 |
| D-28 | -----sample above------ | | | | 550 | C | LL | 60 | 399 |

Table 4 (cont'd)

Illustrative Embodiments,

(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. q.m./g e) |
| D-29 | $Mg(OAc)_2/NH_4USY$ | $H_2O$ | 1.96 | 2.44 | 2.60 | 150 | I | MM | 80 | 48 |
| D-30 | | -----above sample------ | | | | 550 | C | MM | 57 | 402 |
| D-31 | $Ca(OAc)_2$/WshNaMord | $H_2O$ | 2.26 | 0.72 | 2.09 | 150 | I | CC | 64 | 11 |
| D-32 | | -----sample above------ | | | | 550 | C | CC | 69 | 109 |
| D-33 | $Ca(OAc)_2$/Mordenite | $H_2O$ | 1.63 | 0.52 | 1.49 | 150 | I | GG | 86 | 83 |
| D-34 | | -----sample above------ | | | | 550 | C | GG | 78 | 126 |
| D-35 | $Ca(OAc)_2$/LZY82pwdr | $H_2O$ | 3.16 | 1.00 | 2.83 | 150 | I | CC | 68 | 401 |
| D-36 | | -----above sample------ | | | | 550 | C | CC | 63 | 463 |

Table 4

Illustrative Embodiments, (Continued)

(Footnotes are those of Table 3)

| --Description--- | | --Structure-- | | --Basicity Resulting-- | | | | |
|---|---|---|---|---|---|---|---|---|
| | CATALYST System Designation (Salt/Zeolite) | n-C8 wet-ness cc/g f) | Pore vol. cc/g g) | pH 1% aq. h) | INDICATOR COLORS (i) 4-nitro-aniline /DMSO | 4-chloro aniline /DMSO | 4-nitro-aniline /benzene | 4-chloro-aniline /benzene |
| D-1 | $Mg(OAc)_2$/WshNaY | ---- | 0.32 | 11 | dark grn/lt grn liq | nd | nd | lt lavender |
| D-2 | $Ca(OAc)_2$/WshNaY | ---- | | | | | | |
| | $NH_4OH$ on above | ---- | | | | | | |
| | | ---- | | | | | | |
| | | ---- | 0.33 | 8 | dark green | nd | nd | dark brown |
| D-3 | $Ca(NO_3)_2$/NaY | | | | | | | |
| | $K_2CO_3$ on above | | | | | | | |
| D-4 | Lime wash of above | -- | --- | 9 | yellow | nd | nd | white |
| D-5 | | ---- | --- | 9 | yellow&green | nd | nd | white |
| D-6 | | | | | | | | |
| D-7 | $Mg(OAc)_2$/WshKL | ---- | 0.064 | 7 | blue green | nd | nd | cream |
| D-8 | | ---- | 0.092 | | dark green | nd | nd | pale tan |
| D-9 | $Mg(OAc)_2$/Mordenit | -- | 0.277 | 8 | lt lime green | nd | nd | cream |

Table 4 (cont'd)

Illustrative Embodiments, (Continued)

(Footnotes are those of Table 3)

| --Description--- CATALYST System Designation (Salt/Zeolite) | | --Structure-- n-C8 wetness cc/g f) | Pore vol. cc/g g) | pH 1% aq. h) | 4-nitroaniline /DMSO | 4-chloro aniline /DMSO | 4-nitroaniline /benzene | 4-chloroaniline /benzene |
|---|---|---|---|---|---|---|---|---|
| D-10 | | ---- | 0.299 | 8 | dk grn/yell liq | nd | nd | dk purple |
| D-11 | $Mg(OAc)_2$/WshNaMord | | | | | | | |
| D-12 | | ---- | 0.052 | 8 | pale green | nd | nd | cream |
| D-13 | | ---- | 0.037 | 8 | dark green | nd | nd | lt lavender |
| D-14 | $Mg(OAc)_2$/NH Morden | - | 0.077 | 9 | lt&dk green | nd | nd | cream |
| D-15 | | ---- | 0.128 | 8 | dark green | nd | nd | lt gray |
| D-16 | $Mg(OAc)_2$/LZY82pwdr | - | 0.219 | 6 | yellow | nd | nd | cream |
| D-17 | | ---- | 0.274 | 8 | black/green | nd | nd | dk purple |
| D-18 | $Mg(OAc)_2/NH_4$Ferri. | - | 0.10 | 9 | pale green | nd | nd | white |
| D-19 | | ---- | 0.17 | 9 | lt/dk green | nd | nd | cream/yell |
| D-20 | $Mg(OAc)_2$/KL | ---- | 0.07 | 8 | pale blue/grn | nd | nd | white |
| D-21 | | ---- | 0.07 | 10 | dk blue/grn | nd | nd | lavender |
| D-22 | $Mg(OAc)_2/NH_4$VUSY | --- | 0.32 | 8 | yellow | nd | nd | cream |
| D-23 | | ---- | 0.38 | 8 | dark green | nd | nd | lavender |
| D-24 | $Mg(OAc)_2$/Chabazite | - | 0.13 | 8 | yellow grn | nd | nd | cream |

Basicity Resulting INDICATOR COLORS (i)

Table 4 (cont'd)

Illustrative Embodiments, (Continued)

(Footnotes are those of Table 3)

| --Description--- | | --Structure-- | | ------------Basicity Resulting------------- | | | | |
|---|---|---|---|---|---|---|---|---|
| | CATALYST System Designation (Salt/Zeolite) | n-C8 wet-ness cc/g f) | Pore vol. cc/g g) | pH 1% aq. h) | INDICATOR COLORS ---(i)----- 4-nitro-aniline /DMSO | 4-chloro aniline /DMSO | 4-nitro-aniline /benzene | 4-chloro-aniline /benzene |
| D-25 | | ---- | 0.17 | 9 | dk blue grn | nd | nd | light tan |
| D-26 | $Mg(OAc)_2$/LZY82plts | | | | | | | |
| D-27 | | ---- | 0.23 | 8 | white plts/grn liq | nd | nd | white |
| D-28 | | ---- | 0.32 | 9 | dark-green | purple | dk-purple | dark plts |
| D-29 | $Mg(OAc)_2/NH_4USY$ | ---- | 0.07 | 7 | yellow | nd | nd | white |
| D-30 | | ---- | 0.26 | 9 | blackish green | nd | nd | dark purple |
| D-31 | $Ca(OAc)_2$/WshNaMord | -- | 0.03 | 8 | yellow grn | nd | nd | white |
| D-32 | | ---- | 0.08 | 10 | black/golden liq | nd | nd | dk purp&lvd |
| D-33 | $Ca(OAc)_2$/Mordenite | -- | 0.25 | 7 | yellow | nd | nd | cream |
| D-34 | | ---- | 0.26 | 9 | black&green | nd | nd | deep purple |
| D-35 | $Ca(OAc)_2$/LZY82pwdr | -- | 0.25 | 6 | yellow | nd | nd | cream |
| D-36 | | ---- | 0.29 | 8 | dark green | nd | nd | deep purple |

Table 4

Illustrative Embodiments,

(Footnotes are those of Table 3)

| --Description--- | | ----Materials used----- | | | | -----Conditions----- | | | -Structure- | |
|---|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| E-1 | $Ca(OAc)_2$/WshKL | $H_2O$ | 3.76 | 1.19 | 3.47 | 150 | I | CC | 69 | 28 |
| E-2 | | ----sample above----- | | | | 550 | C | CC | 79 | 61 |
| E-3 | $Ca(OAc)_2/NH_4$Morden | $H_2O$ | 3.01 | 0.95 | 2.76 | 150 | I | CC | 64 | 182 |
| E-4 | | -----sample above---- | | | | 550 | C | CC | 50 | 242 |
| E-5 | $Ca(OAc)_2$/KL | $H_2O$ | 3.88 | 1.23 | 3.58 | 150 | I | CC | 60 | 17 |
| E-6 | | -----Sample above---- | | | | 550 | C | CC | 75 | 88 |
| E-7 | $Ca(OAc)_2/NH_4$Ferrie | $H_2O$ | 1.75 | 0.55 | 1.61 | 150 | I | CC | 59 | 180 |
| E-8 | | -----sample above---- | | | | 550 | C | CC | 88 | 226 |
| E-9 | $Ca(OAc)_2$/LZY82plts | $H_2O$ | 5.53 | 1.76 | 5.11 | 150 | I | CC | 57.6 | 276 |
| E-10 | | -----sample above---- | | | | 550 | C | CC | 58.3 | 429 |
| E-11 | $Ca(OAc)_2/NH_4$VUSY | $H_2O$ | 2.44 | 0.77 | 2.25 | 150 | I | CC | 50.6 | -- |
| E-13 | | ----sample above---- | | | | 550 | C | CC | 60.6 | 468 |

Table 4

Illustrative Embodiments,
(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| E-14 $Ca(OAc)_2$/Chabazite | $H_2O$ | 0.76 | 0.24 | 1.04 | 150 | I | CC | 88.2 | 265 |
| E-15 | ----sample above---- | | | | 550 | C | CC | 38.9 | 20 |
| E-16 $Ca(OAc)_2/NH_4USY$ | $H_2O$ | 2.74 | 0.87 | 2.53 | 150 | I | CC | 75.2 | 429 |
| E-17 | ----sample above---- | | | | 550 | C | CC | 59.5 | 496 |
| E-18 $Sr(OAc)^2$/WshNaY | $H_2O$ | 2.53 | 0.99 | 2.11 | 150 | I | CC | 73 | 182 |

Table 4 (cont'd)

Illustrative Embodiments,
(Footnotes are those of Table 3)

| --Description--- | ----Materials used----- | | | | -----Conditions----- | | | -Structure- | |
|---|---|---|---|---|---|---|---|---|---|
| CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| E-19 | ----sample above---- | | | | 550 | C | CC | 49 | 361 |
| E-20 $Sr(OAc)_2$/WshNaMord | $H_2O$ | 2.43 | 0.95 | 2.02 | 150 | I | CC | 70 | 14 |
| E-21 | ----sample above---- | | | | 550 | C | CC | 48 | 14 |
| E-22 $Sr(OAc)_2/NH_4$Morden | $H_2O$ | 2.54 | 1.00 | 2.11 | 150 | I | CC | 66.9 | 186 |
| E-23 | ----above material-- | | | | 550 | C | CC | 55 | 133 |
| E-24 $Sr(OAc)_2$/LZY82plts | $H_2O$ | 5.02 | 1.97 | 4.17 | 150 | I | CC | 43.1 | 272 |
| E-25 | ----sample above---- | | | | 550 | C | CC | 54.4 | 382 |
| E-26 $Sr(OAc)_2$/LZY82pwdr | $H_2O$ | 2.2 | 0.86 | 1.82 | 150 | I | CC | 52.7 | 310 |
| E-27 | ----sample above---- | | | | 550 | C | CC | 47.7 | 381 |
| E-28 $Ba(OAc)_2$/LZY82pwdr | $H_2O$ | 1.78 | 1.27 | 2.23 | 150 | I | CC | 22.6 | 247 |
| E-29 | ----sample above---- | | | | 550 | C | CC | 13.9 | 354 |

Table 4 (cont'd)

Illustrative Embodiments,
(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| E-30 $Ba(OAc)_2/NH_4$Morden | $H_2O$ | 1.63 | 1.15 | 1.92 | 150 | I | CC | 34.4 | 66 |
| E-31 | ----sample above---- | | | | 550 | C | CC | 25.3 | 102 |
| E-32 $Ba(OAc)_2$/WshNaMord | $H_2O$ | 1.52 | 1.08 | 1.82 | 150 | I | CC | 35 | 7 |
| E-33 | ----sample above---- | | | | 550 | C | CC | 24.4 | 23 |
| E-34 $Ba(OAc)_2$/LZY82plts | $H_2O$ | 2.77 | 1.96 | 3.49 | 150 | I | CC | 22.5 | 227 |

Table 4 (cont'd)

Illustrative Embodiments,
(Footnotes are those of Table 3)

| --Description--- | | ----Materials used----- | | | -----Conditions----- | | | -Structure- | |
|---|---|---|---|---|---|---|---|---|---|
| | CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| E-35 | | ----sample above---- | | | | 550 | C | CC | 18.9 | 381 |
| E-36 | $Ba(OAc)_2$/WshNaY | $H_2O$ | 1.46 | 1.03 | 1.82 | 150 | I | CC | 18.2 | 82 |
| E-37 | | ----sample above---- | | | | 550 | C | CC | 10 | 234 |
| E-38 | $Mg(OAc)_2$/Mg/$NH_4$Mord | MeOH | 0.3 | 0.21 | 1.00 | 150 | I | NN | 86.7 | 143 |
| E-39 | | ----sample above---- | | | | 550 | C | NN | 46.7 | 265 |
| E-40 | $Mg(OAc)_2$/Mg/$NH_4$Mord | MeOH | 1.0 | 0.7 | 1.04 | 150 | I | NN | --- | -- |
| E-41 | | MeOH | 0.5 | -above----- | | 150 | "I" | RR | 79.1 | nd |
| E-42 | | ----sample above---- | | | | 550 | C | RR | 45.9 | 258 |
| E-43 | $Mg(OAc)_2$/Mg/LZY82 | MeOH | 0.3 | 0.2 | 1.00 | 150 | I | NN | 75.6 | nd |
| E-44 | | ----sample above---- | | | | 550 | C | NN | 81 | 493 |
| E-45 | $Mg(OAc)_2$/$Mg(OAc)_2$/ | MeOH | 0.6 | 0.4 | 1.00 | 150 | I | NN | | |

Table 4 (cont'd)

Illustrative Embodiments,
(Footnotes are those of Table 3)

| --Description--- CATALYST System Designation (Salt/Zeolite) | | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|---|
| E-46 | LZY82 | MeOH | 0.5 | -sample above- | | 150 | "I" | RR | 66.6 | 277 |
| E-47 | | -----sample above----- | | | | 550 | C | RR | 65.3 | 387 |
| E-48 | $Mg(OAc)_2$/$Mg(OAc)_2$/ | DMSO | 0.25 | 0.15 | 1.07 | 200 | I | OO | 56.3 | 125 |
| E-49 | $NH_4$+Mordenite | ----sample above----- | | | | 550 | C | OO | 53.9 | 264 |
| E-50 | $Mg(OAc)_2$/$Mg(OAc)_2$/ | DMSO | 0.25 | 0.15 | 1.02 | 200 | I | OO | 41.8 | 363 |
| E-51 | LZY82 | ----sample above----- | | | | 550 | C | OO | 74.8 | 452 |

Table 4

Illustrative Embodiments, (Continued)

(Footnotes are those of Table 3)

| --Description--- CATALYST System Designation (Salt/Zeolite) | | --Structure-- n-C8 wetness cc/g f) | Pore vol. cc/g g) | --Basicity Resulting-- pH 1% aq. h) | INDICATOR COLORS --(i)-- 4-nitroaniline /DMSO | 4-chloroaniline /DMSO | 4-nitroaniline /benzene | 4-chloroaniline /benzene |
|---|---|---|---|---|---|---|---|---|
| E-1 | $Ca(OAc)_2$/WshKL | ---- | 0.074 | 7 | dk grn/yell liq | nd | nd | cream |
| E-2 | | ---- | 0.091 | 11 | dark-green | dk-pink/bwn | bwn | deep-purple |
| E-3 | $Ca(OAc)_2$/NH4Morden | --- | 0.11 | 7 | yellow | nd | nd | white |
| E-4 | | ---- | 0.154 | 8 | dark grn/gold | nd | nd | deep purple |
| E-5 | $Ca(OAc)_2$/KL | ---- | 0.04 | 7 | lime green | nd | nd | white |
| E-6 | | ---- | 0.091 | 11 | dark green | nd | nd | deep purple |
| E-7 | $Ca(OAc)_2$/$NH_4$Ferrie | --- | 0.129 | 7 | yellow | nd | nd | white |
| E-8 | | ---- | 0.165 | 9 | black/golden | nd | nd | deep purple |
| E-9 | $Ca(OAc)_2$/LZY82plts | --- | 0.232 | 7 | yellow | nd | nd | white |
| E-10 | | ---- | 0.332 | 7 | black/yellow | nd | nd | dark |
| E-11 | $Ca(OAc)_2$/$NH_4$VUSY | ---- | --- | 7 | yellow | nd | nd | white |
| E-13 | | ---- | 0.392 | 9 | dark green | nd | nd | deep purple |

Table 4

Illustrative Embodiments, (Continued)

(Footnotes are those of Table 3)

| --Description--- | --Structure-- | | ------------Basicity Resulting------------ | | | | |
|---|---|---|---|---|---|---|---|
| CATALYST System Designation (Salt/Zeolite) | n-C8 wet-ness cc/g f) | Pore vol. cc/g g) | pH 1% aq. h) | ----------- INDICATOR COLORS --(i)---- 4-nitro-aniline /DMSO | 4-chloro aniline /DMSO | 4-nitro-aniline /benzene | 4-chloro-aniline /benzene |
| E-14 $Ca(OAc)_2$/Chabazite | --- | 0.12 | 7 | yellow | nd | nd | white |
| E-15 | ---- | 0.02 | 7 | black/yellow | nd | nd | deep purple |
| E-16 $Ca(OAc)_2/NH_4USY$ | ---- | 0.266 | 6 | yellow | nd | nd | cream |
| E-17 | ---- | 0.313 | 7 | blackish green | nd | nd | dark purple |

Table 4 (cont'd)

Illustrative Embodiments, (Continued)

(Footnotes are those of Table 3)

| --Description--- | | --Structure-- | | ------------Basicity Resulting------------ | | | | |
|---|---|---|---|---|---|---|---|---|
| | CATALYST System Designation (Salt/Zeolite) | n-C8 wetness cc/g f) | Pore vol. cc/g g) | pH 1% aq. h) | 4-nitroaniline /DMSO | 4-chloro aniline /DMSO | 4-nitro-aniline /benzene | 4-chloro-aniline /benzene |
| | | | | | ----------- INDICATOR COLORS --(i)---- | | | |
| E-18 | $Sr(OAc)_2$/WshNaY | ---- | 0.12 | 8 | yellow | nd | nd | cream |
| E-19 | | ---- | 0.21 | 11 | dk-green | dk-purple | dk-olive | dk-purple |
| E-20 | $Sr(OAc)_2$/WshNaMord | --- | 0.23 | 8 | lime green | nd | nd | white |
| E-21 | | ---- | 0.24 | 9 | blck/gold | dk-purple | dk-purple | dk-purple |
| E-22 | $Sr(OAc)_2$/$NH_4$Morden | --- | 0.12 | 8 | yellow | nd | nd | white |
| E-23 | | ---- | 0.09 | 9 | dk grn/gold liq | dark purple | dark purple | dark purple |
| E-24 | $Sr(OAc)_2$/LZY82plts | --- | 0.22 | 6 | yellow | nd | nd | white |
| E-25 | | ---- | 0.294 | 8 | dk grn/gold liq | gray | bwn | purple |
| E-26 | $Sr(OAc)_2$/LZY82pwdr | --- | 0.19 | 6 | yellow | nd | nd | cream |
| E-27 | | ---- | 0.234 | 8 | dark green | nd | nd | dark purple |
| E-28 | $Ba(OAc)_2$/LZY82pwdr | --- | 0.16 | 6 | yellow | nd | nd | cream |
| E-29 | | ---- | 0.22 | 8 | black-green | nd | nd | dark purple |
| E-30 | $Ba(OAc)_2$/$NH_4$Morden | --- | 0.05 | 7 | yellow | nd | nd | white |
| E-31 | | ---- | 0.08 | 8 | black/gold liq | nd | nd | dark purple |

Table 4 (cont'd)

Illustrative Embodiments, (Continued)

(Footnotes are those of Table 3)

| --Description--- CATALYST System Designation (Salt/Zeolite) | | --Structure-- n-C8 wet-ness cc/g f) | Pore vol. cc/g g) | Basicity Resulting pH 1% aq. h) | INDICATOR COLORS (i) 4-nitro-aniline /DMSO | 4-chloro aniline /DMSO | 4-nitro-aniline /benzene | 4-chloro-aniline /benzene |
|---|---|---|---|---|---|---|---|---|
| E-32 | $Ba(OAc)_2$/WshNaMord | --- | 0.02 | 8 | lt lime green | nd | nd | white |
| E-33 | | ---- | 0.03 | 8 | dk-green | dk-purple | dk-purple | dk-purple |
| E-34 | $Ba(OAc)_2$/LZY82plts | --- | 0.20 | 7 | yellow | nd | nd | white |
| E-35 | | ---- | 0.291 | 8 | black/gold liq | nd | nd | dark purple |
| E-36 | $Ba(OAc)_2$/WshNaY | ---- | 0.07 | 7 | yellow | nd | nd | white |
| E-37 | | ---- | 0.139 | 10 | dk green | nd | nd | dk purple |
| E-38 | $Mg(OAc)_2$/Mg/NHMord | --- | 0.091 | 7 | yellow | nd | nd | cream |
| E-39 | | ---- | 0.15 | 8 | black/gold liq | nd | nd | dark purple |
| E-40 | $Mg(OAc)_2$/Mg/$NH_4$Mord. | - | --- | | | | | |
| E-41 | | ---- | nd | 9 | blue green | nd | nd | cream |
| E-42 | | ---- | 0.16 | 8 | black/gold liq | nd | nd | black |
| E-43 | $Mg(OAc)_2$/Mg/LZY82 | ---- | nd | 7 | yellow | nd | nd | cream |
| E-44 | | ---- | 0.302 | 7 | black/gold liq | nd | nd | dk purple |
| E-45 | $Mg(OAc)_2$/$Mg(OAc)_2$/ | | | | | | | |

Table 4 (cont'd)

Illustrative Embodiments, (Continued)

(Footnotes are those of Table 3)

| --Description--- |  | --Structure-- |  | ---Basicity Resulting--- |  |  |  |  |
|---|---|---|---|---|---|---|---|---|
|  | CATALYST System Designation (Salt/Zeolite) | n-C8 wetness cc/g f) | Pore vol. cc/g g) | pH 1% aq. h) | INDICATOR COLORS --(i)-- 4-nitroaniline /DMSO | 4-chloro aniline /DMSO | 4-nitro-aniline /benzene | 4-chloro-aniline /benzene |
| E-46 | LZY82 | ---- | 0.182 | 7 | green | nd | nd | cream |
| E-47 |  | ---- | 0.237 | 8 | black/gold liq | nd | nd | dark purple |
| E-48 | $Mg(OAc)_2/Mg(OAc)_2/$ | --- | 0.09 | 7 | yellow | nd | nd | cream |
| E-49 | $NH_4$+Mordenite | -- | 0.16 | 8 | black/gold liq | nd | nd | black |
| E-50 | $Mg(OAc)_2/Mg(OAc)_2/$ | ---- | 0.22 | 7 | yellow | nd | nd | cream |
| E-51 | LZY82 | ---- | 0.28 | 7 | black/gold liq | nd | nd | black |

Table 4

Illustrative Embodiments,

(Footnotes are those of Table 3)

| --Description--- | | ----Materials used----- | | | | -----Conditions----- | | | -Structure- | |
|---|---|---|---|---|---|---|---|---|---|---|
| CATALYST System Designation | (Salt/Zeolite) | Solvent | Solv. Wt. | Salt Wt. | Zeol. Wt. | Temp. a) | Method b) | Example c) | XRD % xtal d) | S.A. sq.m. /g e) |
| F-1 | $Sr(OAc)_2$/5A | $H_2O$ | 2.45 | 0.97 | 2.02 | 150 | I | CC | 70.4 | 235 |
| F-2 | | ----sample above--- | | | | 550 | C | CC | 79.0 | 268 |
| F-3 | $Ba(OAc)_2$/5A | $H_2O$ | 1.79 | 1.27 | 2.26 | 150 | I | CC | 22.4 | 64 |
| F-4 | | ----sample above--- | | | | 550 | C | CC | 32.0 | 124 |
| F-5 | $Ca(OAc)_2$/5A | $H_2O$ | 2.27 | 0.72 | 2.10 | 150 | I | CC | 84.2 | 266 |
| F-6 | | ----sample above--- | | | | 550 | C | CC | 80.8 | 273 |
| F-7 | $Ba(OAc)_2$/5A | $H_2O$ | 5.0 | 1.72 | 4.03 | 150 | I | CC | --- | -- |
| | $NH_4OH/Ba(OAc)_2$/5A | 1.6cc of 10N NH4OH | | | | room | P | DD | --- | -- |
| F-8 | | 5.5cc 1N $NH_4OH$ above | | | | 150 | S/W | DD | 53.3 | 64 |
| F-9 | | -----sample above--- | | | | 550 | C | DD | 48.0 | 61 |
| F-10 | $Sr(OAc)_2$/5A | $H_2O$ | 4.0 | 1.47 | 4.00 | 150 | I | CC | --- | -- |
| | $NH_4OH/Sr(OAc)_2$/5A | 1.6cc of 10N $NH_4OH$ | | | | room | P | DD | --- | -- |

Table 4

Illustrative Embodiments,
(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| F-11 | 5.5cc 1N $NH_4OH$ above | | | | 150 | S/W | DD | 84.5 | -- |
| F-12 | -----sample above--- | | | | 550 | C | DD | 85.7 | 322 |
| F-13 Mg(OAc)$_2$/5A | MeOH | 0.2 | 0.14 | 1.00 | 150 | I | GG | 90.7 | -- |
| F-14 | -----sample above--- | | | | 550 | C | GG | 111.9 | 417 |

Table 4 (cont'd)

Illustrative Embodiments,

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| F-15 $Ba(OAc)_2$/5A | $H_2O$ | 3.5 | 1.69 | 4.02 | 150 | I | CC | --- | -- |
| $K_2CO_3$/$Ba(OAc)_2$/5A | $H_2O$ | 1.43 | 1.43 | above | room | P | KK | --- | -- |
| F-16 $K_2CO_3$ on above | $H_2O$ | 5.5 | 0.5 | above | 150 | S/W | KK | 44.6 | -- |
| F-17 | -----sample above--- | | | | 550 | C | KK | 46.5 | 15 |
| F-18 $Sr(OAc)_2$/5A | $H_2O$ | 3.5 | 1.51 | 4.00 | 150 | I | CC | --- | -- |
| $K_2CO_3$/$Sr(OAc)_2$/5A | $H_2O$ | 1.39 | 1.39 | above | room | P | KK | --- | -- |
| F-19 $K_2CO_3$ on above | $H_2O$ | 5.5 | 0.5 | above | 150 | S/W | KK | 55.6 | -- |
| F-20 | -----sample above--- | | | | 550 | C | KK | 61.7 | 30 |
| F-21 $Mg(OAc)_2$/5A | DMSO | 0.5 | 0.26 | 2.05 | 150 | I | OO | 86.1 | 387 |
| F-22 | -----sample above--- | | | | 550 | C | OO | 105.1 | -- |

Table 4 (cont'd)

Illustrative Embodiments,

(Footnotes are those of Table 3)

| --Description--- CATALYST System Designation (Salt/Zeolite) | ----Materials used----- Solvent | Solv. Wt. | Salt Wt. | Zeol. Wt. | -----Conditions----- Temp. a) | Method b) | Example c) | -Structure- XRD % xtal d) | S.A. sq.m. /g e) |
|---|---|---|---|---|---|---|---|---|---|
| F-23 Ba(OAc)$_2$/5A | $H_2O$ | 6.94 | 4.57 | 10.2 | 150 | I | CC | --- | -- |
| $NH_3$/Ba(OAc)$_2$/5A | 19g NH3 gas | | above | | room | P | AA | --- | -- |
| | 200cc lime wsh on above | | | | | W | AA | | |
| F-24 Lime wash above | 200cc lime wsh above | | | | 100 | W | AA | 59.6 | 380 |
| F-25 | -----sample above--- | | | | 450 | C | AA | 54.2 | 356 |
| F-26 Ca Butyrate/5A | dry ground | | 2.24 | 21.36 | room | G | PP | 111 | -- |
| F-27 | -----sample above--- | | | | 550 | M,C | PP | 104 | 408 |

Table 4 (cont'd)

Illustrative Embodiments,

(Footnotes are those of Table 3)

| --Description--- | ----Materials used----- | | | | -----Conditions----- | | | -Structure- | |
|---|---|---|---|---|---|---|---|---|---|
| CATALYST System Designation (Salt/Zeolite) | Solvent | Solv. Wt. | Salt Wt. | Zeol. Wt. | Temp. a) | Method b) | Example c) | XRD % xtal d) | S.A. sq.m. /g e) |
| F-28 Ca Oleate/5A | dry grind | | 3.02 | 10.02 | 100 | G,M | PP | | |
| | dry grind | | above+20g | 5A | 100 | G,M | PP | | |
| | dry grind | | above | sample | 550 | G,C | PP | 98.7 | 426 |
| F-29 $Mg(OAc)_2$ on | $H_2O$ | .75 | .47 | 1.01 | 150 | I | QQ | | |
| F-30 Ca/Morden (C10) | -----sample above--- | | | | 550 | C | QQ | 63.5 | -- |
| F-31 $Mg(OAc)_2$ on | $H_2O$ | .75 | .47 | 1.01 | 150 | I | QQ | | |
| F-32 Ca/LZY82 (C14) | -----sample above--- | | | | 550 | C | QQ | 87.5 | 402 |
| F-33 $Ca(OAc)_2$ on | $H_2O$ | .66 | .21 | 1.01 | 150 | I | QQ | | |
| F-34 Ca/Morden (C10) | -----sample above--- | | | | 550 | C | QQ | 56.6 | 230 |
| F-35 $Ca(OAc)_2$ on | $H_2O$ | .77 | .24 | 1.05 | 150 | I | QQ | | |
| F-36 Ca/LZY82 (C14) | -----sample above--- | | | | 550 | C | QQ | 87.7 | 486 |

Table 4

Illustrative Embodiments (Continued)

(Footnotes are those of Table 3)

| --Description--- CATALYST System Designation (Salt/Zeolite) | | --Structure-- n-C8 wet-ness cc/g f) | Pore vol. cc/g g) | Basicity Resulting: pH 1% aq. h) | INDICATOR COLORS (i): 4-nitro-aniline /DMSO | 4-chloro aniline /DMSO | 4-nitro-aniline /benzene | 4-chloro-aniline /benzene |
|---|---|---|---|---|---|---|---|---|
| F-1 | $Sr(OAc)_2/5A$ | ---- | 0.171 | 7 | yellow | nd | nd | cream |
| F-2 | | ---- | 0.214 | 10 | green solid | nd | nd | gray |
| F-3 | $Ba(OAc)_2/5A$ | ---- | 0.068 | 7 | yellow | nd | nd | cream |
| F-4 | | ---- | 0.143 | 9 | dark gray | nd | nd | gray |
| F-5 | $Ca(OAc)_2/5A$ | ---- | 0.149 | 7 | yellow | gray | gray | cream |
| F-6 | | ---- | 0.239 | 8 | light&dk grn | nd | nd | light gray |
| F-7 | $Ba(OAc)_2/5A$ | ---- | --- | - | ---- | -- | -- | ------- |
| | $NH_4OH/Ba(OAc)_2/5A$ | --- | --- | - | ---- | -- | -- | ------- |
| F-8 | | ---- | 0.067 | 7 | yellow | nd | nd | cream |
| F-9 | | ---- | 0.065 | 8 | light-green | lt-gray | lt-gray | v pale gray |

Table 4

Illustrative Embodiments (Continued)

(Footnotes are those of Table 3)

| --Description--- CATALYST System Designation (Salt/Zeolite) | --Structure-- n-C8 wetness cc/g f) | Pore vol. cc/g g) | Basicity Resulting: pH 1% aq. h) | INDICATOR COLORS --(i): 4-nitro-aniline /DMSO | 4-chloro aniline /DMSO | 4-nitro-aniline /benzene | 4-chloro-aniline /benzene |
|---|---|---|---|---|---|---|---|
| F-10 $Sr(OAc)_2/5A$ | ---- | --- | - | ---- | -- | -- | ------- |
| $NH_4OH/Sr(OAc)_2/5A$ | --- | --- | - | ---- | -- | -- | ------- |
| F-11 | ---- | --- | 7 | yellow | nd | nd | cream |
| F-12 | ---- | 0.254 | 8 | green | nd | nd | light gray |
| F-13 $Mg(OAc)_2/5A$ | ---- | --- | 7 | yellow | nd | nd | cream |
| F-14 | ---- | 0.309 | 8 | green | nd | nd | cream |

Table 4 (cont'd)

Illustrative Embodiments (Continued)

(Footnotes are those of Table 3)

| --Description--- | --Structure-- | | ------------Basicity Resulting------------ | | | | |
|---|---|---|---|---|---|---|---|
| CATALYST System Designation (Salt/Zeolite) | n-C8 wetness cc/g f) | Pore vol. cc/g g) | pH 1% aq. h) | INDICATOR COLORS (i): 4-nitroaniline /DMSO | 4-chloroaniline /DMSO | 4-nitroaniline /benzene | 4-chloroaniline /benzene |
| F-15 $Ba(OAc)_2/5A$ $K_2CO_3/Ba(OAc)_2/5A$ | ---- --- | --- --- | - - | ---- ---- | -- -- | -- -- | ------- ------- |
| F-16 $K_2CO_3$ on above | ---- | --- | 7 | yellow | nd | nd | cream |
| F-17 | ---- | 0.04 | 8 | dark green | nd | nd | pale gray |
| F-18 $Sr(OAc)_2/5A$ $K_2CO_3/Sr(OAc)_2/5A$ | ---- --- | --- --- | - - | ---- ---- | -- -- | -- -- | ------- ------- |
| F-19 $K_2CO_3$ on above | ---- | --- | 8 | yellow | nd | nd | cream |
| F-20 | ---- | 0.11 | 9 | green | nd | nd | light gray |
| F-21 $Mg(OAc)_2/5A$ | ---- | 0.28 | 7 | yellow | nd | nd | cream |
| F-22 | ---- | 0.325 | 8 | green | nd | nd y | gray |

Table 4 (cont'd)

Illustrative Embodiments (Continued)

(Footnotes are those of Table 3)

| --Description--- CATALYST System Designation (Salt/Zeolite) | --Structure-- n-C8 wetness cc/g f) | Pore vol. cc/g g) | Basicity Resulting: pH 1% aq. h) | INDICATOR COLORS (i): 4-nitroaniline /DMSO | 4-chloro aniline /DMSO | 4-nitroaniline /benzene | 4-chloroaniline /benzene |
|---|---|---|---|---|---|---|---|
| F-23 $Ba(OAc)_2/5A$ | ---- | --- | - | ---- | -- | -- | ------- |
| $NH_{\circ}/Ba(OAc)_2/5A$ | ---- | --- | - | ---- | -- | -- | ------- |
| F-24 Lime wash above | ---- | 0.291 | 7 | yellow | nd | nd | cream |
| F-25 | ---- | 0.268 | 8 | green | lt gray | olive | v lt-lav |
| F-26 Ca Butyrate/5A | ---- | --- | 7 | yellow | nd | nd | beige |
| F-27 | ---- | 0.32 | 9 | lt-brown | pink-purple | olive/ drab | lt-purple/ bwn |

Table 4 (cont'd)

Illustrative Embodiments (Continued)

(Footnotes are those of Table 3)

| --Description--- | --Structure-- | | ------------Basicity Resulting------------- | | | | |
|---|---|---|---|---|---|---|---|
| CATALYST | n-C8 | Pore | pH | --------- INDICATOR COLORS --(i)-------- | | | |
| System | wet- | vol. | 1% | 4-nitro- | 4-chloro | 4-nitro- | 4-chloro- |
| Designation | ness | cc/g | aq. | aniline | aniline | aniline | aniline |
| (Salt/Zeolite) | cc/g f) | g) | h) | /DMSO | /DMSO | /benzene | /benzene |
| F-28 Ca Oleate/5A | ---- | | | | | | |
| | ---- | ---- | 6 | ---- | -- | -- | -------- |
| | ---- | 0.33 | 10 | green | nd | nd | purple |
| F-29 $Mg(OAc)_2$ on | | | | | | | |
| F-30 Ca/Morden (C10) | ---- | 0.122 | 8 | dk green | nd | nd | dk purple |
| F-31 $Mg(OAc)_2$ on | | | | | | | |
| F-32 Ca/LZY82 (C14) | ---- | 0.271 | 8 | dk green | nd | nd | dk purple |
| F-33 $Ca(OAc)_2$ on | | | | | | | |
| F-34 Ca/Morden (C10) | ---- | 0.15 | 8 | dk gray | nd | nd | dk gray |
| F-35 $Ca(OAc)_2$ on | | | | | | | |
| F-36 Ca/LZY82 (C14) | ---- | 0.299 | 8 | dk gray | nd | nd | dk purple |

As described hereinbefore, double bond isomerization is one of the chemical processes which can be carried out advantageously with the basic zeolites according to the present invention.

Double bond isomerization is a reaction catalyzed by basic and acidic catalysts. Basic catalysts are normally more selective, since they do not generate cations which can undergo skeletal isomerizations.

NaY zeolite itself is reported in the literature to catalyze cracking processes by a radical mechanism. The cracking observed along with isomerization by NaY zeolite is largely consistent with the reported radical mechanism, perhaps with a contribution from weak acid sites. When NaY zeolite and CaY zeolites are converted to the instant compositions, the resulting products lose their cracking activity.

The instant compositions are thus particularly useful for the isomerization of additive range ($C_4$ to $C_8$) olefins and detergent range ($C_{10}$ to $C_{18}$) olefins, although higher range olefins can be isomerized. Particularly desired feedstocks to be isomerized are alpha olefins. Isomerization is carried out in a gas or liquid phase at isomerization conditions. Isomerization conditions typically include a temperature in the range of from 0°C to 500°C, preferably from 100°C to 150°C, a pressure in the range of from about 1.07 bar to about 138.8 bar and a weight hourly space velocity in the range of from 0.1 to about 20.

## Catalyst Preparation

The catalysts utilized were prepared as follows:

### I. Comparative NaY Zeolite

Baylith CP-190 NaY zeolite (straight out of the can) was calcined at 500°C for one hour in flowing nitrogen.

### II. Low Base Composition

Baylith CP-190 NaY zeolite was dried in a 100°C vacuum oven for 16 hrs. 125 Grams of this dried NaY zeolite was impregnated with 4.81g of potassium carbonate (MCB ACS reagent) dissolved in 62.4cc of deionized water. The zeolite was mixed in a dish during impregnation and then dried 16hrs in a 100°C vacuum oven. This dried material was calcined at 550°C for 55 minutes in flowing nitrogen. This material contained about 1.8 equivalents of potassium per zeolite supercage.

### III. High Base Composition

Baylith CP-190 NaY zeolite was dried in a 100°C vacuum oven for 16hrs. 61.7 Grams of this dried NaY zeolite was impregnated with 36.53g of potassium carbonate (MCB ACS reagent) dissolved in 61cc of deionized water. The zeolite was mixed in a dish during impregnation and then dried 66hrs in a 100°C vacuum oven. The dried zeolite was pressed in bags in an isostatic press at 1379 bar for 2 minutes. It was then ground and sieved to 16-30 mesh particles. These particles were calcined at 550°C for one hour in flowing nitrogen. This material contained about 20 equivalents of potassium per zeolite supercage.

## Catalyst Testing

Isomerization catalysts were tested in a stainless steel flow reactor measuring 37.5 cm in length and 1.6 cm in internal diameter. The reactor was packed bottom to top as follows: a small wad of glass wool, 33cc of catalyst, a small wad of glass wool, 25cc 80 grit silicon carbide, followed by another small wad of glass wool. The reactor was operated in a vertical mode in an upright furnace. The feed entered from the top. During isomerization the reactor was operated at 400°C for one hour with a nitrogen flow rate of 20 l/h and a 1-octene flow rate as indicated in Table 5. After one hour of operation a gas sample was taken and analyzed and the results reported in Table 5. Organic liquid which had been collected in a dry ice trap for the duration of the run was also analyzed and the results reported in Table 5.

Table 5

| | Products | | | | |
|---|---|---|---|---|---|
| Catalyst | 1-Octene Feed | 1-Octene | 2-Octene | 3-Octene | Aromatics |
| NaY<br>Low Base<br>High Base | 5.7g/h<br>6.1g/h<br>5.2g/h | 67.6<br>89.5<br>47.5 | 15.7<br>3.6<br>19.1 | 12.0<br>3.9<br>32.1 | 0.19<br>0.05<br>none |
| | Products | | | | |
| | < | Gaseous Cracking Products | Liquid[a] Cracking Products | n-Octane[b] | |
| NaY<br>Low Base<br>High Base | | 0.55<br>0.87<br>0.04 | 1.03<br>0.9<br>0.0 | 2.82<br>1.48<br>1.3 | |

a) Defined as new material eluting before n-octane elution.
b) gc analysis of feed indicated about 1.32% n-octane impurity.

As can be seen from the Table, the instant compositions show substantially no alkane make when compared to NaY zeolite. The high base composition also shows more isomerization activity than NaY zeolite and shows almost no cracking activity. Further analysis shows skeletal isomerization characteristic of acid catalysis in the sodium exchanged zeolite Y products, and not in the high base products. The lack of skeletal isomerization and cracking in the high base case confirm the virtual absence of acid sites in this basic zeolite. At the high base loadings, the instant composition gave a selectivity of 99.9% or greater to olefin double bond isomerization, while the standard ion exchanged NaY gave a selectivity of less than 87%.

Further Illustrative Embodiment

Catalyst Preparation

The catalysts utilized were prepared as follows:

Fully metal exchanged zeolites Calcium-A (5A), Sodium-X (13X) and Sodium-Y (LZY-52) were used as comparative catalysts in an isomerization test and to prepare the basified catalysts of the instant invention.

CA-1

60.72 Grams of 5A zeolite pellets and 1 litre Barnstead water were stirred for approximately 16hrs, then filtered. An additional 900cc Barnstead water was poured through the filter funnel. The cake was then dried for 1 hour in a 150°C vacuum oven.

A-1

20.02 Grams of the washed 5A zeolite above were impregnated with 3.93g $Ca(NO_3)_2.4H_2O$ dissolved in 1.59cc water. The zeolite was mixed in a pyrex dish during impregnation, and allowed to rest for 9 minutes before drying 1 hour in a 150°C vacuum oven. The zeolite was then impregnated with 4cc water. After 29 minutes, the zeolite was dried 70 minutes in a 150°C vacuum oven. The powder was stirred with 100cc 1N KOH for 1 hour, then filtered. The cake was stirred with an additional 100cc 1N KOH for 1 hour, then filtered, and dried overnight in a 150°C vacuum oven.

CA-2

60.91 Grams of 13X zeolite pellets were stirred overnight with 1 litre Barnstead water, then filtered. An additional 900cc water was poured through the filter funnel. The zeolite was dried 1 hour in a 150°C vacuum oven.

A-2

20.0 Grams of the washed 13X zeolite above were impregnated with 3.93g $Ca(NO_3)_2.4H_2O$ dissolved in 1.59cc Barnstead water. The zeolite was mixed in a pyrex dish during impregnation, then allowed to rest for 12 minutes, and then dried 62 minutes in a 150°C vacuum oven. The zeolite was then impregnated with 4cc water, allowed to rest for 31 minutes, then dried 65 minutes in a 150°C vacuum oven. The dried powder was stirred with 100CC 1N KOH for 1 hour then filtered. The cake was then stirred with an additional 100cc 1N KOH for 1 hour, then filtered, then dried overnight in a 150°C vacuum oven.

CA-3

130.4 Grams of LZY52 powder from Union Carbide Corporation were stirred for 1 hour with 1.5 litres water, then filtered. An additional 400cc water was poured through the filter. The cake was dried 1 hour in a 150°C vacuum oven.

A-3

20.03 Grams of the washed LZY52 powder above were impregnated with 3.93g $Ca(NO_3)_2.4H_2O$ dissolved in 1.59cc water. The zeolite was mixed in a pyrex dish during impregnation, allowed to rest for 15 minutes, then dried 62 minutes in a 150°C vacuum oven. The zeolite was then impregnated with 4cc water, allowed to rest for 31 minutes, then dried 70 minutes in a 150°C vacuum oven. The dried powder was stirred 65 minutes with 100cc 1N KOH, then filtered. The cake was stirred 1 hour with an additional 100cc 1N KOH, filtered and dried overnight in a 150°C vacuum oven.

A-4

20.02 Grams of the washed LZY52 powder above were impregnated with 4.37g $Mg(NO_3)_2.6H_2O$ dissolved in 2.40cc water. The zeolite was mixed in a pyrex dish during impregnation, allowed to rest for 37 minutes, then dried 105 minutes in a 150°C vacuum oven. The zeolite was then impregnated with 4cc water, allowed to rest for 24 minutes, then dried 67 minutes in a 150°C vacuum oven. The dried powder was stirred 53 minutes with 100cc 1N KOH, then filtered. The cake was stirred overnight with an additional 100cc 1N KOH, filtered and dried overnight in a 150°C vacuum oven.

The catalysts and comparative catalysts are listed in Table 6.

Table 6

| Isomerization Catalyst | | |
|---|---|---|
| Catalyst No. | Zeolite | Basifying Agent |
| CA-1 | 5A(Ca-A) | none |
| A-1 | 5A(Ca-A) | $Ca(OH)_2$ |
| CA-2 | 13X(Na-X) | none |
| A-2 | 13X(Na-X) | $Ca(OH)_2$ |
| CA-3 | LZY-52(Na-Y) | none |
| A-3 | LZY-52(Na-Y) | $Ca(OH)_2$ |
| A-4 | LZY-52(Na-Y) | MgO |

Catalyst Testing

Isomerization catalysts were tested in a stainless steel flow reactor as described hereinbefore. The reactor was packed from top to bottom as follows: a small wad of glass wool, 25cc of 60-80 grit SiC, 14cc of catalyst, a small wad of glass wool, 10cc 60-80 grit silicon carbide, followed by another small wad of glass wool. The reactor was operated in a vertical mode in an upright furnace. During isomerization the reactor was heated to 275 °C under a nitrogen flow rate of about 20 litres per hour and a 1-octene flow rate of about 10 grams per hour. After one hour of operation the reaction was stopped and organic liquid trapped in an attached cold trap was analyzed and the results reported in Table 7. The temperature was then increased to 400 °C and the next isomerization test was conducted with organic liquid sample being collected for one hour of operation for analysis.

Table 7

| Isomerization of 1-Octene | | | |
|---|---|---|---|
| Catalyst No. | Temp | Cracking | Double Bond Isom |
| | °C | wt % | mole % |
| CA-1 | 400 | 4.4 | 39.5 |
| A-1 | 400 | <0.1 | 57.1 |
| CA-2 | 400 | 62.3 | 10.3 |
| A-2 | 400 | ~0.4 | 45.9 |
| CA-3 | 275 | 7.8 | 42.1 |
| CA-3 | 400 | 8.7 | 36.5 |
| A-3 | 275 | <0.2 | 12.2 |
| A-3 | 400 | 0-.6 | 49.9 |
| A-4 | 275 | ~0.01 | 59.2 |
| A-4 | 400 | <0.3 | 78.3 |

As can be seen from Table 7, the basified zeolites of the instant invention produce significantly less cracking than the corresponding non-basified zeolites and are more active.

## Claims

1. A basic composition comprising a zeolite and one or more metal compounds of Group IA, IIA, the Transition Metals or the Rare Earth Metals wherein the sum of the amount of the metal compounds in said compound and any metal cation exchanged into the zeolite is in excess of that required to provide a fully metal cation-exchanged zeolite.

2. A composition according to claim 1 which comprises a zeolite and an alkali(ne earth) metal compound wherein the sum of the amount of alkali(ne earth) metal in said compound(s) and any metal cation exchanged into the zeolite is in excess of that required to provide a fully metal cation-exchanged zeolite.

3. A composition according to claim 2 which comprises a zeolite and an alkali(ne earth) metal compound and a Transition Metal and/or Rare Earth Metal compound wherein the sum of the amount of alkali(ne earth) metal and Transition Metal and/or Rare Earth Metal in said compound(s) and any metal cation exchanged into the zeolite is in excess of that required to provide a fully metal cation-exchanged zeolite.

4. A composition according to claim 3 which comprises a zeolite and an alkali metal compound and a Transition Metal and/or Rare Earth Metal compound wherein the sum of the amount of alkali metal and Transition Metal and/or Rare Earth Metal in said compound(s) and any cation exchanged into the zeolite is in excess of that required to provide a fully metal cation-exchanged zeolite.

5. A composition according to claim 3 which comprises a zeolite and an alkaline earth metal compound and a Transition Metal and/or Rare Earth Metal compound wherein the sum of the amount of alkaline earth and Transition Metal and/or Rare Earth Metal in said compound(s) and any cation exchanged into the zeolite is in excess of that required to provide a fully metal cation-exchanged zeolite.

6. A composition according to one or more of claims 1-5, wherein the sum of the amount of one or more metals of Group IA, IIA, the Transition Metals or the Rare Earth Metals in said compound(s) and any metal cation exchanged into the zeolite is in excess of about 1.05 times, in particular in excess of about 1.1 times the amount required to provide a fully metal cation-exchanged zeolite.

7. A composition according to claim 7, wherein the sum is in excess of about 1.2 times, in particular in excess of about 1.5 times and preferably in excess of about 2 times the amount required to provide a fully metal cation-exchanged zeolite.

8. A basic composition comprising a fully metal cation-exchanged zeolite and one or more metal compounds of Group IA, IIA, the Transition Metals or the Rare Earth Metals.

9. A composition according to claim 8, wherein the amount of metal compound(s) of Group IA, IIA, the Transition Metals or the Rare Earth Metals present in said composition is greater than about 0.05 times, in particular greater than about 0.1 times the amount that would be required to provide a fully metal cation-exchanged zeolite.

10. A composition according to claim 9, wherein the amount of metal compound(s) is greater than about 0.2 times, in particular greater than about 0.5 times and preferably greater than about 1 times the amount that would be required to provide a fully metal cation-exchanged zeolite.

11. A composition according to one or more of the preceding claims wherein the metal compound(s) is (are) not hydroxide(s).

12. A basic zeolite containing in compound form an excess of one or more metal compounds of Group IA, IIA, the Transition Metals or the Rare Earth Metals over that necessary to provide a fully metal cation-exchanged zeolite.

13. A basic zeolite according to claim 12 containing micropores from about four to about twelve angstrom units in diameter.

14. A basic zeolite according to claim 12 or 13 wherein the sum of the amount of metal compound(s) in said zeolite and any metal cation exchanged into the zeolite is in excess of about 1.05 times, in particular in excess of about 1.1 times the amount required to provide a fully metal cation-exchanged zeolite.

15. A basic zeolite according to claim 14 wherein the sum is in excess of about 1.2 times, in particular in excess of about 1.5 times and preferably in excess of about 2 times the amount required to provide a fully metal cation-exchanged zeolite.

16. A basic zeolite according to one or more of claims 12 - 15, wherein the metal compound(s) is (are) not hydroxide(s).

17. A structured metal-containing alumino silicate containing micropores from about four to about twelve angstrom units in diameter and further containing in compound form an excess of metal compound(s) of one or more Group IA, IIA, the Transition Metals or the Rare Earth Metals over that necessary to provide a fully metal cation-exchanged zeolite.

18. A structured metal-containing alumino silicate according to claim 17, wherein the sum of the amount of metal compound(s) in said alumino silicate and any metal cation exchanged into the alumino silicate is in excess of about 1.05 times, in particular in excess of about 1.1 times the amount required to provide a fully metal cation-exchanged zeolite.

19. A structured metal-containing alumino silicate according to claim 18, wherein the sum is in excess of about 1.2 times, in particular in excess of about 1.5 times and preferably in excess of about 2 times the amount required to provide a fully metal cation-exchanged zeolite.

20. A structured metal-containing alumino silicate according to one or more of claims 17-19, wherein the metal compound(s) is (are) not hydroxide(s).

21. A composition prepared by impregnating a zeolite with a solution of one or more metal compounds of Group IA, IIA, the Transition Metals or the Rare Earth Metals wherein the sum of the amount of metal compound(s) in said zeolite and any metal cation exchanged into the zeolite is in excess of that required to provide a fully metal cation-exchanged zeolite, drying the thus impregnated zeolite and calcining the zeolite at a temperature ranging from 150 °C to 850 °C.

22. A composition according to claim 21 wherein a solution of one or more metal compounds is used wherein said metal compound(s) is (are) thermally decomposable upon calcination in the presence of said zeolite and wherein the sum of said metal(s) impregnated into the zeolite and any metal cation exchanged into the zeolite is in excess of that required to provide a fully metal cation-exchanged zeolite, drying the thus impregnated zeolite and calcining the zeolite at a temperature in excess of the temperature at which the metal compound(s) thermally decompose(s).

23. A composition according to claim 21 or 22 wherein the metal salt(s) comprise(s) one or more carbonates, bicarbonates, acetates or oxalates.

24. A composition according to claim 21 or 22 wherein the impregnated zeolite is contacted with a precipitating agent whereby an insoluble Group IIA, Transition Metal or Rare Earth Metal compound, in particular an insoluble Group IIA compound precipitates into the zeolite.

25. A composition according to claim 25 wherein the precipitating agent is a an aqueous solution of a hydroxide of an alkali metal, ammonium and/or mixtures thereof.

26. A composition according to claim 24 wherein the precipitating agent is selected from hydroxide, sulphate, carbonate or mixtures thereof.

27. A composition according to claim 24 wherein the precipitating agent is selected from ammonia, carbon dioxide, sulphur trioxide and mixtures thereof.

28. A composition according to claim 24 wherein the impregnated zeolite is additionally impregnated with a solution of one or more soluble Group IA, IIA, Transition Metal or Rare Earth Metal salt(s) or mixtures thereof.

29. A composition prepared by impregnating a zeolite with one or more molten or vaporous metal compounds of Group IA, IIA, the Transition Metals or the rare Earth Metals wherein the sum of the amount of metal compound(s) in said zeolite is in excess of that required to provide a fully metal cation-exchanged zeolite and calcining the zeolite at a temperature ranging from 150 °C to 850 °C.

30. A composition according to claim 29 wherein one or more molten or vaporous metal compound(s) is (are) used wherein said metal compound(s) is (are) thermally decomposable upon calcination in the presence of said zeolite and wherein the sum of said metal(s) impregnated into the zeolite and any metal cation exchanged into the zeolite is in excess of that required to provide a fully metal cation-exchanged zeolite and calcining the zeolite at a temperature in excess of the temperature at which the metal compound(s) decompose(s).

31. A composition according to one or more of claims 21-30 wherein the calcination is carried out at a temperature ranging from 200 °C to 750 °C, in particular between 200 °C and 600 °C.

32. A composition according to one or more of claims 21, 22, 29 or 30 wherein the sum of the amount of the metal compound(s) impregnated into the zeolite and any metal cation exchanged into the zeolite is in excess of about 1.05 times, in particular in excess of about 1.1 times the amount required to provide a fully metal cation-exchanged zeolite.

33. A composition according to claim 32 wherein the sum is in excess of about 1.2 times, in particular in excess of about 1.5 times and preferably in excess of about 2 times the amount required to provide a fully metal cation-exchanged zeolite.

34. A composition according to one or more of the preceding claims which contains alumina incorporated in the pores and/or the supercages of the zeolite which alumina has been incorporated prior to treatment with one or more metal compounds of Group IA, IIA, the Transition Metals or the Rare Earth Metals.

35. A process for preparing a composition-of-matter comprising a zeolite and one or more metal compound(s) of Group Ia, IIA, the Transition Metals or the Rare Earth Metals which comprises impregnating a zeolite with a solution of one or more of said metal compounds wherein the sum of said metal compound-(s) impregnated into the zeolite and any metal cation exchanged into the zeolite is in excess of that required to provide a fully metal cation-exchanged zeolite, drying the thus impregnated zeolite and calcining the zeolite at a temperature ranging from 150 °C to 850 °C.

36. A process according to claim 35 wherein a solution of one or more metal compounds is used wherein said metal compound(s) is (are) thermally decomposable upon calcination in the presence of said zeolite and wherein the sum of said metal(s) impregnated into the zeolite is in excess of that required to provide a fully metal cation-exchanged zeolite, drying the thus impregnated zeolite and calcining the zeolite at a temperature in excess of the temperature at which the metal compound(s) decompose(s).

37. A process for preparing a composition-of-matter comprising a zeolite and one or more metal compounds of Group Ia, IIA, the Transition Metals or the Rare Earth Metals which comprises impregnating a zeolite with one or more molten or vaporous metal compounds wherein the sum of the amount of metal compound(s) impregnated into the zeolite and any metal cation exchanged into the zeolite is in excess of that required to provide a fully metal cation-exchanged zeolite and calcining the zeolite at a temperature ranging from 150 °C to 850 °C.

38. A process according to claim 37 wherein one or more molten or vaporous metal compounds are used wherein said metal compound(s) is (are) thermally decomposable upon calcination in the presence of said zeolite and wherein the sum of said metal(s) impregnated into the zeolite and any metal cation exchanged into the zeolite is in excess of that required to provide a fully metal cation-exchanged zeolite and calcining the zeolite at a temperature in excess of the temperature at which the metal compound(s) thermally decompose(s).

39. A process according to claim 35 wherein the impregnated zeolite is reacted with a precipitating agent whereby an insoluble Group IIA, Transition Metal or Rare Earth Metal compound, in particular an insoluble Group IIA compound precipitates within the zeolite.

40. A process according to claim 39 wherein the precipitating agent is selected from hydroxide, sulphate, carbonate and mixtures thereof.

41. A process according to claim 39 wherein the precipitating agent is selected from ammonia, carbon dioxide, sulphur trioxide and mixtures thereof.

42. A process according to claim 39 wherein the impregnated zeolite is additionally impregnated with a solution of one or more soluble Group IA, IIA, Transition Metal or Rare Earth metal salt(s) or a mixture thereof.

43. A process according to one or more of claims 35-42 wherein the calcination is carried out at a temperature ranging from 200 °C to 750 °C, in particular between 200 °C and 600 °C.

44. A process according to one or more of claims 35-43 wherein the sum of the amount of metal compound(s) impregnated into the zeolite and any metal cation exchanged into the zeolite is in excess of about 1.05 times, in particular in excess of about 1.1 times the amount required to provide a fully metal cation-exchanged zeolite.

45. A process according to claim 44 wherein the sum is in excess of about 1.2 times, in particular in excess of about 1.5 times and preferably in excess of about 2 times the amount required to provide a fully metal cation-exchanged zeolite.

46. A process according to one or more of claims 35-45 wherein the metal salt(s) comprise one or more carbonates, bicarbonates, acetates or oxalates.

47. A process for double bond isomerization of olefins which comprises contacting one or more olefins with a catalyst comprising a zeolite and one or more Group IA, IIA, Transition Metal or Rare Earth Metal compounds as claimed in one or more of claims 1-34, preferably prepared according to a process as claimed in one or more of claims 35-46.

48. A process according to claim 47 wherein the isomerization is carried out at a temperature in the range between 0 °C and 500 °C, in particular between 100 °C and 150 °C.

49. A process for alkylating aromatic compounds which comprises reacting one or more lower alkanols and one or more aromatic compounds in the presence of a catalyst comprising a zeolite and one or more Group IA, IIA, Transition Metal or Rare Earth Metal compounds as claimed in one or more of claims 1-34, preferably prepared according to a process as claimed in one or more of claims 35-46.

50. A process according to claim 49 wherein methanol is used as alkylating agent for phenol or toluene.

51. A process for preparing alkanols and/or aldehydes which comprises reacting one or more paraffins with an oxygen-containing gas in the presence of a catalyst comprising a zeolite and one or more Group IA, IIA, Transition Metal or Rare Earth Metal compounds together with a metal compound having oxidative properties wherein a zeolite is used as claimed in one or more of claims 1-34, preferably prepared according to a process as claimed in one or more of claims 35-46.

52. A process as claimed in claim 51 wherein air is used to produce primary alkanols and/or aldehydes in the presence of a zeolite containing a Mn, Ce and/or Sm compound.

53. Basic zeolites substantially as described hereinbefore with specific reference to the appropriate Examples.

54. Basic zeolite-containing compositions substantially as described hereinbefore with specific reference to the appropriate Examples.

55. A process for preparing basic zeolites or basic zeolite-containing compositions substantially as described hereinbefore with specific reference to the appropriate Examples.

56. Isomerized olefins whenever produced according to a process as claimed in one or more of claims 46-48.

57. Alkylated aromatic compounds whenever produced according to a process as claimed in claim 49 or 50.

58. Primary alkanols and/or aldehydes whenever produced according to a process as claimed in claim 51 or 52.